# EUROPEAN PATENT APPLICATION

(11) **EP 4 768 503 A1**
(43) Date of publication of application: **01.07.2026**
(21) Application number: 24858467.4
(22) Date of filing: 23.08.2024
(51) Int. Cl.: C07K 16/18, C12N 15/09, A61K 39/395, A61P 25/28

(54) **DEVELOPMENT AND USE OF THERAPEUTIC AGENT FOR ALZHEIMER'S DISEASE**

(30) Priority: 25.08.2023 CN 202311083911
(71) Applicant: Keymed Biosciences (Chengdu) Co., Ltd., Chengdu, Sichuan 610219 (CN)
(72) Inventor: CHEN, Bo, Chengdu, Sichuan 610219 (CN); XU, Gang, Chengdu, Sichuan 610219 (CN); GUO, Bujing, Chengdu, Sichuan 610219 (CN); HOU, Qiaoyun, Chengdu, Sichuan 610219 (CN)
(74) Representative: PGA S.p.A., Milano, Succursale di Lugano
(86) International application number: PCT/CN2024/114232
(87) International publication number: WO 2025/044931

(57) **Abstract**

Provided is an antibody or an antigen-binding fragment thereof that binds to β-Amyloid (Aβ). Further provided are a nucleic acid encoding the antibody or the antigen-binding fragment thereof, a cell comprising the antibody or the antigen-binding fragment thereof or nucleic acid thereof, a pharmaceutical composition, a kit, and the use of the antibody or the antigen-binding fragment thereof in the preparation of a drug used for treating or preventing a disease caused by abnormal accumulation or deposition of Aβ in subjects.

## Description

The present application claims the right of priority for Chinese Patent Application No. 202311083911.0 filed on August 25, 2023, which is incorporated herein by reference in its entirety.

### Technical Field

The present invention belongs to the field of biopharmaceuticals, and in particular relates to an antibody that binds to β-Amyloid (Aβ) and the use thereof.

### Background Art

Alzheimer's disease (AD) is a neurodegenerative disease that predominantly occurs in the elderly, accounting for 50% to 70% of all dementia cases worldwide and is the most common form of dementia. AD is a physical brain disease characterized by impaired brain functions such as memory, language, thinking and behaviour, and is a progressive disease caused by gradual degeneration of brain cells. Clinically, AD is characterized by progressive memory loss, cognitive impairment and behavioural abnormalities. The typical pathological manifestations include amyloid deposition, neurofibrillary tangles (NFTs), loss of neurons, abnormalities in axons and synapses, granulovacuolar degeneration, etc. With the progressive aging of the population, the incidence of Alzheimer's disease is gradually increasing, imposing heavy economic and psychological burdens on society and families due to its medical and caregiving demands. Even after adjusting for other risk factors, the relative mortality risk associated with Alzheimer's disease remains higher than that of other forms of dementia, particularly among individuals under the age of 75.

The pathogenesis of Alzheimer's disease remains incompletely understood, with the β-amyloid hypothesis and the Tau protein hypothesis being the most prominent theories. The amyloid cascade hypothesis has long held a dominant position in the pathogenesis of Alzheimer's disease (Hardy J A, et al., Science, (1992), 368:387-403). The hypothesis holds that: The abnormal metabolism of amyloid protein precursor (APP) in the brain leads to increased production and decreased degradation of Aβ, resulting in massive accumulation of Aβ. Excessive accumulation of Aβ forms amyloid plaques, which produce neurotoxicity.

The main cause of Aβ deposition in brain tissue is the imbalance in Aβ transport, which is induced by abnormal Aβ anabolic metabolism and reduced catabolic metabolism. The anabolism of Aβ mainly involves α-secretase, β-secretase and γ-secretase. The α-secretase can prevent the production of Aβ, while the β-secretase and γ-secretase promote the anabolism of Aβ. The β-secretase, also referred to as β-site amyloid protein precursor-cleaving enzyme-1 (BACE-1), is a critical ratelimiting enzyme in the production of Aβ. Therefore, the BACE-1 gene is currently a research focus in Alzheimer's disease studies. However, all inhibitors developed targeting BACE-1 have failed in clinical trials so far.

It is currently believed that excess Aβ is a necessary but not sufficient condition among the triggering factors of AD. AD therapeutic drugs targeting Aβ have become one of the main directions in clinical research. Aβ deposition can cause the onset of Alzheimer's disease, and reducing the deposition of Aβ in brain tissue can delay or alleviate the symptoms of Alzheimer's disease. Therefore, strategies to clear Aβ and thereby improve cognitive function are of significant importance.

Over the past thirty years, the majority of antibody drug developments targeting Aβ by major pharmaceutical companies have ended in failure. In August 2012, Pfizer and Johnson & Johnson announced the discontinuation of further research on the anti-Aβ monoclonal antibody Bapineuzumab. Prior to this, in the Phase III clinical trial conducted, Bapineuzumab did not improve memory loss in patients and failed to reach the clinical endpoints, and the clinical research was declared a failure. In December 2014, Gantenerumab, an anti-Alzheimer's drug that Roche had invested heavily in developing, failed during its Phase III clinical trials. On November 23, 2016, Lilly announced the results of the EXPEDITION3 trial: Solanezumab failed to achieve the expected ideal efficacy. With this, the Alzheimer's disease candidate drug, which had cost 3 billion US dollars, was declared a failure. On January 30, 2019, Roche announced the termination of two Phase III clinical studies, namely CREAD I and CREAD 2, which were designed to evaluate Crenezumab in the treatment of patients with early Alzheimer's disease (prodromal or mild AD patients). On November 6, 2020, the FDA Advisory Committee held a vote on issues regarding the clinical research evidence of Aducanumab developed by Biogen, concluding that the EMERGE trial failed to provide evidence of efficacy, and similarly, the PRIME trial also failed to provide evidence of efficacy. However, on June 7, 2021, the FDA officially approved Aducanumab (trade name: Aduhelm) developed by Biogen for the treatment of Alzheimer's disease. However, after its market launch, the drug brought limited benefits to patients and was associated with the risk of severe cerebral oedema. On January 6, 2023, the FDA approved the marketing of lecanemab-irmb, a new drug for Alzheimer's disease jointly developed by Eisai and Biogen, through the accelerated approval pathway. Lecanemab can alleviate cognitive decline to a certain extent, but it is also associated with the risk of cerebral oedema. Donanemab developed by Lilly has shown good Aβ plaque clearance efficacy in Phase II and early Phase III clinical trials, and is expected to receive marketing approval in the future.

Years of development experience, lessons, and research outcomes have shown that antibody drugs targeting Aβ can clear cerebral Aβ, alleviate cognitive decline, and ameliorate brain pathological symptoms. However, although the Aβ antibody drugs developed by Eisai and Lilly can alleviate cognitive decline to a certain extent, they still have issues with drug half-life and safety. For example, in the Phase I clinical trial of Lecanemab, the half-life in the 10 mg/kg group was 6.9 days. In the Phase I/II clinical trial of Donanemab, the half-life was 3.19 days in the 1 mg/kg intravenous administration group and 10.5 days in the 10 mg/kg group. Studies have shown that patients with Alzheimer's disease require long-term medication. Therefore, the development of long-acting anti-Aβ antibodies, which aim to enhance the convenience of antibodies and reduce antibody dosage, has broad prospects.

Therefore, since monoclonal antibody drugs with a longer half-life may achieve better efficacy, it is still necessary to improve anti-Aβ antibodies. Currently, there is an urgent need to develop a new generation of antibody drugs targeting Alzheimer's disease or other diseases caused by Aβ deposition.

### Summary of the Invention

In the present invention, mice are immunized with amyloid protofibril or pyroglutamate-modified β-amyloid, resulting in multiple high-affinity antibodies that recognize amyloid. Activity evaluation demonstrates that the antibodies can effectively clear amyloid, exhibit favourable PK parameters and low immunogenicity, have a long half-life, and thus can be used for the treatment of Alzheimer's disease and other diseases characterized by abnormal accumulation or deposition of amyloid.

According to a first aspect of the present invention, the present invention provides an antibody or an antigen-binding fragment thereof that binds to β-amyloid (Aβ), the antibody comprising a heavy chain variable region (VH) and a light chain variable region (VL), wherein
(1) the VH comprises a heavy chain complementarity determining region (HCDR) 1 as set forth in SEQ ID NO: 23, a HCDR2 as set forth in SEQ ID NO: 24, and a HCDR3 as set forth in SEQ ID NO: 25, and the VL comprises a light chain complementarity determining region (LCDR) 1 as set forth in SEQ ID NO: 28, a LCDR2 as set forth in SEQ ID NO: 29, and a LCDR3 as set forth in SEQ ID NO: 30;
(2) the VH comprises a HCDR1 as set forth in SEQ ID NO: 69, a HCDR2 as set forth in SEQ ID NO: 70, and a HCDR3 as set forth in SEQ ID NO: 71, and the VL comprises a LCDR1 as set forth in SEQ ID NO: 74, a LCDR2 as set forth in SEQ ID NO: 248, and a LCDR3 as set forth in SEQ ID NO: 76;
(3) the VH comprises a HCDR1 as set forth in SEQ ID NO: 69, a HCDR2 as set forth in SEQ ID NO: 70, and a HCDR3 as set forth in SEQ ID NO: 71, and the VL comprises a LCDR1 as set forth in SEQ ID NO: 74, a LCDR2 as set forth in SEQ ID NO: 75, and a LCDR3 as set forth in SEQ ID NO: 76;
(4) the VH comprises a HCDR1 as set forth in SEQ ID NO: 3, a HCDR2 as set forth in SEQ ID NO: 4, and a HCDR3 as set forth in SEQ ID NO: 5, and the VL comprises a LCDR1 as set forth in SEQ ID NO: 8, a LCDR2 as set forth in SEQ ID NO: 9, and a LCDR3 as set forth in SEQ ID NO: 10;
(5) the VH comprises a HCDR1 as set forth in SEQ ID NO: 13, a HCDR2 as set forth in SEQ ID NO: 14, and a HCDR3 as set forth in SEQ ID NO: 15, and the VL comprises a LCDR1 as set forth in SEQ ID NO: 18, a LCDR2 as set forth in SEQ ID NO: 19, and a LCDR3 as set forth in SEQ ID NO: 20;
(6) the VH comprises a HCDR1 as set forth in SEQ ID NO: 33, a HCDR2 as set forth in SEQ ID NO: 34, and a HCDR3 as set forth in SEQ ID NO: 35, and the VL comprises a LCDR1 as set forth in SEQ ID NO: 38, a LCDR2 as set forth in SEQ ID NO: 19, and a LCDR3 as set forth in SEQ ID NO: 20;
(7) the VH comprises a HCDR1 as set forth in SEQ ID NO: 41, a HCDR2 as set forth in SEQ ID NO: 42, and a HCDR3 as set forth in SEQ ID NO: 43, and the VL comprises a LCDR1 as set forth in SEQ ID NO: 46, a LCDR2 as set forth in SEQ ID NO: 47, and a LCDR3 as set forth in SEQ ID NO: 48;
(8) the VH comprises a HCDR1 as set forth in SEQ ID NO: 51, a HCDR2 as set forth in SEQ ID NO: 52, and a HCDR3 as set forth in SEQ ID NO: 53, and the VL comprises a LCDR1 as set forth in SEQ ID NO: 56, a LCDR2 as set forth in SEQ ID NO: 19, and a LCDR3 as set forth in SEQ ID NO: 20;
(9) the VH comprises a HCDR1 as set forth in SEQ ID NO: 59, a HCDR2 as set forth in SEQ ID NO: 60, and a HCDR3 as set forth in SEQ ID NO: 61, and the VL comprises a LCDR1 as set forth in SEQ ID NO: 64, a LCDR2 as set forth in SEQ ID NO: 65, and a LCDR3 as set forth in SEQ ID NO: 66;
(10) the VH comprises a HCDR1 as set forth in SEQ ID NO: 3, a HCDR2 as set forth in SEQ ID NO: 4, and a HCDR3 as set forth in SEQ ID NO: 79, and the VL comprises a LCDR1 as set forth in SEQ ID NO: 82, a LCDR2 as set forth in SEQ ID NO: 83, and a LCDR3 as set forth in SEQ ID NO: 84;
(11) the VH comprises a HCDR1 as set forth in SEQ ID NO: 87, a HCDR2 as set forth in SEQ ID NO: 88, and a HCDR3 as set forth in SEQ ID NO: 89, and the VL comprises a LCDR1 as set forth in SEQ ID NO: 92, a LCDR2 as set forth in SEQ ID NO: 93, and a LCDR3 as set forth in SEQ ID NO: 94;
(12) the VH comprises a HCDR1 as set forth in SEQ ID NO: 130, a HCDR2 as set forth in SEQ ID NO: 131, and a HCDR3 as set forth in SEQ ID NO: 279, and the VL comprises a LCDR1 as set forth in SEQ ID NO: 74, a LCDR2 as set forth in SEQ ID NO: 134, and a LCDR3 as set forth in SEQ ID NO: 76;
(13) the VH comprises a HCDR1 as set forth in SEQ ID NO: 59, a HCDR2 as set forth in SEQ ID NO: 137, and a HCDR3 as set forth in SEQ ID NO: 138, and the VL comprises a LCDR1 as set forth in SEQ ID NO: 141, a LCDR2 as set forth in SEQ ID NO: 65, and a LCDR3 as set forth in SEQ ID NO: 66;
(14) the VH comprises a HCDR1 as set forth in SEQ ID NO: 121, a HCDR2 as set forth in SEQ ID NO: 122, and a HCDR3 as set forth in SEQ ID NO: 144, and the VL comprises a LCDR1 as set forth in SEQ ID NO: 147, a LCDR2 as set forth in SEQ ID NO: 148, and a LCDR3 as set forth in SEQ ID NO: 149;
(15) the VH comprises a HCDR1 as set forth in SEQ ID NO: 121, a HCDR2 as set forth in SEQ ID NO: 122, and a HCDR3 as set forth in SEQ ID NO: 123, and the VL comprises a LCDR1 as set forth in SEQ ID NO: 218, a LCDR2 as set forth in SEQ ID NO: 65, and a LCDR3 as set forth in SEQ ID NO: 127;
(16) the VH comprises a HCDR1 as set forth in SEQ ID NO: 121, a HCDR2 as set forth in SEQ ID NO: 122, and a HCDR3 as set forth in SEQ ID NO: 123, and the VL comprises a LCDR1 as set forth in SEQ ID NO: 126, a LCDR2 as set forth in SEQ ID NO: 65, and a LCDR3 as set forth in SEQ ID NO: 127;
(17) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 103, and a LCDR3 as set forth in SEQ ID NO: 104;
(18) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 235, and a LCDR3 as set forth in SEQ ID NO: 104;
(19) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 238, and a LCDR3 as set forth in SEQ ID NO: 104;
(20) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 245, and a LCDR3 as set forth in SEQ ID NO: 104;
(21) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 102, a LCDR2 as set forth in SEQ ID NO: 103, and a LCDR3 as set forth in SEQ ID NO: 104;
(22) the VH comprises a HCDR1 as set forth in SEQ ID NO: 107, a HCDR2 as set forth in SEQ ID NO: 108, and a HCDR3 as set forth in SEQ ID NO: 99, and the VL comprises a LCDR1 as set forth in SEQ ID NO: 102, a LCDR2 as set forth in SEQ ID NO: 103, and a LCDR3 as set forth in SEQ ID NO: 111; or
(23) the VH comprises a HCDR1 as set forth in SEQ ID NO: 114, a HCDR2 as set forth in SEQ ID NO: 98, and a HCDR3 as set forth in SEQ ID NO: 115, and the VL comprises a LCDR1 as set forth in SEQ ID NO: 102; a LCDR2 as set forth in SEQ ID NO: 118, and a LCDR3 as set forth in SEQ ID NO: 104.

In some embodiments, the antibody or the antigen-binding fragment thereof of the present invention binds to pyroE Aβ3-42 peptide. In some embodiments, the antibody or the antigen-binding fragment thereof of the present invention binds to Aβ1-42 protofibril.

In some embodiments, the VH and VL of the antibody comprise the following amino acid sequences, respectively:
(1) the VH and VL comprise amino acid sequences that have at least 80%, at least 85%, at least 90%, at least 95%, at least 96%, at least 97%, at least 98%, at least 99% or 100% sequence identity to SEQ ID NO: 164 and SEQ ID NO: 219, respectively;
(2) the VH and VL comprise amino acid sequences that have at least 80%, at least 85%, at least 90%, at least 95%, at least 96%, at least 97%, at least 98%, at least 99% or 100% sequence identity to SEQ ID NO: 166 and SEQ ID NO: 219, respectively;
(3) the VH and VL comprise amino acid sequences that have at least 80%, at least 85%, at least 90%, at least 95%, at least 96%, at least 97%, at least 98%, at least 99% or 100% sequence identity to SEQ ID NO: 174 and SEQ ID NO: 219, respectively;
(4) the VH and VL comprise amino acid sequences that have at least 80%, at least 85%, at least 90%, at least 95%, at least 96%, at least 97%, at least 98%, at least 99% or 100% sequence identity to SEQ ID NO: 176 and SEQ ID NO: 219, respectively;
(5) the VH and VL comprise amino acid sequences that have at least 80%, at least 85%, at least 90%, at least 95%, at least 96%, at least 97%, at least 98%, at least 99% or 100% sequence identity to SEQ ID NO: 21 and SEQ ID NO: 26, respectively;
(6) the VH and VL comprise amino acid sequences that have at least 80%, at least 85%, at least 90%, at least 95%, at least 96%, at least 97%, at least 98%, at least 99% or 100% sequence identity to SEQ ID NO: 197 and SEQ ID NO: 246, respectively;
(7) the VH and VL comprise amino acid sequences that have at least 80%, at least 85%, at least 90%, at least 95%, at least 96%, at least 97%, at least 98%, at least 99% or 100% sequence identity to SEQ ID NO: 197 and SEQ ID NO: 249, respectively;
(8) the VH and VL comprise amino acid sequences that have at least 80%, at least 85%, at least 90%, at least 95%, at least 96%, at least 97%, at least 98%, at least 99% or 100% sequence identity to SEQ ID NO: 67 and SEQ ID NO: 72, respectively;
(9) the VH and VL comprise amino acid sequences that have at least 80%, at least 85%, at least 90%, at least 95%, at least 96%, at least 97%, at least 98%, at least 99% or 100% sequence identity to SEQ ID NO: 1 and SEQ ID NO: 6, respectively;
(10) the VH and VL comprise amino acid sequences that have at least 80%, at least 85%, at least 90%, at least 95%, at least 96%, at least 97%, at least 98%, at least 99% or 100% sequence identity to SEQ ID NO: 11 and SEQ ID NO: 16, respectively;
(11) the VH and VL comprise amino acid sequences that have at least 80%, at least 85%, at least 90%, at least 95%, at least 96%, at least 97%, at least 98%, at least 99% or 100% sequence identity to SEQ ID NO: 31 and SEQ ID NO: 36, respectively;
(12) the VH and VL comprise amino acid sequences that have at least 80%, at least 85%, at least 90%, at least 95%, at least 96%, at least 97%, at least 98%, at least 99% or 100% sequence identity to SEQ ID NO: 39 and SEQ ID NO: 44, respectively;
(13) the VH and VL comprise amino acid sequences that have at least 80%, at least 85%, at least 90%, at least 95%, at least 96%, at least 97%, at least 98%, at least 99% or 100% sequence identity to SEQ ID NO: 49 and SEQ ID NO: 54, respectively;
(14) the VH and VL comprise amino acid sequences that have at least 80%, at least 85%, at least 90%, at least 95%, at least 96%, at least 97%, at least 98%, at least 99% or 100% sequence identity to SEQ ID NO: 57 and SEQ ID NO: 62, respectively;
(15) the VH and VL comprise amino acid sequences that have at least 80%, at least 85%, at least 90%, at least 95%, at least 96%, at least 97%, at least 98%, at least 99% or 100% sequence identity to SEQ ID NO: 77 and SEQ ID NO: 80, respectively;
(16) the VH and VL comprise amino acid sequences that have at least 80%, at least 85%, at least 90%, at least 95%, at least 96%, at least 97%, at least 98%, at least 99% or 100% sequence identity to SEQ ID NO: 85 and SEQ ID NO: 90, respectively;
(17) the VH and VL comprise amino acid sequences that have at least 80%, at least 85%, at least 90%, at least 95%, at least 96%, at least 97%, at least 98%, at least 99% or 100% sequence identity to SEQ ID NO: 128 and SEQ ID NO: 132, respectively;
(18) the VH and VL comprise amino acid sequences that have at least 80%, at least 85%, at least 90%, at least 95%, at least 96%, at least 97%, at least 98%, at least 99% or 100% sequence identity to SEQ ID NO: 135 and SEQ ID NO: 139, respectively;
(19) the VH and VL comprise amino acid sequences that have at least 80%, at least 85%, at least 90%, at least 95%, at least 96%, at least 97%, at least 98%, at least 99% or 100% sequence identity to SEQ ID NO: 142 and SEQ ID NO: 145, respectively;
(20) the VH and VL comprise amino acid sequences that have at least 80%, at least 85%, at least 90%, at least 95%, at least 96%, at least 97%, at least 98%, at least 99% or 100% sequence identity to SEQ ID NO: 160 and SEQ ID NO: 216, respectively;
(21) the VH and VL comprise amino acid sequences that have at least 80%, at least 85%, at least 90%, at least 95%, at least 96%, at least 97%, at least 98%, at least 99% or 100% sequence identity to SEQ ID NO: 152 and SEQ ID NO: 216, respectively;
(22) the VH and VL comprise amino acid sequences that have at least 80%, at least 85%, at least 90%, at least 95%, at least 96%, at least 97%, at least 98%, at least 99% or 100% sequence identity to SEQ ID NO: 156 and SEQ ID NO: 216, respectively;
(23) the VH and VL comprise amino acid sequences that have at least 80%, at least 85%, at least 90%, at least 95%, at least 96%, at least 97%, at least 98%, at least 99% or 100% sequence identity to SEQ ID NO: 158 and SEQ ID NO: 216, respectively;
(24) the VH and VL comprise amino acid sequences that have at least 80%, at least 85%, at least 90%, at least 95%, at least 96%, at least 97%, at least 98%, at least 99% or 100% sequence identity to SEQ ID NO: 162 and SEQ ID NO: 216, respectively;
(25) the VH and VL comprise amino acid sequences that have at least 80%, at least 85%, at least 90%, at least 95%, at least 96%, at least 97%, at least 98%, at least 99% or 100% sequence identity to SEQ ID NO: 160 and SEQ ID NO: 214, respectively;
(26) the VH and VL comprise amino acid sequences that have at least 80%, at least 85%, at least 90%, at least 95%, at least 96%, at least 97%, at least 98%, at least 99% or 100% sequence identity to SEQ ID NO: 152 and SEQ ID NO: 214, respectively;
(27) the VH and VL comprise amino acid sequences that have at least 80%, at least 85%, at least 90%, at least 95%, at least 96%, at least 97%, at least 98%, at least 99% or 100% sequence identity to SEQ ID NO: 162 and SEQ ID NO: 214, respectively;
(28) the VH and VL comprise amino acid sequences that have at least 80%, at least 85%, at least 90%, at least 95%, at least 96%, at least 97%, at least 98%, at least 99% or 100% sequence identity to SEQ ID NO: 119 and SEQ ID NO: 124, respectively;
(29) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 230, respectively;
(30) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 233, respectively;
(31) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 236, respectively;
(32) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 239, respectively;
(33) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 241, respectively;
(34) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 243, respectively;
(35) the VH and VL comprise amino acid sequences that have at least 80%, at least 85%, at least 90%, at least 95%, at least 96%, at least 97%, at least 98%, at least 99% or 100% sequence identity to SEQ ID NO: 95 and SEQ ID NO: 100, respectively;
(36) the VH and VL comprise amino acid sequences that have at least 80%, at least 85%, at least 90%, at least 95%, at least 96%, at least 97%, at least 98%, at least 99% or 100% sequence identity to SEQ ID NO: 105 and SEQ ID NO: 109, respectively; or
(37) the VH and VL comprise amino acid sequences that have at least 80%, at least 85%, at least 90%, at least 95%, at least 96%, at least 97%, at least 98%, at least 99% or 100% sequence identity to SEQ ID NO: 112 and SEQ ID NO: 116, respectively.

In some embodiments, the antibody of the present invention is selected from a murine antibody, a chimeric antibody, a humanized antibody or a fully human antibody. In some embodiments, the antibody is of an antibody isotype selected from: IgG, IgA, IgM, IgE and IgD. In some embodiments, the antibody is of an antibody subclass selected from: IgG1, IgG2, IgG3 and IgG4.

In some embodiments, the antibody of the present invention comprises an IgG1 constant region (Fc region). In some embodiments, the Fc region comprises an amino acid substitution that extends the half-life of the antibody. In some embodiments, the amino acid substitution comprises L234A and L235A substitutions.

In some embodiments, the antibody of the present invention comprises a heavy chain (HC) and a light chain (LC), wherein
(1) the HC and LC comprise amino acid sequences that have at least 80%, at least 85%, at least 90%, at least 95%, at least 96%, at least 97%, at least 98%, at least 99% or 100% sequence identity to SEQ ID NO: 275 and SEQ ID NO: 277, respectively; or
(2) the HC and LC comprise amino acid sequences that have at least 80%, at least 85%, at least 90%, at least 95%, at least 96%, at least 97%, at least 98%, at least 99% or 100% sequence identity to SEQ ID NO: 271 and SEQ ID NO: 273, respectively.

In some embodiments of the antibody or the antigen-binding fragment thereof of the present invention, the antigen-binding fragment is selected from: Fab, Fab', F(ab')₂, Fv, single-chain Fv (scFv) and ds-scFv.

In some embodiments of the antibody or the antigen-binding fragment thereof of the present invention, the antibody is a monovalent antibody, a bivalent antibody or a multivalent antibody.

In some embodiments of the antibody or the antigen-binding fragment thereof of the present invention, the antibody is a monoclonal antibody, a bispecific antibody or a multispecific antibody.

According to a second aspect of the present invention, the present invention provides a nucleic acid molecule, which comprises a nucleotide sequence encoding the antibody or the antigen-binding fragment thereof according to the first aspect of the present invention.

According to a third aspect of the present invention, the present invention provides a vector, which comprises the nucleic acid molecule according to the second aspect of the present invention.

According to a fourth aspect of the present invention, the present invention provides a cell, which comprises the nucleic acid molecule according to the second aspect of the present invention or the vector according to the third aspect of the present invention.

According to a fifth aspect of the present invention, the present invention provides a method for preparing the antibody or the antigen-binding fragment thereof according to the first aspect of the present invention, which method comprises:
a) culturing the cell according to the fourth aspect of the present invention under conditions suitable for expression of the antibody or the antigen-binding fragment thereof; and
b) isolating the antibody or the antigen-binding fragment thereof from a culture and/or a culture supernatant of the cell.

According to a sixth aspect of the present invention, the present invention provides a pharmaceutical composition, which comprises the antibody or the antigen-binding fragment thereof according to the first aspect of the present invention, the nucleic acid molecule according to the second aspect of the present invention, the vector according to the third aspect of the present invention or the cell according to the fourth aspect of the present invention, and optionally, a pharmaceutically acceptable carrier or excipient.

According to a seventh aspect of the present invention, the present invention provides a kit, which comprises the antibody or the antigen-binding fragment thereof according to the first aspect of the present invention, the nucleic acid molecule according to the second aspect of the present invention, the vector according to the third aspect of the present invention, the cell according to the fourth aspect of the present invention or the pharmaceutical composition according to the sixth aspect of the present invention, and instructions for use; optionally, the kit further comprises an administration device.

According to an eighth aspect of the present invention, the present invention provides the use of the antibody or the antigen-binding fragment thereof according to the first aspect of the present invention, the nucleic acid molecule according to the second aspect of the present invention, the vector according to the third aspect of the present invention, the cell according to the fourth aspect of the present invention or the pharmaceutical composition according to the sixth aspect of the present invention in the preparation of a drug for treating or preventing a disease caused by abnormal accumulation or deposition of Aβ in a subject.

According to a ninth aspect of the present invention, the present invention provides the use of the antibody or the antigen-binding fragment thereof according to the first aspect of the present invention, the nucleic acid molecule according to the second aspect of the present invention, the vector according to the third aspect of the present invention, the cell according to the fourth aspect of the present invention or the pharmaceutical composition according to the sixth aspect of the present invention in the preparation of a drug for clearing or alleviating abnormal accumulation or deposition of Aβ in a subject.

In some embodiments of the eighth and ninth aspects of the present invention, the subject has a degenerative disease of the central nervous system. In some embodiments, the subject has Alzheimer's disease (AD), Parkinson's disease (PD), Down syndrome or cerebral amyloid angiopathy, or is at risk of having AD, PD, Down syndrome or cerebral amyloid angiopathy. In some embodiments, the subject has preclinical AD or clinical AD, e.g., prodromal AD, mild AD, moderate AD or severe AD.

### Brief Description of the Drawings

FIG. 1 shows the activity of anti-Aβ chimeric antibodies in promoting the phagocytosis of Aβ by microglia.
FIG. 2 shows the binding activity of anti-Aβ chimeric antibodies to antigen pyroE Aβ3-16 and Aβ1-42 protofibril, where 151N5 and 200O1 exhibit strong binding to pyroE Aβ3-16 but weak binding to Aβ3-16, and 6B5 and 7C8 exhibit strong binding to protofibril.
FIG. 3 shows the affinity detection results of 6B5 and 7C8 humanized antibodies, where the affinity of multiple humanized antibodies of 6B5 and 7C8 shows no significant change compared with that of the parent antibody.
FIG. 4 shows the affinity detection results of 200O1 and 151N5 humanized antibodies, where the affinity of multiple humanized antibodies of 200O1/151N5 shows no significant change compared with that of the parent antibody.
FIG. 5 shows the phagocytic activity detection results of 200O1 and 7C8 humanized antibodies, where 200O1 H1K1 and 7C8 H6K2, similar to parent antibodies thereof, can effectively promote the clearance of amyloid in brain sections by microglia.
FIG. 6 shows the half-life results of 200O1 and 7C8 humanized antibodies and variants thereof in mice, where the half-life of 200O1 H1K1 is comparable to that of Donanemab, and the half-life of 200O1 H1K1 LA is 2.45 times that of Donanemab; and the half-life of 7C8 H6K2 is comparable to that of Lecanemab, and the half-life of 7C8 H6K2 LA is 2.3 times that of Lecanemab.
FIG. 7 shows the phagocytic activity detection results of variants of 200O1 and 7C8 humanized antibodies, where both 200O1 H1K1 LA and 7C8 H6K2 LA can effectively promote the phagocytosis of Aβ in brain sections by microglia.
FIG. 8 shows the immunogenicity detection results of variants of 200O1 and 7C8 humanized antibodies, where the immunogenicity of both 200O1 H1K1 LA and 7C8 H6K2 LA is superior to that of Donanemab and Lecanemab.
FIG. 9 shows the binding activity of variants of 200O1 and 7C8 humanized antibodies to negative screening cells, where 200O1 H1K1 LA does not bind to cells overexpressing the amyloid precursor protein (APP) in negative screening, while 7C8 H6K2 LA shows weaker binding to negative screening cells than Lecanemab.
FIG. 10 shows that seven days after administration, the intracerebral concentration of the variant of 7C8 humanized antibody tends to be higher than that of Lecanemab, while the intracerebral concentration of the variant of 20001 humanized antibody is significantly higher than that of Donanemab.
FIG. 11 shows that after three months of administration in 5X FAD transgenic mice, the variants of 7C8 and 20001 humanized antibodies are more effective in reducing the Aβ level in the brain than Lecanemab and Donanemab.
FIG. 12 shows that after 13 weeks of administration in 5X FAD transgenic mice, the 7C8 humanized antibody significantly reduces the soluble and insoluble Aβ levels in the brain.
FIG. 13 shows that after 13 weeks of administration in 6-week-old FAD 4T transgenic mice, the 7C8 humanized antibody significantly reduces the soluble and insoluble Aβ levels in the brain.
FIG. 14 shows that after 13 weeks of administration in 15-week-old FAD 4T transgenic mice, the 7C8 humanized antibody significantly reduces the soluble and insoluble Aβ levels in the brain.

### Detailed Description of the Invention

In the present invention, the scientific and technical terms used herein have the meanings as commonly understood by those skilled in the art unless otherwise specified. In addition, the terms and laboratory operation steps related to the protein and nucleic acid chemistry, molecular biology, cell and tissue culture, microbiology and immunology used herein are all terms and conventional steps that are widely used in the corresponding art. Moreover, for a better understanding of the present invention, definitions and explanations of related terms are provided below.

As used herein, the singular forms "a", "an" and "the" include plural referents, unless otherwise clearly indicated in the context. Therefore, for example, reference to "an antibody" includes a plurality of antibodies, and in some embodiments, reference to "an antibody" includes a plurality of antibodies, and so forth.

Unless otherwise stated or defined, the terms "comprise" and variations thereof, such as "include", "contain" and "have", should be understood to mean the inclusion of the stated elements or steps or groups of elements or steps, but not the exclusion of any other elements or steps or groups of elements or steps.

As used herein, "and/or" means and includes any and all possible combinations of one or more of the associated listed items. For example, a composition comprising A and/or B may be interpreted as a composition comprising A, a composition comprising B, or a composition comprising A and B.

All numerical designations (e.g., pH, temperature, time, concentration, and molecular weight, including ranges) are approximations, which are appropriately varied ((+) or (-)) by increments of 1.0 or 0.1, or alternatively by variations of ±15%, ±10%, ±5%, or ±2%. It should be understood that all numerical designations are preceded by the term "about". It should also be understood that the reagents described herein are merely exemplary, and equivalents thereof are known in the art. When referring to a measurable value such as an amount or concentration, the term "about" as used herein means encompassing variations within 20%, 10%, 5%, 1%, 0.5% or 0.1% of the specified amount.

As used herein, the term "antibody" refers to an immunoglobulin molecule that has the ability to specifically bind to a particular antigen. Antibodies generally comprise variable and constant regions in each heavy and light chain. The variable regions of the heavy and light chains of an antibody contain binding domains that interact with antigens. The constant regions of an antibody can mediate the binding of the immunoglobulin to host tissues or factors, including various cells of the immune system (e.g., effector cells) and components of the complement system such as C1q (the first component in the classical pathway of complement activation). Therefore, most antibodies have a heavy chain variable region (VH) and a light chain variable region (VL) that together form the antigen-binding portion of the antibody.

A "light chain variable region" (VL) or a "heavy chain variable region" (VH) consists of "framework" regions separated by three "complementarity determining regions" or "CDRs". The framework regions are used to align CDRs that specifically bind to antigenic epitopes. The CDRs include amino acid residues in an antibody that are primarily responsible for antigen binding. Both the VL domain and the VH domain comprise, from the amino terminus to the carboxyl terminus, the following framework regions (FRs) and CDR regions: FR1, CDR1, FR2, CDR2, FR3, CDR3 and FR4. CDR1, CDR2 and CDR3 of the VL domain are also referred to herein as LCDR1, LCDR2 and LCDR3, respectively; CDR1, CDR2 and CDR3 of the VH domain are also referred to herein as HCDR1, HCDR2 and HCDR3, respectively.

The arrangement of amino acids in each VL domain and VH domain is consistent with any conventional definition of CDRs. Conventional definitions include Kabat definition (Kabat, Sequences of Proteins of Immunological Interest (National Institutes of Health, Bethesda, MD, 1987 and 1991)), Chothia definition (Chothia and Lesk, J. Mol. Biol., 196: 901-917, 1987; Chothia et al., Nature, 342: 878-883, 1989); a combination of Chothia and Kabat CDRs, wherein CDR-H1 is a combination of the Chothia CDR and the Kabat CDR; AbM definition used by Oxford Molecular's antibody modelling software; and CONTACT definition by Martin et al. (world wide web bioinfo.org.uk/abs).

In the present application, the amino acid sequences of the CDRs are all shown according to the rules of Kabat definition. However, it is well known to those skilled in the art that the CDRs of an antibody can be defined by a variety of methods in the art. In the technical solutions of the present invention, amino acid residues in variable domain sequences can also be determined according to the rules of Combined definition that incorporates both Kabat definition and Chothia definition. The rules of Combined definition refer to the combination of the ranges of Kabat definition and Chothia definition, based on which a larger scope is taken. It should be understood by those skilled in the art that unless otherwise specified, the terms "CDR" and "complementarity determining region" of a given antibody or region thereof (e.g., a variable region) should be understood to encompass the complementarity determining region as defined by any of the above-mentioned known schemes as described in the present invention. Although the scope of protection as claimed in the present invention is based on the sequences shown according to the rules of Kabat definition, corresponding amino acid sequences according to the rules of other CDR definitions shall also fall within the scope of protection of the present invention.

The term "antibody" as used herein should be understood in its broadest sense, and includes monoclonal antibodies (including full-length monoclonal antibodies), polyclonal antibodies, antibody fragments, and multispecific antibodies comprising at least two different antigen-binding regions (e.g., bispecific antibodies). Antibodies may contain additional modifications, such as non-naturally occurring amino acids, mutations in the Fc region, and mutations in glycosylation sites. Antibodies also include post-translationally modified antibodies, fusion proteins containing antigenic determinants of the antibodies, and immunoglobulin molecules containing any other modifications to antigen recognition sites, as long as these antibodies exhibit the desired biological activity.

As used herein, the term "monoclonal antibody" generally refers to an antibody obtained from a population of substantially homogeneous antibodies, that is, individual antibodies in the population are identical except for a small number of natural mutations that may be present. Monoclonal antibodies are generally highly specific for a single antigenic site. Furthermore, in contrast to a conventional polyclonal antibody preparation (which usually have different antibodies for different determinants), each monoclonal antibody is for a single determinant on the antigen. In addition to the specificity the monoclonal antibody, the monoclonal antibody has the advantage that it can be synthesised by hybridoma culture and is not contaminated by other immunoglobulins. The modifier "monoclonal" indicates the characteristic of an antibody obtained from a population of substantially homogeneous antibodies, and is not to be construed as requiring the antibody to be produced by any particular method. For example, the monoclonal antibodies used according to the present invention can be prepared in hybridoma cells, or can be prepared by the recombinant DNA method.

The term "bispecific antibody" in the context of the present invention should be understood as an antibody having two different antigen-binding regions defined by different antibody sequences. This can be understood as binding to different targets, but also includes binding to different epitopes of one target. As used herein, the term "bispecific antibody" should be understood in its broadest sense and includes full-length bispecific antibodies and antigen-binding fragments thereof. Bispecific antibodies may contain additional modifications, such as non-naturally occurring amino acids, mutations in the Fc region, and mutations in glycosylation sites. Bispecific antibodies also include post-translationally modified antibodies, fusion proteins containing antigenic determinants of the antibodies, and immunoglobulin molecules containing any other modifications to antigen recognition sites, as long as these antibodies exhibit the desired biological activity.

In the present application, the term "chimeric antibody" generally refers to an antibody in which the variable region is derived from one species and the constant region is derived from another species. Generally, the variable region is derived from an antibody of an experimental animal such as a rodent ("parent antibody"), and the constant region is derived from a human antibody, such that the resulting chimeric antibody has a reduced likelihood of eliciting an adverse immune response in a human individual compared to the parent (e.g., mouse-derived) antibody.

In the present application, the term "humanized antibody" generally refers to an antibody in which some or all of the amino acids outside the CDRs of a non-human antibody (e.g., a mouse antibody) are replaced with corresponding amino acids derived from a human immunoglobulin. Small additions, deletions, insertions, replacements or modifications of amino acids in the CDRs may also be permitted as long as they retain the ability of the antibody to bind to a particular antigen. A humanized antibody may optionally comprise at least a portion of a human immunoglobulin constant region. "Humanized antibody" retains antigen specificity similar to that of the original antibody. "Humanized" forms of non-human (e.g., murine) antibodies may be chimeric antibodies minimally comprising sequences derived from non-human immunoglobulins. In certain cases, CDR residues of a human immunoglobulin (recipient antibody) may be replaced with CDR residues from a non-human species (donor antibody) (such as mouse, rat, rabbit, or non-human primate) having the desired properties, affinity and/or capabilities. In certain cases, FR residues of a human immunoglobulin may be replaced with corresponding non-human residues. In addition, humanized antibodies may comprise amino acid modifications that are not present in the recipient antibody or in the donor antibody. These modifications may be made to further improve the properties of the antibodies, such as binding affinity.

As used herein, the term "fully human antibody" generally refers to an antibody that is expressed in a genetically engineered antibody gene-deficient animal, into which human antibody-encoding genes are transferred. All portions of the antibody, including variable and constant regions of the antibody, are encoded by humanderived genes. The fully human antibody can greatly reduce the immune side effects caused by a heterologous antibody to the human body. Methods for obtaining the fully human antibody in the art may include phage display technique, transgenic mouse technique, ribosome display technique, RNA-polypeptide technique, etc.

As used herein, the term "antigen-binding fragment" of an antibody refers to one or more fragments of the antibody that retain the ability to specifically bind to an antigen (e.g., Aβ described in the present invention). It has been demonstrated that the antigen-binding function of an antibody can be performed by fragments of a full-length antibody.

Examples of the term "antigen-binding fragment" of an antibody include: (i) a Fab fragment, which is a monovalent fragment consisting of VL, VH, CL and CH1 domains; (ii) an F(ab')₂ fragment, which is a divalent fragment comprising two Fab fragments linked via disulphide bonds in the hinge region; (iii) a Fab' fragment, which is essentially an Fab with a portion of the hinge region; (iv) an Fd fragment, which consists of VH and CH1 domains; (v) an Fd' fragment, which has VH and CH1 domains and one or more cysteine residues at the C-terminus of the CH1 domain; (vi) an Fv fragment, which consists of VL and VH domains of a single arm of an antibody; (vii) a dAb fragment, which consists of a VH domain; (viii) a separate complementarity determining region (CDR); (ix) a nanobody, which is a heavy chain variable region containing a single variable domain and two constant domains. Furthermore, although the two domains of the Fv fragment, VL and VH, are encoded by different genes, they can be linked via a synthetic linker using recombinant methods, to form a single protein chain, in which the VL and VH regions pair to form a monovalent molecule (referred to as a single-chain Fv (scFv)). Such single-chain antibodies are also intended to be encompassed within the term "antigen-binding fragment" of an antibody. In addition, the term further includes "linear antibodies" comprising a pair of tandem Fd fragments (VH-CH1-VH-CH1) which, together with complementary light chain polypeptides and any modified forms of the above fragments that retain antigen-binding activity, form antigen-binding regions.

These antigen-binding fragments can be obtained using conventional techniques known to those skilled in the art and are screened for utility in the same manner as for intact antibodies.

As used herein, the term "binding" or "specific binding" refers to a non-random binding reaction between two molecules, such as between an antibody and a target antigen thereof. The binding specificity of an antibody can be determined on the basis of affinity and/or avidity. Affinity is expressed by the equilibrium dissociation constant (KD) of an antigen from an antibody, and is a measure of the binding strength between an antigenic determinant and the antigen-binding site of the antibody: the smaller the value of KD, the stronger the binding strength between the antigenic determinant and the antibody. Alternatively, affinity can also be expressed as the affinity constant (KA), which is 1/KD. Avidity is a measure of the binding strength between an antibody and a cognate antigen. Avidity involves the affinity between an antigenic determinant and the antigen-binding site of an antibody and the number of relevant binding sites present on the antibody.

The specific binding of an antibody to an antigen or an antigenic determinant can be determined in any known suitable manner, including, for example, Scatchard analysis and/or competitive binding assays such as radioimmunoassay (RIA), enzyme immunoassay (EIA) and sandwich competition assay, and various variations thereof known in the art.

In the present invention, the term "KD" generally refers to the equilibrium dissociation constant. "KD" is the ratio of the dissociation rate constant (kdis, also referred to as "off-rate (koff) " or "kd") to the association rate constant (kon, also referred to as "on-rate (kon)" or "ka"). The binding affinity of an antigen-binding protein (e.g., an antibody) for an antigen can be expressed using the association rate constant (kon), dissociation rate constant (kdis) and equilibrium dissociation constant (KD). Methods for determining association and dissociation rate constants are well known in the art, including but not limited to bio-layer interferometry (BLI), radioimmunoassay (RIA), equilibrium dialysis, surface plasmon resonance (SPR), fluorescence resonance energy transfer (FRET), co-immunoprecipitation (Co-IP) and protein chip technology. The measured affinity of a particular protein-protein interaction may be different if measured under different conditions (e.g., salt concentration or pH).

The term "epitope" refers to a site on an antigen to which an antibody binds. Epitopes can be formed by contiguous amino acids or by non-contiguous amino acids juxtaposed by the tertiary folding of one or more proteins. Epitopes formed by contiguous amino acids (also referred to as linear epitopes) are generally retained upon exposure to denaturing solvents, whereas epitopes formed by tertiary folding (also referred to as conformational epitopes) are generally lost upon treatment with denaturing solvents. An epitope generally comprises at least 3, more generally at least 5 or 8-10 amino acids, in a unique spatial conformation. An epitope defines the minimum binding site of an antibody and is therefore the specific target for the antibody or an antigen-binding fragment thereof.

As used herein, the terms "nucleic acid", "nucleic acid molecule", "nucleic acid sequence", "nucleotide sequence" and "polynucleotide" are used interchangeably and refer to a polymerized form of nucleotides (ribonucleotides or deoxyribonucleotides) of any length. Therefore, the term includes, but is not limited to, single-stranded, double-stranded or double-stranded DNA or RNA, genomic DNA, cDNA, a DNA-RNA hybrid, or a polymer comprising, consisting of, or consisting essentially of purine and pyrimidine bases, or other natural, chemically or biochemically modified, non-natural or derived nucleoside bases.

The terms "protein", "peptide", "polypeptide" and "amino acid sequence" are used interchangeably and, in their broadest sense, refer to a polymerized form of two or more amino acid subunits, amino acid analogues or peptidomimetics. "Protein", "peptide", "polypeptide" and "amino acid sequence" contain at least two amino acids, with no restriction on the maximum number of amino acids. As used herein, the term "amino acid" refers to natural and/or non-natural or synthetic amino acids, including D and L optical isomers and amino acid analogues.

As used herein, "expression" refers to the process by which a nucleic acid sequence is transcribed into mRNA and/or the subsequent process by which the transcribed mRNA is translated into a peptide, polypeptide, an amino acid sequence or a protein. If the nucleic acid sequence is derived from genomic DNA, expression may include the splicing of mRNA in eukaryotic cells.

The term "encoding", when applied to a nucleic acid sequence, means that the nucleic acid sequence "encodes" a polypeptide if, in its native state or when manipulated by methods well known to those skilled in the art, it can be transcribed to produce mRNA and/or translated to produce a polypeptide. The antisense strand is the complement of such a nucleic acid, and the coding sequence can be derived therefrom.

The term "sequence identity" refers to the sequence similarity between two polypeptides or between two nucleic acid sequences. Percent identity can be determined by comparing positions in each sequence, which sequences can be aligned for the purpose of comparison. When a position in the compared sequence is occupied by the same base or amino acid, the molecule is identical at that position. The degree of identity between sequences depends on the number of matching positions shared. An "unrelated" or "non-homologous" sequence shares less than 40% identity, or less than 25% identity, with one of the sequences of the present invention. The alignment and percentage of sequence identity of the nucleic acid or amino acid sequences provided herein can be determined by importing the nucleic acid or amino acid sequences into ClustalW (available at https://genome.jp/tools-bin/clustalw/) and using the ClustalW.

When referring to particular molecules, biological materials or cellular substances, the terms "equivalent" or "functional variant" are used interchangeably and refer to those that have minimal homology while still retaining the desired structure or function. Non-limiting examples of equivalent polypeptides include polypeptides having at least about 60%, at least about 65%, at least about 70%, at least about 75%, at least about 80%, at least about 85%, at least about 90%, at least about 95%, at least about 96%, at least about 97%, at least about 98% or at least about 99% identity to a reference polypeptide (e.g., the antibody or the antigen-binding fragment thereof described in the present invention); or polypeptides encoded by polynucleotides having at least about 70%, at least about 75%, at least about 80%, at least about 85%, at least about 90%, at least about 95%, at least about 96%, at least about 97%, at least about 98% or at least about 99% identity to a reference polynucleotide (e.g., a polynucleotide encoding the antibody or the antigen-binding fragment thereof described in the present invention).

Equivalents having one or more amino acid modifications compared to the antibody or the antigen-binding fragment thereof described herein are also encompassed within the scope of the present invention, provided that the one or more amino acid modifications do not affect or do not substantially affect the ability of the antibody or the antigen-binding fragment thereof to specifically bind to an antigen. As used herein, an amino acid modification may be an amino acid substitution, an amino acid deletion or an amino acid insertion. An amino acid substitution may be a conservative amino acid substitution or a non-conservative amino acid substitution. A conservative substitution (also referred to as a conservative mutation, a conservative replacement or a conservative variation) is an amino acid replacement in a protein that changes a given amino acid to a different amino acid with similar biochemical properties (e.g., charge, hydrophobicity or size). As used herein, "conservative substitution" refers to the replacement of an amino acid residue with another biologically similar residue. Examples of conservative substitutions include the substitution of one hydrophobic residue with another hydrophobic residue, such as isoleucine, valine, leucine or methionine; or the substitution of one charged or polar residue with another charged or polar residue, such as substitution of lysine with arginine, substitution of aspartic acid with glutamic acid, and substitution of asparagine with glutamine. Other exemplary examples of conservative substitutions include the following changes: alanine to serine; asparagine to glutamine or histidine; aspartic acid to glutamic acid; cysteine to serine; glycine to proline; histidine to asparagine or glutamine; lysine to arginine, glutamine or glutamic acid; phenylalanine to tyrosine, serine to threonine; threonine to serine; tryptophan to tyrosine; tyrosine to tryptophan or phenylalanine; etc.

As used herein, the term "subject" generally refers to a mammal. Mammals include, but are not limited to, domesticated animals (e.g., cows, sheep, cats, dogs and horses), primates (e.g., humans and non-human primates such as monkeys), rabbits and rodents (e.g., mice and rats). The term "primate" generally refers to monkey and ape species, and includes monkey species such as monkeys from the genus *Macaca* (such as cynomolgus monkey (*Macaca fascicularis*) and/or rhesus monkey (*Macaca mulatta*)) and baboons (*Papio ursinus*), as well as marmosets (species from the genus *Callithrix*), squirrel monkeys (species from the genus *Saimiri*) and tamarins (species from the genus *Saguinus*), and ape species such as chimpanzees (*Pan troglodytes*), and also includes *Homo sapiens*.

The term "vector" generally refers to a nucleic acid molecule capable of selfreplication in a suitable host, which transfers the inserted nucleic acid molecule into and/or between host cells. The vector may include a vector primarily used for inserting DNA or RNA into a cell, a vector primarily used for replicating DNA or RNA, and a vector primarily used for the transcriptional and/or translational expression of DNA or RNA. The vector also includes a vector having a variety of the above functions. The vector may be a polynucleotide that can be transcribed and translated into a polypeptide when introduced into a suitable host cell. Generally, the vector may produce a desired expression product by means of culturing a suitable host cell containing the vector.

As used herein, the term "cell" generally refers to an individual cell, cell line or cell culture that may contain or has contained a plasmid or vector containing the nucleic acid molecule of the present invention, or can express the antibody or the antigen-binding fragment thereof of the present invention. The cell may comprise the progeny of a single host cell. Due to natural, accidental or intentional mutations, progeny cells may not necessarily be completely identical to original parent cells in terms of morphology or genome, as long as they can express the antibody or the antigen-binding fragment thereof of the present application. The cell may be obtained by means of transfecting a cell *in vitro* using the vector of the present application. The cell may be a prokaryotic cell (e.g., *Escherichia coli*), or a eukaryotic cell (e.g., a yeast cell, e.g., a COS cell, a Chinese hamster ovary (CHO) cell, a HeLa cell, an HEK293 cell, a COS-1 cell, an NS0 cell or a myeloma cell). In certain cases, the cell may be a mammalian cell. For example, the mammalian cell may be a CHO-K1 cell. As used herein, the term "recombinant cell" generally refers to a cell into which a recombinant expression vector has been introduced. The recombinant host cell includes not only particular cells but also progeny of such cells.

The term "pharmaceutical composition" generally refers to a preparation that is present in a form allowing the biological activity of the active ingredient to be effective and that does not contain additional ingredients having unacceptable toxicity to a subject to which the composition is to be administered. The composition is sterile. A "sterile" composition is sterilized or free of all living microorganisms and spores thereof.

The term "kit" generally refers to a packaged product comprising components for administering the antibody or the antigen-binding fragment thereof of the present invention to treat or prevent a relevant disease caused by abnormal accumulation or deposition of amyloid. The components of the kit may be contained in separate vials (i.e., a kit with separate parts) or provided within a single vial. A kit may comprise reagents such as a buffer, a protein stabilizing reagent, a signal generating system (e.g., a fluorescent signal generating system), an antibody, a control protein and a test container. The kit may further comprise instructions for performing the method. In some embodiments of the present invention, the kit may further comprise an administration device capable of administering to a subject a pharmaceutically active ingredient of the kit, such as the antibody or the antigen-binding fragment thereof of the present invention, in a suitable manner.

As used herein, the term "administration" is intended to mean delivering a substance to a subject, such as an animal or a human. Administration can be performed in a single dose, continuously or intermittently throughout the entire treatment process. Methods for determining the most effective administration method and dosage are known to those skilled in the art, and will vary depending on factors such as the composition used for treatment, the purpose of treatment, and the age, health status or gender of the subject being treated. In some embodiments, single or multiple administrations may be performed, with the dose level and pattern selected by a physician, or in the case of pets and other animals, by a veterinarian.

The term "disease caused by abnormal accumulation or deposition of Aβ" refers to a disease pathologically characterized by Aβ deposits in the brain or cerebrovascular system. This includes diseases such as Alzheimer's disease, Down syndrome and cerebral amyloid angiopathy. The clinical diagnosis, staging or progression of Alzheimer's disease can be readily determined by the attending diagnostic physician or health care professional as those skilled in the art through the use of known techniques and observational findings. This generally includes some forms of brain plaque imaging, mental or cognitive assessments (e.g., Clinical Dementia Rating-summary of boxes (CDR-SB), Mini-Mental State Exam (MMSE) or Alzheimer's Disease Assessment Scale-Cognitive (ADAS-Cog)), or functional assessments (e.g., Alzheimer's Disease Cooperative Study-Activities of Daily Living (ADCS-ADL)). Cognitive and functional assessments can be used to determine changes in the cognition (e.g., cognitive decline) and function (e.g., functional decline) of a patient.

As used herein, "clinical Alzheimer's disease" or "clinical AD" is the diagnostic stage of Alzheimer's disease. The clinical Alzheimer's disease includes conditions diagnosed as prodromal Alzheimer's disease, mild Alzheimer's disease, moderate Alzheimer's disease and severe Alzheimer's disease. The term "preclinical Alzheimer's disease" or "preclinical AD" is the stage prior to clinical Alzheimer's disease, in which measurable changes in biomarkers (such as CSF Aβ42 levels or brain plaque deposition via amyloid PET) indicate the earliest signs of progression to clinical Alzheimer's disease in patients with Alzheimer's pathology. This stage generally occurs before symptoms such as memory loss and confusion become significant. Preclinical Alzheimer's disease further includes presymptomatic autosomal dominant carriers and patients at high risk of developing AD due to carrying one or two APOE e4 alleles.

As used herein, "treatment" includes limiting, slowing or stopping the progression or severity of existing symptoms, conditions, diseases or disorders in a patient. As understood in the art, "treatment" is an approach for obtaining beneficial or desired results, including clinical outcomes. For the purposes of the present invention, beneficial or desired results may include reducing the severity of one or more diseases or conditions, delaying or slowing the progression of the diseases or conditions, and improving or alleviating the status of the diseases or conditions (whether partially or completely). The term "prevention" means that the antibody or the antigen-binding fragment thereof of the present invention is prophylactically administered to an asymptomatic patient or a patient with preclinical Alzheimer's disease to prevent the onset or progression of the disease.

### Anti-Aβ antibody

The amyloid hypothesis is currently the most widely accepted explanation for the primary cause of AD occurrence. This theory holds that Aβ deposited around neurons causes neuronal loss by inducing apoptosis. The β-sheet structure of Aβ molecules can promote the aggregation of Aβ into insoluble fibres, which are resistant to protease degradation and further form extremely insoluble precipitates, thereby producing neurotoxicity. Currently, the preventive and therapeutic measures targeting the neurotoxic effects of Aβ mainly include: reducing the production of Aβ; promoting the degradation and clearance of Aβ; preventing the aggregation and deposition of Aβ, etc.

Aβ is formed by the cleavage of amyloid protein precursor (APP) by β-secretase and γ-secretase. Aβ exists in multiple subtypes, e.g., Aβ1-42, Aβ1-40, and pyroE Aβ3-42. pyroE Aβ3-42 is a truncated form of Aβ, which lacks the first two amino acid residues at the N-terminus of Aβ and has a pyroglutamate modification derived from glutamic acid at the third amino acid position. Studies have found that deposits in the plaques of human patients are composed of a heterogeneous mixture of Aβ peptides, and pyroE Aβ3-42 has been demonstrated to have aggressive aggregation properties and accumulate early in the deposition cascade.

The amino acid sequence of human Aβ1-42 is: DAEFRHDSGYEVHHQKLVFFAEDVGSNKGAIIGLMVGGVVIA (SEQ ID NO: 280).

In one aspect, the present invention provides an antibody or an antigen-binding fragment thereof that binds to β-amyloid (Aβ), the antibody comprising a heavy chain variable region (VH) and a light chain variable region (VL), wherein
(1) the VH comprises a heavy chain complementarity determining region (HCDR) 1 as set forth in SEQ ID NO: 23, a HCDR2 as set forth in SEQ ID NO: 24, and a HCDR3 as set forth in SEQ ID NO: 25, and the VL comprises a light chain complementarity determining region (LCDR) 1 as set forth in SEQ ID NO: 28, a LCDR2 as set forth in SEQ ID NO: 29, and a LCDR3 as set forth in SEQ ID NO: 30;
(2) the VH comprises a HCDR1 as set forth in SEQ ID NO: 69, a HCDR2 as set forth in SEQ ID NO: 70, and a HCDR3 as set forth in SEQ ID NO: 71, and the VL comprises a LCDR1 as set forth in SEQ ID NO: 74, a LCDR2 as set forth in SEQ ID NO: 248, and a LCDR3 as set forth in SEQ ID NO: 76;
(3) the VH comprises a HCDR1 as set forth in SEQ ID NO: 69, a HCDR2 as set forth in SEQ ID NO: 70, and a HCDR3 as set forth in SEQ ID NO: 71, and the VL comprises a LCDR1 as set forth in SEQ ID NO: 74, a LCDR2 as set forth in SEQ ID NO: 75, and a LCDR3 as set forth in SEQ ID NO: 76;
(4) the VH comprises a HCDR1 as set forth in SEQ ID NO: 3, a HCDR2 as set forth in SEQ ID NO: 4, and a HCDR3 as set forth in SEQ ID NO: 5, and the VL comprises a LCDR1 as set forth in SEQ ID NO: 8, a LCDR2 as set forth in SEQ ID NO: 9, and a LCDR3 as set forth in SEQ ID NO: 10;
(5) the VH comprises a HCDR1 as set forth in SEQ ID NO: 13, a HCDR2 as set forth in SEQ ID NO: 14, and a HCDR3 as set forth in SEQ ID NO: 15, and the VL comprises a LCDR1 as set forth in SEQ ID NO: 18, a LCDR2 as set forth in SEQ ID NO: 19, and a LCDR3 as set forth in SEQ ID NO: 20;
(6) the VH comprises a HCDR1 as set forth in SEQ ID NO: 33, a HCDR2 as set forth in SEQ ID NO: 34, and a HCDR3 as set forth in SEQ ID NO: 35, and the VL comprises a LCDR1 as set forth in SEQ ID NO: 38, a LCDR2 as set forth in SEQ ID NO: 19, and a LCDR3 as set forth in SEQ ID NO: 20;
(7) the VH comprises a HCDR1 as set forth in SEQ ID NO: 41, a HCDR2 as set forth in SEQ ID NO: 42, and a HCDR3 as set forth in SEQ ID NO: 43, and the VL comprises a LCDR1 as set forth in SEQ ID NO: 46, a LCDR2 as set forth in SEQ ID NO: 47, and a LCDR3 as set forth in SEQ ID NO: 48;
(8) the VH comprises a HCDR1 as set forth in SEQ ID NO: 51, a HCDR2 as set forth in SEQ ID NO: 52, and a HCDR3 as set forth in SEQ ID NO: 53, and the VL comprises a LCDR1 as set forth in SEQ ID NO: 56, a LCDR2 as set forth in SEQ ID NO: 19, and a LCDR3 as set forth in SEQ ID NO: 20;
(9) the VH comprises a HCDR1 as set forth in SEQ ID NO: 59, a HCDR2 as set forth in SEQ ID NO: 60, and a HCDR3 as set forth in SEQ ID NO: 61, and the VL comprises a LCDR1 as set forth in SEQ ID NO: 64, a LCDR2 as set forth in SEQ ID NO: 65, and a LCDR3 as set forth in SEQ ID NO: 66;
(10) the VH comprises a HCDR1 as set forth in SEQ ID NO: 3, a HCDR2 as set forth in SEQ ID NO: 4, and a HCDR3 as set forth in SEQ ID NO: 79, and the VL comprises a LCDR1 as set forth in SEQ ID NO: 82, a LCDR2 as set forth in SEQ ID NO: 83, and a LCDR3 as set forth in SEQ ID NO: 84;
(11) the VH comprises a HCDR1 as set forth in SEQ ID NO: 87, a HCDR2 as set forth in SEQ ID NO: 88, and a HCDR3 as set forth in SEQ ID NO: 89, and the VL comprises a LCDR1 as set forth in SEQ ID NO: 92, a LCDR2 as set forth in SEQ ID NO: 93, and a LCDR3 as set forth in SEQ ID NO: 94;
(12) the VH comprises a HCDR1 as set forth in SEQ ID NO: 130, a HCDR2 as set forth in SEQ ID NO: 131, and a HCDR3 as set forth in SEQ ID NO: 279, and the VL comprises a LCDR1 as set forth in SEQ ID NO: 74, a LCDR2 as set forth in SEQ ID NO: 134, and a LCDR3 as set forth in SEQ ID NO: 76;
(13) the VH comprises a HCDR1 as set forth in SEQ ID NO: 59, a HCDR2 as set forth in SEQ ID NO: 137, and a HCDR3 as set forth in SEQ ID NO: 138, and the VL comprises a LCDR1 as set forth in SEQ ID NO: 141, a LCDR2 as set forth in SEQ ID NO: 65, and a LCDR3 as set forth in SEQ ID NO: 66;
(14) the VH comprises a HCDR1 as set forth in SEQ ID NO: 121, a HCDR2 as set forth in SEQ ID NO: 122, and a HCDR3 as set forth in SEQ ID NO: 144, and the VL comprises a LCDR1 as set forth in SEQ ID NO: 147, a LCDR2 as set forth in SEQ ID NO: 148, and a LCDR3 as set forth in SEQ ID NO: 149;
(15) the VH comprises a HCDR1 as set forth in SEQ ID NO: 121, a HCDR2 as set forth in SEQ ID NO: 122, and a HCDR3 as set forth in SEQ ID NO: 123, and the VL comprises a LCDR1 as set forth in SEQ ID NO: 218, a LCDR2 as set forth in SEQ ID NO: 65, and a LCDR3 as set forth in SEQ ID NO: 127;
(16) the VH comprises a HCDR1 as set forth in SEQ ID NO: 121, a HCDR2 as set forth in SEQ ID NO: 122, and a HCDR3 as set forth in SEQ ID NO: 123, and the VL comprises a LCDR1 as set forth in SEQ ID NO: 126, a LCDR2 as set forth in SEQ ID NO: 65, and a LCDR3 as set forth in SEQ ID NO: 127;
(17) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 103, and a LCDR3 as set forth in SEQ ID NO: 104;
(18) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 235, and a LCDR3 as set forth in SEQ ID NO: 104;
(19) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 238, and a LCDR3 as set forth in SEQ ID NO: 104;
(20) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 245, and a LCDR3 as set forth in SEQ ID NO: 104;
(21) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 102, a LCDR2 as set forth in SEQ ID NO: 103, and a LCDR3 as set forth in SEQ ID NO: 104;
(22) the VH comprises a HCDR1 as set forth in SEQ ID NO: 107, a HCDR2 as set forth in SEQ ID NO: 108, and a HCDR3 as set forth in SEQ ID NO: 99, and the VL comprises a LCDR1 as set forth in SEQ ID NO: 102, a LCDR2 as set forth in SEQ ID NO: 103, and a LCDR3 as set forth in SEQ ID NO: 111; or
(23) the VH comprises a HCDR1 as set forth in SEQ ID NO: 114, a HCDR2 as set forth in SEQ ID NO: 98, and a HCDR3 as set forth in SEQ ID NO: 115, and the VL comprises a LCDR1 as set forth in SEQ ID NO: 102; a LCDR2 as set forth in SEQ ID NO: 118, and a LCDR3 as set forth in SEQ ID NO: 104.

In some embodiments, the antibody or the antigen-binding fragment thereof of the present invention binds to pyroE Aβ3-42 peptide. In some embodiments, the antibody or the antigen-binding fragment thereof of the present invention binds to Aβ1-42 protofibril.

In some embodiments, the VH and VL of the antibody comprise the following amino acid sequences, respectively:
(1) the VH and VL comprise amino acid sequences that have at least 80%, at least 85%, at least 90%, at least 95%, at least 96%, at least 97%, at least 98%, at least 99% or 100% sequence identity to SEQ ID NO: 164 and SEQ ID NO: 219, respectively;
(2) the VH and VL comprise amino acid sequences that have at least 80%, at least 85%, at least 90%, at least 95%, at least 96%, at least 97%, at least 98%, at least 99% or 100% sequence identity to SEQ ID NO: 166 and SEQ ID NO: 219, respectively;
(3) the VH and VL comprise amino acid sequences that have at least 80%, at least 85%, at least 90%, at least 95%, at least 96%, at least 97%, at least 98%, at least 99% or 100% sequence identity to SEQ ID NO: 174 and SEQ ID NO: 219, respectively;
(4) the VH and VL comprise amino acid sequences that have at least 80%, at least 85%, at least 90%, at least 95%, at least 96%, at least 97%, at least 98%, at least 99% or 100% sequence identity to SEQ ID NO: 176 and SEQ ID NO: 219, respectively;
(5) the VH and VL comprise amino acid sequences that have at least 80%, at least 85%, at least 90%, at least 95%, at least 96%, at least 97%, at least 98%, at least 99% or 100% sequence identity to SEQ ID NO: 21 and SEQ ID NO: 26, respectively;
(6) the VH and VL comprise amino acid sequences that have at least 80%, at least 85%, at least 90%, at least 95%, at least 96%, at least 97%, at least 98%, at least 99% or 100% sequence identity to SEQ ID NO: 197 and SEQ ID NO: 246, respectively;
(7) the VH and VL comprise amino acid sequences that have at least 80%, at least 85%, at least 90%, at least 95%, at least 96%, at least 97%, at least 98%, at least 99% or 100% sequence identity to SEQ ID NO: 197 and SEQ ID NO: 249, respectively;
(8) the VH and VL comprise amino acid sequences that have at least 80%, at least 85%, at least 90%, at least 95%, at least 96%, at least 97%, at least 98%, at least 99% or 100% sequence identity to SEQ ID NO: 67 and SEQ ID NO: 72, respectively;
(9) the VH and VL comprise amino acid sequences that have at least 80%, at least 85%, at least 90%, at least 95%, at least 96%, at least 97%, at least 98%, at least 99% or 100% sequence identity to SEQ ID NO: 1 and SEQ ID NO: 6, respectively;
(10) the VH and VL comprise amino acid sequences that have at least 80%, at least 85%, at least 90%, at least 95%, at least 96%, at least 97%, at least 98%, at least 99% or 100% sequence identity to SEQ ID NO: 11 and SEQ ID NO: 16, respectively;
(11) the VH and VL comprise amino acid sequences that have at least 80%, at least 85%, at least 90%, at least 95%, at least 96%, at least 97%, at least 98%, at least 99% or 100% sequence identity to SEQ ID NO: 31 and SEQ ID NO: 36, respectively;
(12) the VH and VL comprise amino acid sequences that have at least 80%, at least 85%, at least 90%, at least 95%, at least 96%, at least 97%, at least 98%, at least 99% or 100% sequence identity to SEQ ID NO: 39 and SEQ ID NO: 44, respectively;
(13) the VH and VL comprise amino acid sequences that have at least 80%, at least 85%, at least 90%, at least 95%, at least 96%, at least 97%, at least 98%, at least 99% or 100% sequence identity to SEQ ID NO: 49 and SEQ ID NO: 54, respectively;
(14) the VH and VL comprise amino acid sequences that have at least 80%, at least 85%, at least 90%, at least 95%, at least 96%, at least 97%, at least 98%, at least 99% or 100% sequence identity to SEQ ID NO: 57 and SEQ ID NO: 62, respectively;
(15) the VH and VL comprise amino acid sequences that have at least 80%, at least 85%, at least 90%, at least 95%, at least 96%, at least 97%, at least 98%, at least 99% or 100% sequence identity to SEQ ID NO: 77 and SEQ ID NO: 80, respectively;
(16) the VH and VL comprise amino acid sequences that have at least 80%, at least 85%, at least 90%, at least 95%, at least 96%, at least 97%, at least 98%, at least 99% or 100% sequence identity to SEQ ID NO: 85 and SEQ ID NO: 90, respectively;
(17) the VH and VL comprise amino acid sequences that have at least 80%, at least 85%, at least 90%, at least 95%, at least 96%, at least 97%, at least 98%, at least 99% or 100% sequence identity to SEQ ID NO: 128 and SEQ ID NO: 132, respectively;
(18) the VH and VL comprise amino acid sequences that have at least 80%, at least 85%, at least 90%, at least 95%, at least 96%, at least 97%, at least 98%, at least 99% or 100% sequence identity to SEQ ID NO: 135 and SEQ ID NO: 139, respectively;
(19) the VH and VL comprise amino acid sequences that have at least 80%, at least 85%, at least 90%, at least 95%, at least 96%, at least 97%, at least 98%, at least 99% or 100% sequence identity to SEQ ID NO: 142 and SEQ ID NO: 145, respectively;
(20) the VH and VL comprise amino acid sequences that have at least 80%, at least 85%, at least 90%, at least 95%, at least 96%, at least 97%, at least 98%, at least 99% or 100% sequence identity to SEQ ID NO: 160 and SEQ ID NO: 216, respectively;
(21) the VH and VL comprise amino acid sequences that have at least 80%, at least 85%, at least 90%, at least 95%, at least 96%, at least 97%, at least 98%, at least 99% or 100% sequence identity to SEQ ID NO: 152 and SEQ ID NO: 216, respectively;
(22) the VH and VL comprise amino acid sequences that have at least 80%, at least 85%, at least 90%, at least 95%, at least 96%, at least 97%, at least 98%, at least 99% or 100% sequence identity to SEQ ID NO: 156 and SEQ ID NO: 216, respectively;
(23) the VH and VL comprise amino acid sequences that have at least 80%, at least 85%, at least 90%, at least 95%, at least 96%, at least 97%, at least 98%, at least 99% or 100% sequence identity to SEQ ID NO: 158 and SEQ ID NO: 216, respectively;
(24) the VH and VL comprise amino acid sequences that have at least 80%, at least 85%, at least 90%, at least 95%, at least 96%, at least 97%, at least 98%, at least 99% or 100% sequence identity to SEQ ID NO: 162 and SEQ ID NO: 216, respectively;
(25) the VH and VL comprise amino acid sequences that have at least 80%, at least 85%, at least 90%, at least 95%, at least 96%, at least 97%, at least 98%, at least 99% or 100% sequence identity to SEQ ID NO: 160 and SEQ ID NO: 214, respectively;
(26) the VH and VL comprise amino acid sequences that have at least 80%, at least 85%, at least 90%, at least 95%, at least 96%, at least 97%, at least 98%, at least 99% or 100% sequence identity to SEQ ID NO: 152 and SEQ ID NO: 214, respectively;
(27) the VH and VL comprise amino acid sequences that have at least 80%, at least 85%, at least 90%, at least 95%, at least 96%, at least 97%, at least 98%, at least 99% or 100% sequence identity to SEQ ID NO: 162 and SEQ ID NO: 214, respectively;
(28) the VH and VL comprise amino acid sequences that have at least 80%, at least 85%, at least 90%, at least 95%, at least 96%, at least 97%, at least 98%, at least 99% or 100% sequence identity to SEQ ID NO: 119 and SEQ ID NO: 124, respectively;
(29) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 230, respectively;
(30) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 233, respectively;
(31) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 236, respectively;
(32) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 239, respectively;
(33) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 241, respectively;
(34) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 243, respectively;
(35) the VH and VL comprise amino acid sequences that have at least 80%, at least 85%, at least 90%, at least 95%, at least 96%, at least 97%, at least 98%, at least 99% or 100% sequence identity to SEQ ID NO: 95 and SEQ ID NO: 100, respectively;
(36) the VH and VL comprise amino acid sequences that have at least 80%, at least 85%, at least 90%, at least 95%, at least 96%, at least 97%, at least 98%, at least 99% or 100% sequence identity to SEQ ID NO: 105 and SEQ ID NO: 109, respectively; or
(37) the VH and VL comprise amino acid sequences that have at least 80%, at least 85%, at least 90%, at least 95%, at least 96%, at least 97%, at least 98%, at least 99% or 100% sequence identity to SEQ ID NO: 112 and SEQ ID NO: 116, respectively.

In some embodiments, the VH and VL of the anti-Aβ antibody include functional variants formed by insertion, deletion and/or substitution of one or more amino acids therein compared to the VH and VL of a reference antibody (e.g., an anti-Aβ antibody comprising the VH and VL shown herein, such as an antibody comprising the VH as set forth in SEQ ID NO: 164 and the VL as set forth in SEQ ID NO: 219), provided that the functional variants retain the ability to bind to Aβ (e.g., Aβ1-42 protofibril and/or pyroE Aβ3-42 peptide).

The functional variants comprise or consist of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the reference antibody.

In the context of functional variants, the number of amino acids inserted, deleted and/or substituted preferably does not exceed 40%, more preferably does not exceed 35%, more preferably 1% to 33%, and more preferably 5% to 30%, more preferably 10% to 25%, more preferably 15% to 20% of the total number of amino acids in the amino acid sequence of the reference antibody. For example, the number of amino acids inserted, deleted and/or substituted may be 1 to 20, preferably 1 to 10, more preferably 1 to 7, still more preferably 1 to 5, and most preferably 1 to 2. In preferred embodiments, the number of amino acids inserted, deleted and/or substituted is 1, 2, 3, 4, 5, 6 or 7.

In some embodiments, insertions, deletions and/or substitutions may be in framework (FR) regions, e.g., in FR1, FR2, FR3 and/or FR4.

In some embodiments, insertions, deletions and/or substitutions may be in CDR regions, e.g., CDR1, CDR2 and/or CDR3, provided that the insertions, deletions and/or substitutions do not affect or do not substantially affect the ability of the antibody or the antigen-binding fragment thereof to bind to Aβ (e.g., Aβ1-42 protofibril and/or pyroE Aβ3-42 peptide).

In some embodiments, the substitution of one or more amino acids may be a conservative substitution of one or more amino acids. Examples of conservative substitutions include the substitution of one hydrophobic residue with another hydrophobic residue, such as isoleucine, valine, leucine or methionine; or the substitution of one charged or polar residue with another charged or polar residue, such as substitution of lysine with arginine, substitution of aspartic acid with glutamic acid, and substitution of asparagine with glutamine. Preferred exemplary examples of conservative substitutions include the following changes: alanine to serine; asparagine to glutamine or histidine; aspartic acid to glutamic acid; cysteine to serine; glycine to proline; histidine to asparagine or glutamine; lysine to arginine, glutamine or glutamic acid; phenylalanine to tyrosine, serine to threonine; threonine to serine; tryptophan to tyrosine; tyrosine to tryptophan or phenylalanine; etc.

In some preferred embodiments, the anti-Aβ antibody comprises a VH comprising the amino acid sequence as set forth in SEQ ID NO: 164 and a VL comprising the amino acid sequence as set forth in SEQ ID NO: 219. In some preferred embodiments, the anti-Aβ antibody comprises a VH comprising the amino acid sequence as set forth in SEQ ID NO: 160 and a VL comprising the amino acid sequence as set forth in SEQ ID NO: 216.

On the basis of the amino acid sequence of the heavy chain constant region of an antibody, immunoglobulin molecules can be divided into five classes (isotypes): IgA, IgD, IgE, IgG and IgM, and can be further divided into different subclasses, such as IgG1, IgG2, IgG3, IgG4, IgA1 and IgA2. On the basis of the amino acid sequences of the light chains, the light chains of an antibody can be divided into a lambda (λ) chain and a kappa (κ) chain. The antibodies disclosed herein may be of any of the above classes or subclasses.

In some embodiments, the antibody is of an antibody isotype selected from: IgG, IgA, IgM, IgE and IgD. In some embodiments, the antibody is of an antibody subclass selected from: IgG1, IgG2, IgG3 and IgG4.

In some embodiments, the antibody of the present invention comprises an Fc region. The Fc region may be of any isotype or subtype, including but not limited to IgG1, IgG2, IgG3 and IgG4, and may comprise one or more substitutions or modifications. In some embodiments, the Fc region is an IgG1 Fc region or is derived therefrom, optionally with one or more substitutions or modifications. In some embodiments, the Fc region comprises one or more amino acid substitutions that extend the half-life of the antibody. In preferred embodiments, the Fc region comprises L234A and L235A amino acid substitutions.

In some embodiments, the anti-Aβ antibody of the present invention comprises a heavy chain (HC) and a light chain (LC), wherein
(1) the HC and LC comprise amino acid sequences that have at least 80%, at least 85%, at least 90%, at least 95%, at least 96%, at least 97%, at least 98%, at least 99% or 100% sequence identity to SEQ ID NO: 275 and SEQ ID NO: 277, respectively; or
(2) the HC and LC comprise amino acid sequences that have at least 80%, at least 85%, at least 90%, at least 95%, at least 96%, at least 97%, at least 98%, at least 99% or 100% sequence identity to SEQ ID NO: 271 and SEQ ID NO: 273, respectively.

In some embodiments, the HC and LC of the anti-Aβ antibody include functional variants formed by insertion, deletion and/or substitution of one or more amino acids therein compared to the HC and LC of a reference antibody (e.g., an antibody comprising a HC having the amino acid sequence as set forth in SEQ ID NO: 275 and a LC having the amino acid sequence as set forth in SEQ ID NO: 277, or an antibody comprising a HC having the amino acid sequence as set forth in SEQ ID NO: 271 and a LC having the amino acid sequence as set forth in SEQ ID NO: 273), provided that the functional variants retain the ability to bind to Aβ (e.g., Aβ1-42 protofibril and/or pyroE Aβ3-42 peptide).

The functional variants comprise or consist of an amino acid sequence having at least 80%, at least 85%, at least 90%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to the amino acid sequence of the reference antibody.

In some embodiments, the number of amino acids inserted, deleted and/or substituted preferably does not exceed 40%, more preferably does not exceed 35%, more preferably 1% to 33%, and more preferably 5% to 30%, more preferably 10% to 25%, more preferably 15% to 20%, of the total number of amino acids in the amino acid sequence of the reference antibody. For example, the number of amino acids inserted, deleted and/or substituted may be 1 to 50, preferably 1 to 20, more preferably 1 to 10, still more preferably 1 to 5, and most preferably 1 to 2. In preferred embodiments, the number of amino acids inserted, deleted and/or substituted is 1, 2, 3, 4, 5, 6 or 7.

In some embodiments, insertions, deletions and/or substitutions may be in framework (FR) regions, e.g., in FR1, FR2, FR3 and/or FR4; and/or in constant regions, e.g., in CL, CH1, CH2 and/or CH3.

In some embodiments, the substitution of one or more amino acids may be a conservative substitution of one or more amino acids. Examples of conservative substitutions are as described above.

In some embodiments, the antibody of the present invention is selected from a murine antibody, a chimeric antibody, a humanized antibody or a fully human antibody.

The antibody of the present invention may be an intact antibody or an antigen-binding fragment thereof. An antigen-binding fragment may be any fragment of an antibody that retains the ability to bind to Aβ. Examples of the antigen-binding fragments include, but are not limited to: full-length antibodies; Fab; F(ab')₂; Fab'; Fd; Fd'; Fv; scFv; dAb; separate complementarity determining regions (CDRs); nanobodies; linear antibodies consisting of a pair of tandem Fd (VH-CH1-VH-CH1), and modified forms of any of the above fragments that retain antigen-binding activity.

In some embodiments of the antibody or the antigen-binding fragment thereof of the present invention, the antigen-binding fragment is selected from: Fab, Fab', F(ab')₂, Fv, single-chain Fv (scFv) and ds-scFv.

In some embodiments, the antibody is a monovalent antibody, a bivalent antibody or a multivalent antibody.

In some embodiments, the antibody is a monoclonal antibody, a bispecific antibody or a multispecific antibody.

### Nucleic acid molecule

In yet another aspect, the present invention provides a nucleic acid molecule, which comprises a nucleotide sequence encoding the antibody or the antigen-binding fragment thereof of the present invention.

For example, the present invention provides a nucleic acid molecule encoding the CDR sequences in any one of the VHs and/or VLs disclosed herein. In some embodiments, the present invention further provides a nucleic acid molecule having at least 90%, at least 95%, at least 96%, at least 97%, at least 98% or at least 99% sequence identity to the nucleic acid molecule encoding the CDR sequences in any one of the VHs and/or VLs disclosed herein.

For another example, the present invention provides a nucleic acid molecule encoding any one of the VHs and/or VLs disclosed herein. In some embodiments, the present invention further provides a nucleic acid molecule having at least 90%, at least 95%, at least 96%, at least 97%, at least 98% or at least 99% sequence identity to the nucleic acid molecule encoding any one of the VHs and/or VLs disclosed herein.

In some preferred embodiments, the nucleic acid molecule comprises a nucleotide sequence having at least 90%, at least 95%, at least 96%, at least 97%, at least 98% or at least 99% sequence identity to SEQ ID NO: 165; and/or a nucleotide sequence having at least 90%, at least 95%, at least 96%, at least 97%, at least 98% or at least 99% sequence identity to SEQ ID NO: 220.

In some preferred embodiments, the nucleic acid molecule comprises a nucleotide sequence having at least 90%, at least 95%, at least 96%, at least 97%, at least 98% or at least 99% sequence identity to SEQ ID NO: 161; and/or a nucleotide sequence having at least 90%, at least 95%, at least 96%, at least 97%, at least 98% or at least 99% sequence identity to SEQ ID NO: 217.

For another example, the present invention provides a nucleic acid molecule encoding any one of the heavy chains and/or light chains of the antibody disclosed herein. In some embodiments, the present invention further provides a nucleic acid molecule having at least 90%, at least 95%, at least 96%, at least 97%, at least 98% or at least 99% sequence identity to the nucleic acid molecule encoding any one of the heavy chains and/or light chains of the antibody disclosed herein.

In some preferred embodiments, the nucleic acid molecule comprises a nucleotide sequence having at least 90%, at least 95%, at least 96%, at least 97%, at least 98% or at least 99% sequence identity to SEQ ID NO: 276; and/or a nucleotide sequence having at least 90%, at least 95%, at least 96%, at least 97%, at least 98% or at least 99% sequence identity to SEQ ID NO: 278.

In some preferred embodiments, the nucleic acid molecule comprises a nucleotide sequence having at least 90%, at least 95%, at least 96%, at least 97%, at least 98% or at least 99% sequence identity to SEQ ID NO: 272; and/or a nucleotide sequence having at least 90%, at least 95%, at least 96%, at least 97%, at least 98% or at least 99% sequence identity to SEQ ID NO: 274.

In a more preferred embodiment, the nucleic acid molecule comprises a nucleotide sequence as set forth in SEQ ID NO: 276 and a nucleotide sequence as set forth in SEQ ID NO: 278. In another more preferred embodiment, the nucleic acid molecule comprises a nucleotide sequence as set forth in SEQ ID NO: 272 and a nucleotide sequence as set forth in SEQ ID NO: 274.

### Vector

In another aspect, the present invention provides a vector comprising the nucleic acid molecule disclosed herein, which nucleic acid molecule encodes the anti-Aβ antibody or the antigen-binding fragment thereof disclosed herein.

In some embodiments, the vector is an expression vector capable of expressing a polypeptide comprising a heavy chain variable region or a light chain variable region of an antibody. For example, the present invention provides an expression vector comprising any of the above nucleic acid molecules.

Any vector may be suitable for use in the present invention. In some embodiments, the vector is a viral vector. In some embodiments, the vector is a retroviral vector, a DNA vector, a murine leukaemia virus vector, an SFG vector, a plasmid, an RNA vector, an adenoviral vector, a baculoviral vector, an Epstein Barr virus vector, a papovavirus vector, a vaccinia virus vector, a herpes simplex virus vector, an adeno-associated virus (AAV) vector, a lentiviral vector, or any combination thereof. Suitable exemplary vectors include, for example, pBY, pGAR, pBABE-puro, pBABE-neo largeTcDNA, pBABE-hygro-hTERT, pMKO.1 GFP, MSCV-IRES-GFP, pMSCV PIG (Puro IRES GFP empty plasmid), pMSCV-loxp-dsRed-loxp-eGFP-Puro-WPRE, MSCV IRES Luciferase, pMIG, MDH1-PGK-GFP_2.0, TtRMPVIR, pMSCV-IRES-mCherry FP, pRetroX GFP T2A Cre, pRXTN, pLncEXP and pLXIN-Luc.

The vector may be any suitable recombinant expression vector. Suitable vectors include vectors designed for proliferation and amplification, or for expression, or for both, such as plasmids and viruses. For example, the vector may be selected from the pUC series (Fermentas Life Sciences, Glen Burnie, Md.), the pBluescript series (Stratagene, La Jolla, Calif.), the pET series (Novagen, Madison, Wis.), the pGEX series (Pharmacia Biotech, Uppsala, Sweden) and the pEX series (Clontech, Palo Alto, Calif.). Phage vectors such as λGT10, λGT11, λZapII (Stratagene), λEMBL4 and λNM1149 may also be used. Examples of plant expression vectors that can be used in the present invention include pBI01, pBI101.2, pBI101.3, pBI121 and pBIN19 (Clontech). Examples of animal expression vectors that can be used in the present invention include pcDNA, pEUK-Cl, pMAM and pMAMneo (Clontech).

Recombinant expression vectors can be prepared using standard recombinant DNA techniques as described in, for example, Sambrook et al., Molecular Cloning: A Laboratory Manual, 3rd ed., Cold Spring Harbor Press, Cold Spring Harbor, N.Y. 2001; and Ausubel et al., Current Protocols in Molecular Biology, Greene Publishing Associates and John Wiley & Sons, NY, 1994. Circular or linear expression vector constructs can be prepared to contain replication systems that are functional in prokaryotic or eukaryotic host cells. Replication systems may be derived from, for example, ColE1, 2µ plasmid, λ, SV40 or bovine papilloma virus.

For example, the vector may be an adenoviral vector comprising a nucleotide sequence encoding the antibody disclosed herein. The vector can be administered to a subject and then enter a cell of the subject *in vivo*, thereby integrating the nucleotide sequence encoding the antibody disclosed herein into the genome of the cell, which then expresses the antibody disclosed herein.

### Cell

In another aspect, the present invention provides a cell comprising the nucleic acid molecule disclosed herein or the vector disclosed herein.

Any cell can be used as a host cell for the nucleic acid molecule or vector of the present invention. In some embodiments, the cell may be a prokaryotic cell, a fungal cell, a yeast cell, or a higher eukaryotic cell such as a mammalian cell. Suitable prokaryotic cells include, but are not limited to, eubacteria, such as Gramnegative or Gram-positive organisms, e.g., *Enterobacteriaceae*, such as *Escherichia* (e.g., *E. coli*); *Enterobacter*; *Erwinia*; *Klebsiella*; *Proteus*; *Salmonella*, e.g., *Salmonella typhimurium*; *Serratia*, e.g., *Serratia marcescans* and *Shigella*; *Bacilli*, such as *B. subtilis* and *B. licheniformis*; *Pseudomonas*, such as *P. aeruginosa*; and *Streptomyces*. In some embodiments, the cell is a human cell. In some embodiments, the cell is an immune cell. In some embodiments, the cell includes, for example, CHO cells, such as CHOS cells and CHO-K1 cells, or HEK293 cells, such as HEK293A, HEK293T and HEK293FS.

The cell of the present invention is prepared by introducing the vector disclosed herein or the nucleic acid disclosed herein *in vitro* or *ex vivo*. The cell of the present invention can be administered to a subject, and the cell expresses the antibody disclosed herein *in vivo*.

### Preparation method

In another aspect, the present invention provides a method for preparing the antibody or the antigen-binding fragment thereof disclosed herein, which method comprises culturing the cell provided by the present invention under conditions suitable for expression of the antibody or the antigen-binding fragment thereof disclosed herein; and isolating the antibody or the antigen-binding fragment thereof from a culture and/or a culture supernatant of the cell.

Any method suitable for producing antibodies can be used to produce the antibody of the present invention. For example, antibodies can be prepared using hybridoma methods, e.g., the method described in Kohler and Milstein, Nature, 256: 495 (1975). In hybridoma methods, mice, hamsters or other suitable host animals are generally immunized with an immunizing agent to elicit lymphocytes that produce or are capable of producing antibodies that will specifically bind to the immunizing agent. Alternatively, lymphocytes can be immunized *in vitro*.

The immunizing agent generally includes a protein antigen, a fragment thereof or a fusion protein thereof. Herein, any suitable form of Aβ can be used as an immunizing agent (antigen) to produce non-human antibodies specific for Aβ and to screen for the biological activity of the antibodies. For example, antigens suitable for use in the present invention include, but are not limited to, Aβ1-42 protofibril, KLH-CV-41-Aβ1-40, Aβ1-40 protofibril and pyroE Aβ3-42 peptide.

The immunizing agent may be used alone or in combination with one or more immunogenicity enhancers known in the art. Generally, if cells of human origin are desired, peripheral blood lymphocytes are used; if cells of non-human mammalian origin are desired, splenocytes or lymph node cells are used. The lymphocytes are then fused with an immortalized cell line using a suitable fusing agent (e.g., polyethylene glycol) to form hybridoma cells (Goding, Monoclonal Antibodies: Principles and Practice, Academic Press, (1986) pp. 59-103). The immortalized cell lines are generally transformed mammalian cells, particularly myeloma cells of rodent, bovine and human origin. Generally, a rat or mouse myeloma cell line is used. The hybridoma cells can be cultured in a suitable medium, which preferably contains one or more substances that inhibit the growth or survival of unfused immortalized cells. For example, if the parent cells lack hypoxanthine-guanine phosphoribosyltransferase (HGPRT or HPRT), the hybridoma medium generally contains hypoxanthine, aminopterin and thymidine ("HAT medium"), which can prevent the growth of HGPRT-deficient cells.

The medium in which the hybridoma cells are cultured can then be determined for the presence of monoclonal antibodies against the antigen. Preferably, the binding specificity of monoclonal antibodies produced by hybridoma cells is determined by immunoprecipitation or by *in-vitro* binding assays such as radioimmunoassay (RIA) or enzyme-linked immunosorbent assay (ELISA). Such techniques and assays are known in the art. The binding affinity of monoclonal antibodies can be determined, for example, by Scatchard analysis by Munson and Pollard, Anal. Biochem., 107: 220 (1980). In addition, in therapeutic applications of monoclonal antibodies, it is important to identify antibodies that have high specificity and high binding affinity for the target antigen.

After the desired hybridoma cells have been identified, clones can be subcloned through limiting dilution procedures and cultured by standard methods. (See Goding, Monoclonal Antibodies: Principles and Practice, Academic Press, (1986) pp. 59-103). Suitable media for this purpose include, for example, Dulbecco's Modified Eagle's Medium and RPMI-1640 medium. Alternatively, hybridoma cells can be grown *in vivo* as ascites in mammals. Monoclonal antibodies secreted by subclones can be isolated or purified from the medium or ascites through conventional immunoglobulin purification procedures, e.g., protein A-Sepharose, hydroxyapatite chromatography, gel electrophoresis, dialysis or affinity chromatography.

Monoclonal antibodies can also be prepared by recombinant DNA methods, such as U.S. Patent No. 4,816,567. DNA encoding the monoclonal antibody of the present invention can be readily isolated and sequenced using conventional procedures (e.g., by using oligonucleotide probes that can specifically bind to genes encoding the heavy and light chains of murine antibodies). The hybridoma cells of the present invention are used as a preferred source of such DNA. The isolated DNA can be placed in an expression vector, which is then transfected into host cells, such as simian COS cells, Chinese hamster ovary (CHO) cells, or myeloma cells, which do not produce immunoglobulins, to obtain antibodies in the recombinant host cells that synthesise monoclonal antibodies. The DNA may also be modified, for example, by replacing homologous murine sequences with coding sequences for human heavy and light chain constant domains (see U.S. Patent Nos. 4,816,567; Morrison, Nature, 368, 812-13 (1994)) or by covalently linking all or part of the coding sequence for a non-immunoglobulin polypeptide to an immunoglobulin coding sequence. Such a non-immunoglobulin polypeptide may be substituted for the constant domain of the antibody of the present invention, or may be substituted for the variable domain of an antigen-binding site of the antibody of the present invention to produce a chimeric bivalent antibody.

The sequence of the antibody of the present invention can be obtained by conventional techniques, such as PCR amplification or genomic library screening. In addition, the coding sequences for the light and heavy chains can be fused together to form a single-chain antibody.

Once the relevant sequences are obtained, a recombinant method can be used to obtain the relevant antibodies in large numbers. This generally involves cloning the sequences into vectors, transferring the vectors into cells, and then isolating the relevant antibody from the proliferated host cells using conventional methods. In addition, the relevant sequences may also be synthesised using artificial synthesis methods, especially when the fragment length is short. Typically, fragments with very long sequences can be obtained by first synthesising multiple small fragments followed by ligation. The nucleic acid molecule can then be introduced into various existing vectors known in the art, which can be used to transform suitable host cells to express proteins, thereby obtaining the antibody of the present invention.

### Pharmaceutical composition

In another aspect, the present invention provides a pharmaceutical composition, which comprises the antibody or the antigen-binding fragment thereof disclosed herein, the nucleic acid molecule disclosed herein, the vector disclosed herein or the cell disclosed herein, and optionally a pharmaceutically acceptable carrier.

The antibody or the antigen-binding fragment thereof of the present invention, and derivatives, fragments, analogues and homologs thereof, can be incorporated into pharmaceutical compositions suitable for administration. Such compositions generally comprise the antibody or the antigen-binding fragment thereof or an agent and a pharmaceutically acceptable carrier.

As used herein, the term "pharmaceutically acceptable" refers to carriers or excipients that are suitable for use in contact with human and animal tissues within the scope of reasonable medical judgment without excessive toxicity, irritation, allergic response, or other problems or complications, and are commensurate with a reasonable benefit/risk ratio.

Exemplary carriers for use in the pharmaceutical compositions of the present invention include saline, buffered saline, glucose and water. Exemplary excipients for use in the composition of the present invention include fillers, binders, disintegrants, coating agents, adsorbents, antiadherents, glidants, preservatives, antioxidants, flavouring agents, colouring agents, sweeteners, solvents, co-solvents, buffers, chelating agents, viscosity-imparting agents, surfactants, diluents, wetting agents, carriers, diluents, preservatives, emulsifiers, stabilizers and tonicity modifiers. It is known to those skilled in the art to select suitable excipients to prepare the pharmaceutical composition of the present invention. In general, the selection of suitable excipients depends particularly on the active agent used, the disease to be treated, and the desired dosage form of the composition.

Liposomes and non-aqueous vehicles such as non-volatile oils may also be used. The use of such media for pharmaceutically active substances is well known in the art. Unless any conventional medium is incompatible with the active compound, use thereof in the pharmaceutical composition is contemplated. Supplementary active compounds may also be incorporated into the composition.

The pharmaceutical composition of the present invention can be formulated to be compatible with the intended route of administration thereof. The route of administration of the pharmaceutical composition of the present invention is preferably parenteral administration, injection administration or oral administration. The injection administration preferably includes routes such as intravenous injection, intramuscular injection, intraperitoneal injection, intradermal injection, or subcutaneous injection. The pharmaceutical composition is in various conventional dosage forms in the art, preferably in solid, semi-solid or liquid form, that is, it may be an aqueous solution, a non-aqueous solution or a suspension, more preferably a tablet, a capsule, a granule, an injection or an infusion, etc. More preferably, it is administered intravascularly, subcutaneously, intraperitoneally, or intramuscularly. Preferably, the pharmaceutical composition may also be administered as an aerosol or a coarse spray, i.e., administered nasally; or administered intrathecally, intramedullarily or intraventricularly. More preferably, the pharmaceutical composition may also be administered transdermally, percutaneously, topically, enterally, intravaginally, sublingually or rectally. The pharmaceutical composition of the present invention can be formulated into various dosage forms as needed, and can be administered by a physician at a dose that is beneficial to a patient based on the type, age, body weight and general disease condition of the patient, the route of administration, etc. The route of administration may be, for example, injection or other treatment routes.

The dosage level for administration of the pharmaceutical composition of the present invention can be adjusted on the basis of the amount of the composition that can achieve the desired diagnostic or therapeutic outcome. The administration regimen may also be a single injection or multiple injections, or adjusted. The dosage level and regimen selected are reasonably adjusted depending on a variety of factors including the activity, stability (i.e., half-life), preparation, and route of administration of the pharmaceutical composition, combinations with other drugs or treatments, a disease or disorder to be detected and/or treated, the health status and prior medical history of a subject to be treated, etc.

### Kit

In another aspect, the present invention provides a kit, which comprises the antibody or the antigen-binding fragment thereof disclosed herein, the nucleic acid molecule disclosed herein, the vector disclosed herein, the cell disclosed herein or the pharmaceutical composition disclosed herein, and instructions for use for administering the above substances. In some embodiments, the kit further comprises an administration device.

In some embodiments, the present invention provides a kit, which may comprise the antibody or the antigen-binding fragment thereof disclosed herein, the nucleic acid molecule disclosed herein, the vector disclosed herein, the cell disclosed herein or the pharmaceutical composition disclosed herein. The kit may comprise the antibody, nucleic acid molecule, vector, cell or pharmaceutical composition of the present invention in a single common container. In certain cases, the kit may comprise instructions for use, including information regarding the antibody, nucleic acid molecule, vector, cell or pharmaceutical composition in the kit, and the dosage form. In general, such information helps patients and physicians to use the encapsulated antibody, nucleic acid molecule, vector, cell or pharmaceutical composition efficiently and safely. Containers used in such kits may generally contain at least one vial, test tube, flask, bottle, syringe or other suitable container.

In some embodiments, the present invention provides a kit, which may comprise the antibody or the antigen-binding fragment thereof disclosed herein, the nucleic acid molecule disclosed herein, the vector disclosed herein, the cell disclosed herein or the pharmaceutical composition disclosed herein, instructions for use for administering the above substances, and an administration device for administering the above substances. The administration device can introduce a substance into a patient via a parenteral route (e.g., intramuscular, subcutaneous or intravenous route). For example, the administration device may be a syringe (e.g., a pre-filled syringe having the antibody or the pharmaceutical composition of the present invention, e.g., an autoinjector), which may include a barrel for containing a fluid to be injected (e.g., the antibody or the pharmaceutical composition of the present invention) and a needle (which may be used to pierce the skin and/or blood vessels). The method of administration may be varied. The route of administration may include oral administration, intramuscular injection, subcutaneous injection, rectal administration, etc.

### Method and use

In another aspect, the present invention provides a method for treating or preventing a disease caused by abnormal accumulation or deposition of Aβ in a subject, which method comprises administering to the subject an effective amount of the antibody or the antigen-binding fragment thereof disclosed herein, the nucleic acid molecule disclosed herein, the vector disclosed herein, the cell disclosed herein or the pharmaceutical composition disclosed herein.

In another aspect, the present invention provides a method for clearing or alleviating abnormal accumulation or deposition of Aβ in a subject, which method comprises administering to the subject an effective amount of the antibody or the antigen-binding fragment thereof disclosed herein, the nucleic acid molecule disclosed herein, the vector disclosed herein, the cell disclosed herein or the pharmaceutical composition disclosed herein.

As used herein, the term "effective amount" means an amount of a compound that, when administered to a subject for the treatment or prevention of a disease, is sufficient to achieve the therapeutic or prophylactic effect described herein. The "effective amount" may vary depending on the compound, the disease and the severity thereof, and the age, weight, etc., of the subject to be treated. A "therapeutically effective amount" refers to an effective amount for therapeutic treatment. A "prophylactically effective amount" refers to an effective amount for prophylactic treatment.

In some embodiments, the dose or effective amount administered to a subject may vary depending on the embodiment, the drug used, the method of administration, and the site and subject to be treated. However, the dose should be sufficient to provide a therapeutic response. A clinician can determine an effective amount to be administered to a human or other subject to treat a medical condition. The precise amount required to be therapeutically effective may depend on many factors, such as the activity of the antibody and the route of administration.

The dose of the antibody, nucleic acid molecule, vector, cell or pharmaceutical composition described herein can be administered to a mammal in a single administration or in a series of sub-doses over a suitable time period, for example, once daily, semi-weekly, weekly, bi-weekly, semi-monthly, bi-monthly, semiannually or annually, as needed. A dosage unit containing an effective amount of the antibody, nucleic acid molecule, vector, cell and/or pharmaceutical composition can be administered as a single daily dose, or the total daily dose can be administered in two, three, four or more divided doses per day as needed.

A suitable method of administration can be selected by a physician. The route of administration may be parenteral administration, such as administration by injection, nasal administration, pulmonary administration or transdermal administration. Systemic or local administration can be performed via intravenous injection, intramuscular injection, intraperitoneal injection or subcutaneous injection. The dose and method of administration may vary depending on the weight, age, conditions, etc., of the subject and may be selected appropriately.

In another aspect, the present invention provides the use of the antibody or the antigen-binding fragment thereof disclosed herein, the nucleic acid molecule disclosed herein, the vector disclosed herein, the cell disclosed herein or the pharmaceutical composition disclosed herein in the preparation of a drug for treating or preventing a disease caused by abnormal accumulation or deposition of Aβ in a subject.

In another aspect, the present invention provides the use of the antibody or the antigen-binding fragment thereof disclosed herein, the nucleic acid molecule disclosed herein, the vector disclosed herein, the cell disclosed herein or the pharmaceutical composition disclosed herein in the preparation of a drug for clearing or alleviating abnormal accumulation or deposition of Aβ in a subject.

In some embodiments, the subject treated by the method described herein has a degenerative disease of the central nervous system. In some embodiments, the subject has Alzheimer's disease (AD), Parkinson's disease (PD), Down syndrome or cerebral amyloid angiopathy, or is at risk of having AD, PD, Down syndrome or cerebral amyloid angiopathy. In some embodiments, the subject has preclinical AD or clinical AD, e.g., prodromal AD, mild AD, moderate AD or severe AD.

In the case of Alzheimer's disease, "treatment" or "prevention" can limit, slow or stop one or more of cognitive symptoms, behavioural symptoms and/or pathological symptoms in a patient, or prevent the onset or progression of one or more of the above symptoms. Cognitive symptoms may include intellectual decline, difficulty in thinking and understanding, sundowning, delusions, disorientation, forgetfulness, confabulation, mental confusion, difficulty in concentrating, inability to form new memories, inability to do simple mathematics, or inability to recognize common objects. Behavioural symptoms may include aggressiveness, agitation, difficulty in self-care, irritability, meaningless self-talk, personality changes, restlessness, lack of restraint, or wandering and getting lost. AD patients may also experience anger, blank stares, general dissatisfaction, loneliness, mood swings, depression, hallucinations, paranoia, lack of muscle coordination, slurred speech, or loss of appetite. Pathological symptoms include the formation and development of amyloid plaques and neurofibrillary tangles, etc.

To make the objectives, technical solutions and advantages of the present invention more clearly understood, the present invention will be further described below in conjunction with specific examples and the accompanying drawings. The advantages and features of the present invention will become more apparent with the description. It should be understood that these examples are merely used for describing the present invention, rather than limiting the scope of the present invention. In the following examples, experimental methods without specific conditions indicated are performed according to conventional conditions in the art, for example, those described in Sambrook and Russeii, et al., Molecular Cloning: A Laboratory Manual (3rd ed., 2001), CSHL Press, or according to conditions recommended by the manufacturer. The experimental materials and reagents used in the following examples are commercially available unless otherwise specified.

### Examples

### Example 1. Production of anti-Aβ antibody

All antigens used in the present invention were synthesised by GL Biochem (Shanghai) Ltd.

Aβ1-42 powder was added to a 10 mM NaOH solution, followed by vortexing for 2 min until Aβ was completely dissolved (concentration: 443 µM). PBS was added, followed by shaking. The mixture was left to stand at 37°C for 48 hours. After centrifugation, the supernatant was collected. Protofibril was purified using a Superdex 75 column, aliquoted, and stored in a refrigerator at -80°C for subsequent immunization and screening.

pyroE Aβ3-42 peptide antigen was dissolved in 50 mM sodium acetate for subsequent immunization and screening.

### 1) Mouse immunization

Six Balb/c mice, all purchased from Zhejiang Vital River Laboratory Animal Technology Co., Ltd., were immunized. Aβ1-42 protofibril and pyroE Aβ3-42 peptide antigen were used, respectively, for a total of four immunizations, wherein complete Freund's adjuvant (CFA) was used for the primary immunization, and the Sigma Adjuvant System was used for subsequent immunizations once a week, with a two-week interval after the primary immunization. For group C1, 79 µL of Aβ1-42 protofibril antigen was taken and mixed with 421 µL of PBS; for group C2, 500 µL of pyroE Aβ3-42 peptide antigen was taken. 500 µL of the prepared antigen mixture was aspirated using a 3 mL syringe, 500 µL of CFA was aspirated using another syringe, and the two were mixed using a T-tee until a water-in-oil, milky white emulsion was formed. A drop of the emulsion was placed in ice water and remained without spreading for 20 min. The prepared antigen was administered at a dose of 50 µg/120 µL/mouse. First, the prepared antigen was injected subcutaneously in the abdomen near the base of the thigh at a dose of 100 µL/mouse. After subcutaneous immunization, the mice were briefly anesthetized with isoflurane, and then immunized with the prepared antigen via footpad injection at a dose of 20 µL/mouse. After the immunization was completed, the mice were returned to mouse cages for continued housing. For groups C1 and C2, the primary immunization used complete Freund's adjuvant, and the interval between the primary immunization and the second immunization was two weeks.

For groups C1 and C2, beginning with the second immunization, Ribi adjuvant was used for immunization once a week for three weeks. For group C1, 24 µL of antigen was aspirated and mixed uniformly with 176 µL of PBS; for group C2, 150 µL of antigen was aspirated and mixed uniformly with 50 µL of PBS. The diluted antigen was added to 200 µL of Sigma Adjuvant System, and the mixture was vortexed thoroughly for 2 min to ensure uniform mixing. After the mice were briefly anesthetized with isoflurane, the hind footpads or elbow joints of the mice were disinfected with alcohol swabs. 25 µL of the antigen-adjuvant mixture was injected into each hind footpad or elbow joint using a disposable insulin syringe. After the immunization was completed, the mice were returned to mouse cages. The mice were monitored until they regained consciousness and showed no abnormalities, after which the cages were placed back on the rack for continued housing. After the entire immunization process was completed, serum titres were measured. If the titres met the requirements for fusion, splenocyte fusion and subsequent hybridoma screening were initiated.

### 2) Splenocyte fusion

After the titre detected met the antibody screening criteria, SP2/0 cells were revived. The SP2/0 cells used in the first fusion were obtained as a gift, whereas the SP2/0 cells in the second and third fusions were purchased from Shanghai Qiwen Biotechnology Co., Ltd. The water bath was preheated to 37°C. In an ultra-clean workbench, 30 mL of Medium A (STEMCELL, 03801) was aseptically transferred into a 50 mL centrifuge tube and placed in the water bath for preheating. The liquid nitrogen tank was opened, and the SP2/0 cells were taken out, quickly transferred to the 37°C water bath, and shaken until completely thawed within 2 minutes. The water on the outer wall of the cryovial was wiped off. 75% ethanol was sprayed. Then the cryovial was placed in the ultra-clean workbench. The cap of the cryovial was unscrewed, and the cells were gently pipetted twice using a pipette and then completely transferred to a 15 mL centrifuge tube. Then, the preheated Medium A (STEMCELL, 03801) was added dropwise, with a total of 5 mL added. The cells were centrifuged at 1000 rpm for 5 minutes, the supernatant was discarded, and the cell pellet was gently dispersed by tapping. The cells were resuspended in 10 mL of Medium A and counted. The cells were then seeded into a T75 flask at a density of 5e4 cells/mL in a total volume of 20 mL, and cultured in an incubator at 37°C with 5% CO₂.

The cells were cultured to an appropriate density for passaging. During each passage, the maximum cell density in a T75 flask could reach 4e5 cells/mL. The day before electrofusion, the cells were seeded into a T175 flask at a density of 1e5 cells/mL. For the fusion the next day, the optimal cell density was 3 to 4e5 cells/mL.

Blood was collected via eyeball enucleation into EP tubes (treated with sodium heparin). After being left to stand at 37°C for 2 h, the blood was centrifuged to separate the serum, which was stored at -20°C for later use (as positive control). The mice were sacrificed by cervical dislocation, immersed in a 75% ethanol solution for 5 min for disinfection, and then fixed in a supine position on a dissecting plate in an ultra-clean workbench. The popliteal lymph nodes, inguinal lymph nodes and iliac lymph nodes of the mice were collected, and then ground repeatedly to collect as many lymphocytes as possible. The cell suspension was then filtered, and the cells were counted.

SP2/0 cells and B cells were mixed at a certain ratio. Finally, the cells were resuspended in an electrofusion buffer, and the cell suspension was immediately added to the electrodes. After setting the parameters, fusion was performed. The cells after electrofusion were left to stand for at least 15 min. The cells were gently removed from the electrofusion cuvette, cultured overnight, and then added to Medium C (STEMCELL, 03803) and Medium D (STEMCELL, 03804). The semisolid medium was seeded in a 6-well plate at a density of 2 mL/well, followed by continued culture. After reaching an appropriate degree, fluorescent clones were picked for subsequent screening.

### 3) Antibody screening

Aβ1-42 protofibril or pyroE Aβ3-42 peptide was diluted with a coating buffer, and added to a microplate, followed by incubation at 4°C overnight. The microplate was taken out and washed three times with PBST. A blocking solution was added to the plate, and the plate was incubated at room temperature for 1 h. The plate was washed once with PBST. Both the sample and control were added to the microplate at 50 µL/well, and the plate was incubated at room temperature for 1 h. The plate was washed with PBST. A secondary antibody was diluted and then added to the plate, and the plate was incubated at room temperature for 1 h. The plate was washed again with TBST. A chromogenic substrate was added, and the plate was left to stand in an incubator at 37°C for colour development for 10 min. A 2 M concentrated sulphuric acid stop solution was added. The absorbance was read using a microplate reader within 15 min (wavelength: 450 nm).

### Example 2. Acquisition of anti-Aβ antibody hybridoma positive clones

On the basis of the results of hybridoma screening, positive monoclonal cells were centrifuged at 1000 rpm, the cells were collected, and the total RNA was extracted using Trizol. Using the total RNA as a template, first-strand cDNA was synthesised. Subsequently, using the first-strand cDNA as a template, the variable region DNA sequences corresponding to the hybridoma cells were amplified. In a 50 µL reaction system, 1 µL of cDNA, 5 µL of 10× PCR buffer, 1 µL of each of forward and reverse primers, 1 µL of dNTP, 1 µL of 25 mmol MgCl₂, 39 µL of H₂O and 1 µL of Taq enzyme were added. The mixture was pre-denatured at 95°C for 10 minutes, followed by temperature cycling for PCR amplification. The reaction conditions were as follows: denaturation at 94°C for 1 minute, annealing at 58°C for 1 minute, and extension at 72°C for 15 seconds, with a total of 30 cycles, followed by incubation at 72°C for 10 minutes. The obtained clones such as 7C8 exhibited binding to Aβ1-42 protofibril or pyroE Aβ3-42 peptide, but weak or no binding to Aβ1-42 monomer. The binding activities of each chimeric antibody to the antigens are shown in Table 1.

**Table 1. Binding of anti-Aβ chimeric antibodies to antigens**

| **Clone** | **Aβ1-42 protofibril** | **pyroE Aβ3-42** | **Aβ1-42 monomer** |
|---|---|---|---|
| 192E15 | + | ++++ | - |
| 179M22 | + | ++++ | - |
| 200O1 | + | ++++ | - |
| 175P19 | + | ++++ | - |
| 178D4 | + | ++++ | - |
| 185O18 | + | ++++ | - |
| 193D22 | + | ++++ | - |
| 151N5 | + | ++++ | - |
| 151N21 | + | ++++ | - |
| 171P1 | + | ++++ | - |
| 174J17 | + | ++++ | - |
| 193M7 | + | ++++ | - |
| 6B5 | ++++ | + | ++ |
| 6C9 | ++++ | + | ++ |
| 11G10 | ++++ | + | ++ |
| 7C8 | ++++ | + | ++ |
| 15E11-2 | + | +++ | + |
| donanemab | ++++ | + | + |
| lecanemab | + | ++++ | ++ |
| Isotype control | - | - | - |

| | | | |
|---|---|---|---|
| "++++" indicates strong binding, "++" indicates weak binding, "+" indicates very weak binding, and "-" indicates no binding. | | | |

The variable region sequences of the above chimeric antibodies are shown in Table 2 below.

**Table 2. Variable region sequences of anti-Aβ chimeric antibodies**

| Clone | VH | | HCD R1 | HCD R2 | HCD R3 | VL | | LCDR1 | LCDR2 | LCDR3 |
|---|---|---|---|---|---|---|---|---|---|---|
| | Amino acid sequence | Nucleotide sequence | Amino acid sequence | Amino acid sequence | Amino acid sequence | Amino acid sequence | Nucleotide sequence | Amino acid sequence | Amino acid sequence | Amino acid sequence |
| 192E15 | 1 | 2 | 3 | 4 | 5 | 6 | 7 | 8 | 9 | 10 |
| 179M22 | 11 | 12 | 13 | 14 | 15 | 16 | 17 | 18 | 19 | 20 |
| 200O1 | 21 | 22 | 23 | 24 | 25 | 26 | 27 | 28 | 29 | 30 |
| 175P19 | 31 | 32 | 33 | 34 | 35 | 36 | 37 | 38 | 19 | 20 |
| 178D4 | 39 | 40 | 41 | 42 | 43 | 44 | 45 | 46 | 47 | 48 |
| 185018 | 49 | 50 | 51 | 52 | 53 | 54 | 55 | 56 | 19 | 20 |
| 193D22 | 57 | 58 | 59 | 60 | 61 | 62 | 63 | 64 | 65 | 66 |
| 151N5 | 67 | 68 | 69 | 70 | 71 | 72 | 73 | 74 | 75 | 76 |
| 156N21 | 77 | 78 | 3 | 4 | 79 | 80 | 81 | 82 | 83 | 84 |
| 171P1 | 85 | 86 | 87 | 88 | 89 | 90 | 91 | 92 | 93 | 94 |
| 6B5 | 95 | 96 | 97 | 98 | 99 | 100 | 101 | 102 | 103 | 104 |
| 6C9 | 105 | 106 | 107 | 108 | 99 | 109 | 110 | 102 | 103 | 111 |
| 11G10 | 112 | 113 | 114 | 98 | 115 | 116 | 117 | 102 | 118 | 104 |
| 7C8 | 119 | 120 | 121 | 122 | 123 | 124 | 125 | 126 | 65 | 127 |
| 174J17 | 128 | 129 | 130 | 131 | 279 | 132 | 133 | 74 | 134 | 76 |
| 193M7 | 135 | 136 | 59 | 137 | 138 | 139 | 140 | 141 | 65 | 66 |
| 15E11-2 | 142 | 143 | 121 | 122 | 144 | 145 | 146 | 147 | 148 | 149 |

In the amino acid sequences of VH or VL as shown below, the CDR sequences are indicated in bold and underlined.

### 192E15

The amino acid sequence of VH is as set forth in SEQ ID NO. 1, the nucleic acid encoding the VH is as set forth in SEQ ID NO. 2, and the HCDR1, HCDR2 and HCDR3 of the VH are as set forth in SEQ ID NOs. 3, 4 and 5, respectively.

<-------------FR1-------------> CDR1<----FR2----> CDR2
EVKLVESGGGLVQPGGSRKLSCAASGFTFSDYGMAWVRQAPGKGPEWVAFISNLAYSIYY
<---------------FR3-------------> CDR3 <---FR4
ADTVTGRFTISRQNAKNTLYLEMSSLRSEDTAMYYCARDRGYDGHSPYYAMDYWGQGTSV
--->
TVSS

### Nucleic acid sequence

GAGGTGAAGCTGGTGGAGTCTGGGGGAGGCTTAGTGCAGCCTGGAGGGTCCCGGAAACTCTCC
TGTGCAGCCTCTGGATTCACTTTCAGTGACTACGGAATGGCGTGGGTTCGACAGGCTCCAGGG
AAGGGGCCTGAGTGGGTGGCATTCATTAGTAATTTGGCATATAGTATTTACTATGCAGACACT
GTGACGGGCCGATTCACCATCTCTAGACAGAATGCCAAGAACACCCTGTACCTGGAAATGAGC
AGTCTGAGGTCTGAGGACACAGCCATGTACTACTGTGCAAGGGATCGGGGGTATGATGGTCAC
TCCCCTTACTATGCTATGGACTACTGGGGTCAGGGAACCTCAGTCACCGTCTCCTCA

The amino acid sequence of VL is as set forth in SEQ ID NO. 6, the nucleic acid encoding the VL is as set forth in SEQ ID NO. 7, and the LCDR1, LCDR2 and LCDR3 of the VL are as set forth in SEQ ID NOs. 8, 9 and 10, respectively.

<---------FR1--------> CDR1 <-----FR2-----> CDR2 <-
QAVVTQESALTTSPGETVTLTCRSSTGAVTTSNYANWVQEKPDHLFTGLIGGTSNRPPGV
-----------FR3-------------> CDR3 <---FR4--->
PARFSGSLIGDKAALTITGAQTEDEAIYFCALWYSHHWVFGGGTKLTVL

### Nucleic acid sequence

CAGGCTGTTGTGACTCAGGAATCTGCACTCACCACATCACCTGGTGAAACAGTCACACTCACT
TGTCGCTCAAGTACTGGGGCTGTTACAACTAGTAACTATGCCAACTGGGTCCAAGAAAAACCA
GATCATTTATTCACTGGTCTAATTGGTGGTACCAGCAACCGACCTCCAGGTGTTCCTGCCAGA
TTCTCAGGCTCCCTGATTGGAGACAAGGCTGCCCTCACCATCACAGGGGCACAGACTGAGGAT
GAGGCAATATATTTCTGTGCTCTATGGTACAGCCACCACTGGGTGTTCGGTGGAGGAACCAAA
CTGACTGTCCTA

### 179M22

The amino acid sequence of VH is as set forth in SEQ ID NO. 11, the nucleic acid encoding the VH is as set forth in SEQ ID NO. 12, and the HCDR1, HCDR2 and HCDR3 of the VH are as set forth in SEQ ID NOs. 13, 14 and 15, respectively.

<-----------FR1-------------> CDR1<----FR2----> CDR2
EVMLVESGRDLVKPGGSLKLSCAASGFTFSAYAMSWVRQTPEKRLEWVATITSRGTYTYY
<---------------FR3-----------> CDR3 <----
PDSVKGRFTISRDNAKNTLYLQMHSLRSEDTAMYYCVRHELITTVVSTDYFSLDYWGQGT
FR4-->
SVTVSS

### Nucleic acid sequence

GAAGTGATGCTGGTGGAGTCTGGGAGAGACTTAGTGAAGCCTGGAGGGTCCCTGAAACTCTCC
TGTGCAGCCTCTGGATTCACTTTCAGTGCCTATGCCATGTCTTGGGTTCGCCAGACTCCGGAG

AAGAGGCTGGAGTGGGTCGCAACCATTACTAGTCGTGGTACTTACACCTACTATCCAGACAGT
GTGAAGGGGCGATTCACCATCTCCAGAGACAATGCCAAGAACACCCTGTACCTGCAAATGCAC
AGTCTGAGGTCTGAGGACACGGCCATGTATTACTGTGTGAGACATGAGCTTATTACTACGGTA
GTATCTACGGATTACTTTTCTCTGGACTACTGGGGTCAAGGAACCTCAGTCACCGTCTCCTCA

The amino acid sequence of VL is as set forth in SEQ ID NO. 16, the nucleic acid encoding the VL is as set forth in SEQ ID NO. 17, and the LCDR1, LCDR2 and LCDR3 of the VL are as set forth in SEQ ID NOs. 18, 19 and 20, respectively.

<-----------FR1--------> CDR1 <-----FR2-----> CDR2
DIVLTQSPASLAVSLGQRATISCRASDSVEYYGTSLMQWYQQKPGQPPKLLIYAASNVES
<------------FR3---------------> CDR3 <--FR4--->
GVPARFSGSGSGTDFSLNIHPVEEDDVAMYFCQQSRKVPLTFGAGTKLELK

### Nucleic acid sequence

GACATTGTGCTCACCCAATCTCCAGCTTCTTTGGCTGTGTCCCTAGGGCAGAGAGCCACCATC
TCCTGTAGAGCCAGTGACAGTGTTGAATATTATGGCACAAGTTTAATGCAGTGGTACCAACAG
AAACCAGGACAGCCACCCAAACTCCTCATCTATGCTGCATCCAACGTAGAATCTGGGGTCCCT
GCCAGGTTTAGTGGCAGTGGGTCTGGGACAGACTTCAGCCTCAACATCCATCCTGTGGAGGAG
GATGATGTTGCAATGTATTTCTGTCAGCAAAGTAGGAAGGTTCCGCTCACGTTCGGTGCTGGG
ACCAAGCTGGAGCTGAAA

### 200O1

The amino acid sequence of VH is as set forth in SEQ ID NO. 21, the nucleic acid encoding the VH is as set forth in SEQ ID NO. 22, and the HCDR1, HCDR2 and HCDR3 of the VH are as set forth in SEQ ID NOs. 23, 24 and 25, respectively.

<-------------FR1-------------> CDR1<----FR2-----> CDR2
QVQLKESGPGLVAPSQSLSITCTVSGFSLSRYIVHWYRQPPGKGLEWLGMIWGGGRIDYN
<-----------------FR3-------------> CDR3 <---FR4--->
SALKSRLSISKDNSKSQVFLKMNSLQTDDTAMYYCARNFGENAEAYWGQGTLVTVSA

### Nucleic acid sequence

CAGGTGCAACTGAAGGAGTCAGGACCTGGCCTGGTGGCACCCTCACAGAGCCTGTCCATCACA
TGCACTGTCTCTGGGTTCTCATTATCCAGATATATTGTACACTGGTATCGGCAGCCTCCAGGA
AAGGGTCTGGAGTGGCTGGGAATGATATGGGGTGGTGGAAGGATAGACTATAATTCAGCTCTC
AAATCCAGACTGAGCATCAGTAAGGACAACTCCAAGAGCCAAGTTTTTTTAAAAATGAACAGT
CTGCAAACTGATGACACAGCCATGTACTACTGTGCCAGAAATTTCGGGGAAAACGCCGAGGCT
TATTGGGGCCAAGGGACTCTGGTCACTGTCTCTGCA

The amino acid sequence of VL is as set forth in SEQ ID NO. 26, the nucleic acid encoding the VL is as set forth in SEQ ID NO. 27, and the LCDR1, LCDR2 and LCDR3 of the VL are as set forth in SEQ ID NOs. 28, 29 and 30, respectively.

<-----------FR1--------->   CDR1   <-----FR2----->   CDR2 <---
DIQMTQTTSSLSASLGDRVTITCRASQDINIYLNWYQQKPDGVVKLLIYYTSRLHSGVPS
-----------FR3-------------->   CDR3   <--FR4--->
RFSGSGSGTDFSLTISNLEQEDIATYFCQQGHTLPYTFGGGTKLEIK

### Nucleic acid sequence

GATATCCAGATGACACAGACTACATCCTCCCTGTCTGCCTCTCTGGGAGACAGAGTCACCATC
ACTTGCAGGGCAAGTCAGGACATTAACATTTATTTAAACTGGTATCAGCAGAAACCAGATGGA
GTTGTTAAACTCCTGATCTACTACACATCAAGATTACATTCAGGAGTCCCATCAAGGTTCAGT
GGCAGTGGGTCTGGAACAGATTTTTCTCTCACCATTAGCAACCTGGAGCAAGAAGATATTGCC
ACTTACTTTTGCCAACAGGGTCATACACTTCCGTACACGTTCGGAGGGGGGACCAAGCTGGAA
ATAAAA

### 175P19

The amino acid sequence of VH is as set forth in SEQ ID NO. 31, the nucleic acid encoding the VH is as set forth in SEQ ID NO. 32, and the HCDR1, HCDR2 and HCDR3 of the VH are as set forth in SEQ ID NOs. 33, 34 and 35, respectively.

<-------------FR1-------------> CDR1<----FR2----> CDR2
EVMLVESGRDLVKPGGSLRLSCAASGFTFSTYAMSWIRQTPEKRLEWVATITSAGTYTYY
<---------------FR3-------- -----> CDR3 <----
PDSVKGRFTISRDNAKNTLYLQMRSLRSEDTAMYYCVRHELITTVVSTDYFSMDYWGQGT
FR4-->
SVTVSS

### Nucleic acid sequence

GAAGTGATGCTGGTGGAGTCTGGGAGAGACTTAGTGAAGCCTGGAGGATCCCTGAGACTCTCC
TGTGCAGCCTCTGGATTCACTTTCAGTACCTATGCCATGTCTTGGATTCGCCAGACTCCGGAG
AAGAGGCTGGAGTGGGTCGCAACCATTACTAGTGCTGGTACTTACACCTACTATCCAGACAGT
GTGAAGGGGCGATTCACCATCTCCAGAGACAATGCCAAGAACACCCTGTACCTGCAAATGCGC
AGTCTGAGGTCTGAGGACACGGCCATGTATTACTGTGTAAGACATGAACTTATTACTACGGTA
GTATCTACGGATTACTTTTCTATGGACTACTGGGGTCAAGGAACCTCAGTCACCGTCTCCTCA

The amino acid sequence of VL is as set forth in SEQ ID NO. 36, the nucleic acid encoding the VL is as set forth in SEQ ID NO. 37, and the LCDR1, LCDR2 and LCDR3 of the VL are as set forth in SEQ ID NOs. 38, 19 and 20, respectively.

<----------FR1-------->      CDR1       <-----FR2----->  CDR2
DIVLTQSPASLAVSLGQRATISCRASESVEYYGTSLMQWYQQKPGQPPKLLIYAASNVES
<--------------FR3-------------->    CDR3    <--FR4--->
GVPARFSGSGSGTDFSLNIHPVEEDDVAMYFCQQSRKVPLTFGAGTKLELK

### Nucleic acid sequence

GACATTGTGCTCACCCAATCTCCAGCTTCTTTGGCTGTGTCTCTAGGGCAGAGAGCCACCATC
TCCTGTAGAGCCAGTGAAAGTGTTGAATATTATGGCACAAGTTTAATGCAGTGGTACCAACAG
AAACCAGGACAGCCACCCAAACTCCTCATCTATGCTGCATCCAACGTAGAATCTGGGGTCCCT
GCCAGGTTTAGTGGCAGTGGGTCTGGGACAGACTTCAGCCTCAACATCCATCCTGTGGAGGAG
GATGATGTTGCAATGTATTTCTGTCAGCAAAGTAGGAAGGTTCCGCTCACGTTCGGTGCTGGG
ACCAAGCTGGAGCTGAAA

### 178D4

The amino acid sequence of VH is as set forth in SEQ ID NO. 39, the nucleic acid encoding the VH is as set forth in SEQ ID NO. 40, and the HCDR1, HCDR2 and HCDR3 of the VH are as set forth in SEQ ID NOs. 41, 42 and 43, respectively.

<-----------FR1-------------> CDR1<----FR2----> CDR2
QVQLQQSGAELVKPGASVKLSCKASGYTFTSYDLNWVRQRPEQGLEWIGWTFPGDGRTKY
<---------------FR3-------------> CDR3 <-----FR4
NEKFKGKATLTTDKSSSTAYMHLSRLTSEDSAVYFCVRWDYYGRSTGYFDVWGAGTTVTV
-->
SS

### Nucleic acid sequence

CAGGTTCAGCTGCAGCAGTCTGGAGCTGAACTGGTAAAGCCTGGGGCTTCAGTGAAGTTGTCC
TGCAAGGCTTCTGGCTACACCTTCACAAGCTATGATCTAAACTGGGTGAGGCAGAGGCCTGAA
CAGGGACTTGAGTGGATTGGATGGACTTTTCCTGGAGATGGTCGTACTAAGTATAATGAGAAG
TTCAAGGGCAAGGCCACACTGACTACAGACAAATCCTCCAGCACAGCCTACATGCACCTCAGC
AGGCTGACATCTGAGGACTCTGCTGTCTATTTCTGTGTAAGATGGGATTACTACGGCAGAAGT
ACTGGGTACTTCGATGTCTGGGGCGCAGGGACCACGGTCACCGTCTCCTCA

The amino acid sequence of VL is as set forth in SEQ ID NO. 44, the nucleic acid encoding the VL is as set forth in SEQ ID NO. 45, and the LCDR1, LCDR2 and LCDR3 of the VL are as set forth in SEQ ID NOs. 46, 47 and 48, respectively.

<-----------FR1--------->   CDR1    <------FR2----->  CDR2 <--
QIVLTQSPAIMSASLGERVTMTCTASSSVSSSYLHWYQQRPGSSPKLWIYGTSNLASGVP
-----------FR3-------------->    CDR3    <--FR4--->
ARFSGSGSGTSYSLTISSMEAEDAATYYCHQYHRSPLTFGAGTKLELK

### Nucleic acid sequence

CAAATTGTTCTCACCCAGTCTCCAGCAATCATGTCTGCATCTCTCGGGGAACGGGTCACCATG
ACCTGCACTGCCAGCTCAAGTGTAAGTTCCAGTTACTTGCACTGGTACCAGCAGAGGCCAGGA
TCCTCCCCCAAACTCTGGATTTATGGCACATCCAACCTGGCTTCTGGAGTCCCAGCTCGCTTC
AGTGGCAGTGGGTCTGGGACCTCTTACTCTCTCACAATCAGCAGCATGGAGGCTGAAGATGCT
GCCACTTATTACTGCCACCAGTATCATCGTTCCCCGCTCACGTTCGGTGCTGGGACCAAGCTG
GAGCTGAAA

### 185O18

The amino acid sequence of VH is as set forth in SEQ ID NO. 49, the nucleic acid encoding the VH is as set forth in SEQ ID NO. 50, and the HCDR1, HCDR2 and HCDR3 of the VH are as set forth in SEQ ID NOs. 51, 52 and 53, respectively.

<------------FR1-------------> CDR1<----FR2----> CDR2
EVMLVESGRDLVKPGGSLKLSCAASGFTFSTYAMSWIRQTPEKRLEWVATITNYGTYTYY
<--------------FR3------------> CDR3 <----
PDSVKGRFTISRDNAKNTLYLQMRSLRSEDTAMYYCVRHELITTVVSTDYFSMDYWGQGT
-FR4->
SVTVSS

### Nucleic acid sequence

GAGGTGATGCTGGTGGAGTCTGGGAGAGACTTAGTGAAGCCTGGAGGATCCCTGAAACTCTCC
TGTGCAGCCTCTGGATTCACTTTCAGTACCTATGCCATGTCTTGGATTCGCCAGACTCCGGAG
AAGAGGCTGGAGTGGGTCGCAACCATTACTAATTATGGTACTTACACCTACTATCCAGACAGT
GTGAAGGGGCGATTCACCATCTCCAGAGACAATGCCAAGAACACCCTGTACCTGCAAATGCGC

AGTCTGAGGTCTGAGGACACGGCCATGTATTACTGTGTAAGACATGAACTTATTACTACGGTA
GTATCTACGGATTACTTTTCTATGGACTACTGGGGTCAAGGAACCTCAGTCACCGTCTCCTCA

The amino acid sequence of VL is as set forth in SEQ ID NO. 54, the nucleic acid encoding the VL is as set forth in SEQ ID NO. 55, and the LCDR1, LCDR2 and LCDR3 of the VL are as set forth in SEQ ID NOs. 56, 19 and 20, respectively.

<-----------FR1-----------> CDR1 <------FR2------> CDR2
DIVLTQSPASLAVSLGQRATISCRASESVEDYGTSLMQWYQQKPGQPPKLLIYAASNVES
<-----------------FR3-----------------> CDR3 <--FR4--->
GVPARFSGSGSGTDFSLNIHPVEEDDVAMYFCQQSRKVPLTFGAGTKLELK

### Nucleic acid sequence

GACATTGTGCTCACCCAATCTCCAGCTTCTTTGGCTGTGTCTCTAGGGCAGAGAGCCACCATC
TCCTGTAGAGCCAGTGAAAGTGTTGAAGATTATGGCACAAGTTTAATGCAGTGGTACCAACAG
AAACCAGGACAGCCACCCAAACTCCTCATCTATGCTGCATCCAACGTAGAATCTGGGGTCCCT
GCCAGGTTTAGTGGCAGTGGGTCTGGGACAGACTTCAGCCTCAACATCCATCCTGTGGAGGAG
GATGATGTTGCAATGTATTTCTGTCAGCAAAGTAGGAAGGTTCCGCTCACGTTCGGTGCTGGG
ACCAAGCTGGAGCTGAAA

### 193D22

The amino acid sequence of VH is as set forth in SEQ ID NO. 57, the nucleic acid encoding the VH is as set forth in SEQ ID NO. 58, and the HCDR1, HCDR2 and HCDR3 of the VH are as set forth in SEQ ID NOs. 59, 60 and 61, respectively.

<-------------FR1-------------> CDR1<----FR2-----> CDR2
EVKLVESGGGLVKPGGSLKLSCAASGFAFSSYDMSWVRQTPEKRLEWVATISNGGSYTYY
<--------------FR3-------------> CDR3 <---FR4
PDSVKGRFSISRDNARNTLYLQMSSLRSEDTALYYCARHPLYYGNYGWWYFDVWGAGTTV
--->
TVSS

### Nucleic acid sequence

GAAGTGAAGCTGGTGGAGTCTGGGGGAGGCTTAGTGAAGCCTGGAGGGTCCCTGAAACTCTCC
TGTGCAGCCTCTGGATTCGCTTTCAGTAGTTATGACATGTCTTGGGTTCGCCAGACTCCGGAG
AAGAGGCTGGAGTGGGTCGCAACCATTAGTAATGGTGGTAGTTACACCTACTATCCAGACAGT
GTGAAGGGCCGATTCTCCATCTCCAGAGACAATGCCAGGAACACCCTGTACCTGCAAATGAGC
AGTCTGAGGTCTGAGGACACGGCCTTGTATTACTGTGCAAGACATCCCCTCTATTATGGTAAC
TACGGCTGGTGGTACTTCGATGTCTGGGGCGCAGGGACCACGGTCACCGTCTCCTCA

The amino acid sequence of VL is as set forth in SEQ ID NO. 62, the nucleic acid encoding the VL is as set forth in SEQ ID NO. 63, and the LCDR1, LCDR2 and LCDR3 of the VL are as set forth in SEQ ID NOs. 64, 65 and 66, respectively.

<-----------FR1-----------> CDR1 <------FR2------> CDR2
DVVMTQTPLSLPVSLGDQASISCRSSQSLVYSNGNTYLYWYLQKPGQSPKLLIYKVSNRF
<-----------------FR3-----------------> CDR3 <---FR4--->
SGVPDRFSGSGSGTDFTLKISRVEAEDLGVYFCSQSTHVPLTFGAGTKLELK

### Nucleic acid sequence

GATGTTGTGATGACCCAAACTCCACTCTCCCTGCCTGTCAGTCTTGGAGATCAAGCCTCCATC
TCTTGCAGATCTAGTCAGAGCCTTGTATACAGTAATGGAAACACCTATTTATATTGGTACCTG
CAGAAGCCAGGCCAGTCTCCAAAGCTCCTGATCTACAAAGTTTCCAACCGATTTTCTGGGGTC
CCAGACAGGTTCAGTGGCAGTGGATCAGGGACAGATTTCACACTCAAGATCAGCAGAGTGGAG
GCTGAGGATCTGGGAGTTTATTTCTGCTCTCAAAGTACACATGTTCCTCTCACGTTCGGTGCT
GGGACCAAGCTGGAGCTGAAA

### 151N5

The amino acid sequence of VH is as set forth in SEQ ID NO. 67, the nucleic acid encoding the VH is as set forth in SEQ ID NO. 68, and the HCDR1, HCDR2 and HCDR3 of the VH are as set forth in SEQ ID NOs. 69, 70 and 71, respectively.

<-------------FR1-------------> CDR1<----FR2----> CDR2
EVQLVESGGGLVKPGGSLKLSCAASGFTFSDFYMYWVRQTPEKRLEWVATISDGGRYTYY
<---------------FR3-------------> CDR3 <----
PDRLKGRFTISRNIAKNNLYLQMSSLKSEDTAMYYCARGRSTMITTTRNYFALDYWGQGT
-FR4->
SVTVSS

### Nucleic acid sequence

GAAGTGCAACTGGTGGAGTCTGGGGGAGGCTTAGTGAAGCCTGGAGGGTCCCTGAAACTCTCC
TGTGCTGCCTCTGGATTCACTTTCAGTGACTTTTACATGTATTGGGTTCGCCAGACTCCGGAA
AAGAGGCTGGAGTGGGTCGCAACCATTAGTGATGGTGGTCGTTACACCTACTATCCAGACAGG
CTGAAGGGGCGATTCACCATCTCCAGAAACATTGCCAAGAACAACCTTTACCTGCAAATGAGC
AGTCTGAAGTCTGAGGACACAGCCATGTATTACTGTGCAAGAGGGAGATCTACTATGATTACG
ACGACGCGGAATTACTTTGCTTTGGACTACTGGGGTCAAGGAACCTCAGTCACCGTCTCCTCA

The amino acid sequence of VL is as set forth in SEQ ID NO. 72, the nucleic acid encoding the VL is as set forth in SEQ ID NO. 73, and the LCDR1, LCDR2 and LCDR3 of the VL are as set forth in SEQ ID NOs. 74, 75 and 76, respectively.

<-----------FR1-----------> CDR1 <-----FR2-----> CDR2 <---
DIQMTQSSSSFSVSLGDRVTITCKASEDIYNRLAWYQQKPGDAPRLLISGAIRLETGVPS
-----------FR3-------------> CDR3 <--FR4--->
RFSGSGSGKDYTLSITSLQTEDGATYYCQQYWRTPLTFGAGIKLELK

### Nucleic acid sequence

GACATCCAGATGACACAATCTTCATCCTCCTTTTCTGTATCTCTAGGAGACAGAGTCACCATT
ACTTGCAAGGCAAGTGAGGACATATATAATCGGTTAGCCTGGTATCAGCAGAAACCAGGAGAT
GCTCCTAGACTCTTGATATCTGGTGCTATCCGTTTGGAAACTGGGGTTCCTTCAAGATTCAGT
GGCAGTGGATCTGGAAAGGATTACACTCTCAGCATTACCAGTCTTCAGACTGAAGATGGTGCT
ACTTATTACTGTCAACAGTATTGGAGAACTCCGCTCACGTTCGGTGCTGGGATCAAGCTGGAG
CTGAAA

### 156N21

The amino acid sequence of VH is as set forth in SEQ ID NO. 77, the nucleic acid encoding the VH is as set forth in SEQ ID NO. 78, and the HCDR1, HCDR2 and HCDR3 of the VH are as set forth in SEQ ID NOs. 3, 4 and 79, respectively.

<-------------FR1-------------> CDR1<----FR2----> CDR2
EVKLVESGGGLVQPGGSRKLSCAASGFTFRDYGMAWVRQAPGKGPEWVAFISNLAYSIYY
<---------------FR3-------------> CDR3 <-----FR4
ADTVTGRFTISRENAKNTLYLEMSSLRSEDTAMYYCARGIHYSGRYYAMDYWGQGTSVTV
-->
SS

### Nucleic acid sequence

GAGGTGAAGCTGGTGGAGTCTGGGGGAGGCTTAGTGCAGCCTGGAGGGTCCCGGAAACTCTCC
TGTGCAGCCTCTGGATTCACTTTCCGTGACTACGGAATGGCGTGGGTTCGACAGGCTCCAGGG
AAGGGGCCTGAGTGGGTAGCATTCATTAGTAATTTGGCATATAGTATCTATTATGCAGACACT
GTGACGGGCCGATTCACCATCTCTAGAGAGAATGCCAAGAACACCCTGTACCTGGAAATGAGC
AGTCTGAGGTCTGAGGACACAGCCATGTACTACTGTGCAAGAGGGATTCATTACTCCGGCCGT
TACTATGCTATGGACTACTGGGGTCAAGGAACCTCAGTCACCGTCTCCTCA

The amino acid sequence of VL is as set forth in SEQ ID NO. 80, the nucleic acid encoding the VL is as set forth in SEQ ID NO. 81, and the LCDR1, LCDR2 and LCDR3 of the VL are as set forth in SEQ ID NOs. 82, 83 and 84, respectively.

<----------FR1---------->      CDR1       <-----FR2----->  CDR2
DIVLTQSPASLAVSLGQRATISCRASKSVSTSGYSYMHWYQQKPGQPPKLLIYFASNLES
<---------------FR3-------------->     CDR3     <--FR4--->
GVPARFSGSGSGTDFTLNIHPVEEEDAATYYCQHSRELPPTFGGGTKLEIK

### Nucleic acid sequence

GACATTGTGCTGACACAGTCTCCTGCTTCCTTAGCTGTATCTCTGGGGCAGAGGGCCACCATC
TCATGTAGGGCCAGCAAAAGTGTCAGTACATCTGGCTATAGTTATATGCACTGGTACCAACAG
AAACCAGGACAGCCACCCAAACTCCTCATCTATTTTGCATCCAACCTAGAATCTGGGGTCCCT
GCCAGGTTCAGTGGCAGTGGGTCTGGGACAGACTTCACCCTCAACATCCATCCTGTGGAGGAG
GAGGATGCTGCAACCTATTACTGTCAGCACAGTAGGGAGCTTCCTCCCACGTTCGGAGGGGGG
ACCAAGCTGGAAATAAAA

### 171P1

The amino acid sequence of VH is as set forth in SEQ ID NO. 85, the nucleic acid encoding the VH is as set forth in SEQ ID NO. 86, and the HCDR1, HCDR2 and HCDR3 of the VH are as set forth in SEQ ID NOs. 87, 88 and 89, respectively.

<-------------FR1-------------> CDR1<----FR2----> CDR2
QVQLEQPGAVSVRPGASVKLSCKASGYTFTSYWINWVKQRPGQGLEWIGNIYPSDSYTHY
<---------------FR3-------------> CDR3 <-----FR4->
NQKFKDKATLTVDKSSTTAYMQISSPTSEDSAVYYCTREGTVVYWGQGTLLTVSA

### Nucleic acid sequence

CAGGTCCAACTGGAGCAGCCTGGGGCTGTTTCGGTGAGGCCTGGGGCTTCAGTGAAGCTGTCC
TGCAAGGCTTCTGGCTACACCTTCACCAGCTACTGGATAAACTGGGTGAAGCAGAGGCCTGGA
CAAGGCCTTGAGTGGATCGGAAATATTTATCCTTCTGATAGTTATACTCACTACAATCAAAAG
TTCAAGGACAAGGCCACATTGACTGTAGACAAATCCTCCACCACGGCCTACATGCAGATCAGC
AGCCCGACATCTGAGGACTCTGCGGTCTATTACTGTACAAGAGAGGGGACGGTTGTTTACTGG
GGCCAAGGGACTCTGCTCACTGTCTCTGCAG

The amino acid sequence of VL is as set forth in SEQ ID NO. 90, the nucleic acid encoding the VL is as set forth in SEQ ID NO. 91, and the LCDR1, LCDR2 and LCDR3 of the VL are as set forth in SEQ ID NOs. 92, 93 and 94, respectively.

<-----------FR1-----------> CDR1 <------FR2-----> CDR2
DVVLTQTPLTLSVTIGQPASMSCKSSQSLLYSNGKTYLNWLFQRPGQSPKRLIYVVSKLD
<-----------------FR3-----------------> CDR3 <--FR4--->
SGVPDRFTGSGSGTDFTLKISRVEAEDLGVYYCVQGTHFPFTFGSGTKLEIK

### Nucleic acid sequence

GATGTTGTGCTGACCCAGACTCCACTCACTTTGTCGGTTACCATTGGACAACCAGCCTCTATG
TCTTGCAAGTCAAGTCAGAGCCTCTTGTATAGTAATGGAAAAACCTATTTGAATTGGTTATTC
CAGAGGCCAGGCCAGTCCCCAAAGCGCCTAATCTATGTGGTGTCTAAACTGGACTCTGGAGTC
CCTGACAGGTTCACTGGCAGTGGATCAGGAACAGATTTTACACTGAAAATCAGCAGAGTGGAG
GCTGAGGATTTGGGAGTTTATTACTGCGTGCAAGGTACACATTTTCCATTCACGTTCGGCTCG
GGGACAAAGTTGGAAATAAAA

### 6B5

The amino acid sequence of VH is as set forth in SEQ ID NO. 95, the nucleic acid encoding the VH is as set forth in SEQ ID NO. 96, and the HCDR1, HCDR2 and HCDR3 of the VH are as set forth in SEQ ID NOs. 97, 98 and 99, respectively.

<----------FR1----------> CDR1<----FR2----> CDR2
EVQLQQSGAELVKPGASVKLSCKASGYTFTTYWMHWVKQRPGQGLEWIGEIDPSDSYTNY
<-------------FR3-----------> CDR3 <----FR4->
NQKFKGKATLTVDKSSSTAYMQLSSLTSEDSAVYYCARSFHGNFFVYWGQGTLVTVSA

### Nucleic acid sequence

GAGGTCCAACTGCAGCAGTCTGGGGCTGAGCTTGTGAAGCCTGGGGCTTCAGTGAAGCTGTCC
TGCAAGGCTTCTGGCTACACCTTCACCACCTACTGGATGCACTGGGTGAAGCAGAGGCCTGGA
CAAGGCCTTGAGTGGATCGGAGAGATTGATCCTTCTGATAGTTATACTAACTACAATCAAAAG
TTCAAGGGCAAGGCCACGTTGACTGTAGACAAATCCTCCAGCACAGCCTACATGCAGCTCAGC
AGCCTGACATCTGAGGACTCTGCGGTCTATTACTGTGCAAGATCGTTTCATGGTAACTTTTTT
GTTTACTGGGGCCAAGGGACTCTGGTCACTGTCTCTGCA

The amino acid sequence of VL is as set forth in SEQ ID NO. 100, the nucleic acid encoding the VL is as set forth in SEQ ID NO. 101, and the LCDR1, LCDR2 and LCDR3 of the VL are as set forth in SEQ ID NOs. 102, 103 and 104, respectively.

<-----------FR1----------> CDR1 <-----FR2-----> CDR2 <----
QIVLTQSPAIMSASPGEKVTITCSASSSVSYMHWFQQKPGTSPKLWIYSTSNLASGVPAR
-----------FR3---------------> CDR3 <--FR4--->
FSGSGSGTSYSLTISRMEAEDAATYYCQQRSSYPLTFGAGTKLELK

### Nucleic acid sequence

CAAATTGTTCTCACCCAGTCTCCAGCAATCATGTCTGCATCTCCAGGGGAGAAGGTCACCATA
ACCTGCAGTGCCAGCTCAAGTGTAAGTTACATGCACTGGTTCCAGCAGAAGCCAGGCACTTCT
CCCAAACTCTGGATTTATAGCACATCCAACCTGGCTTCTGGAGTCCCTGCTCGCTTCAGTGGC
AGTGGATCTGGGACCTCTTACTCTCTCACAATCAGCCGAATGGAGGCTGAAGATGCTGCCACT

TATTACTGCCAGCAAAGGAGTAGTTACCCGCTCACGTTCGGTGCTGGGACCAAGCTGGAGCTG
AAA

### 6C9

The amino acid sequence of VH is as set forth in SEQ ID NO. 105, the nucleic acid encoding the VH is as set forth in SEQ ID NO. 106, and the HCDR1, HCDR2 and HCDR3 of the VH are as set forth in SEQ ID NOs. 107, 108 and 99, respectively.

<-------------FR1-------------> CDR1<----FR2----> CDR2
QVQLQQSGAELVKPGASVKLSCKASGYTFTNYYVHWVKQRPGQGLEWIGEIDPSDSYSNY
<---------------FR3-------------> CDR3 <-----FR4->
NQKFKGKATLTVDKSSSTAYMQLSSLTSEDSAVYYCARSFHGNFFVYWGQGTLVTVSA

### Nucleic acid sequence

CAGGTGCAGCTGCAGCAGTCTGGGGCTGAACTGGTGAAGCCTGGGGCTTCGGTGAAGTTATCC
TGCAAGGCTTCTGGCTACACCTTCACCAACTACTATGTGCACTGGGTGAAGCAGAGGCCTGGA
CAAGGCCTTGAGTGGATCGGAGAGATTGATCCTTCTGATAGTTATAGTAACTACAATCAAAAG
TTCAAGGGCAAGGCCACATTGACTGTAGACAAATCCTCCAGCACAGCCTACATGCAGCTCAGC
AGCCTGACATCTGAGGACTCTGCGGTCTATTACTGTGCAAGATCGTTTCATGGTAACTTTTTT
GTTTACTGGGGCCAAGGGACTCTGGTCACTGTCTCTGCA

The amino acid sequence of VL is as set forth in SEQ ID NO. 109, the nucleic acid encoding the VL is as set forth in SEQ ID NO. 110, and the LCDR1, LCDR2 and LCDR3 of the VL are as set forth in SEQ ID NOs. 102, 103 and 111, respectively.

<----------FR1---------> CDR1 <-----FR2-----> CDR2 <----
QIVLTQSPAIMSASLGEEITLTCSASSSVSYMHWFQQKPGTSPKLWIYSTSNLASGVPAR
----------FR3-------------> CDR3 <--FR4-->
FSGSGSGTSYSLTISRMEAEDAATYYCQQRSSYPPTFGGGTKLEIK

### Nucleic acid sequence

CAAATTGTTCTCACCCAGTCTCCAGCAATCATGTCTGCATCTCTAGGGGAGGAGATCACCCTA
ACCTGCAGTGCCAGCTCGAGTGTAAGTTACATGCACTGGTTCCAGCAGAAGCCAGGCACTTCT
CCCAAACTCTGGATTTATAGCACATCCAACCTGGCTTCTGGAGTCCCTGCTCGCTTCAGTGGC
AGTGGATCTGGGACCTCTTACTCTCTCACAATCAGCCGAATGGAGGCTGAAGATGCTGCCACT
TATTACTGCCAGCAAAGGAGTAGTTACCCACCGACGTTCGGTGGAGGCACCAAGCTGGAAATC
AAA

### 11G10

The amino acid sequence of VH is as set forth in SEQ ID NO. 112, the nucleic acid encoding the VH is as set forth in SEQ ID NO. 113, and the HCDR1, HCDR2 and HCDR3 of the VH are as set forth in SEQ ID NOs. 114, 98 and 115, respectively.

<-------------FR1--------------> CDR1<----FR2----> CDR2
EVQLQQSGAEIVKPGASVKLSCKASGYTFTSYWMHWVKQRPGQGLEWIGEIDPSDSYTNY
<---------------FR3------------> CDR3 <-----FR4->
NQKFKGKATLTVDKSSSTAYMQLRSLTSEDSAVYYCARSFHGNLFVYWGQGTLVTVSA
Nucleic acid sequence

GAGGTTCAACTGCAGCAGTCTGGGGCTGAGATTGTGAAGCCTGGGGCTTCAGTGAAGCTGTCC
TGCAAGGCTTCTGGCTACACCTTCACCAGCTACTGGATGCACTGGGTGAAGCAGAGGCCTGGA
CAAGGCCTTGAGTGGATCGGAGAGATTGATCCTTCTGATAGTTATACTAACTACAATCAAAAG
TTCAAGGGCAAGGCCACATTGACTGTAGACAAATCCTCCAGCACAGCCTACATGCAGCTCAGA
AGCCTGACATCTGAGGACTCTGCGGTCTATTACTGTGCAAGATCGTTTCATGGTAACCTTTTT
GTTTACTGGGGCCAAGGGACTCTGGTCACTGTCTCTGCA

The amino acid sequence of VL is as set forth in SEQ ID NO. 116, the nucleic acid encoding the VL is as set forth in SEQ ID NO. 117, and the LCDR1, LCDR2 and LCDR3 of the VL are as set forth in SEQ ID NOs. 102, 118 and 104, respectively.

<-----------FR1---------->   CDR1   <-----FR2----->   CDR2 <----
QIVLTQSPAIMSASPGEKVTITCSASSSVSYMHWFQQKPGTSPKLWIYSTSNLATGVPAR
-----------FR3-------------->   CDR3   <--FR4--->
FSGSGSGTSYSLTISRMEAEDAATYYCQQRSSYPLTFGAGTKLEIK

### Nucleic acid sequence

CAAATTGTTCTCACCCAGTCTCCAGCAATCATGTCTGCATCTCCAGGGGAGAAGGTCACCATA
ACCTGCAGTGCCAGCTCAAGTGTAAGTTACATGCACTGGTTCCAGCAGAAGCCAGGCACTTCT
CCCAAACTCTGGATTTATAGCACATCCAACCTGGCCACTGGAGTCCCTGCTCGCTTCAGTGGC
AGTGGATCTGGGACCTCTTACTCTCTCACAATCAGCCGAATGGAGGCTGAAGATGCTGCCACT
TATTACTGCCAGCAAAGGAGTAGTTACCCGCTCACGTTCGGTGCTGGGACCAAGCTGGAAATC
AAA

### 7C8

The amino acid sequence of VH is as set forth in SEQ ID NO. 119, the nucleic acid encoding the VH is as set forth in SEQ ID NO. 120, and the HCDR1, HCDR2 and HCDR3 of the VH are as set forth in SEQ ID NOs. 121, 122 and 123, respectively.

<-------------FR1-------------> CDR1 <-----FR2-----> CDR2
QVTLKESGPGILQPSQTLSLTCSFSGFSLSTSGMGVSWIRQPSGKGLEWLAHIYWDDDKR
<-----------------FR3-----------------> CDR3 <---FR4--->
YNPSLKSRLTISKDTSRNQVFLKITSVDTADTATYYCARYGKGFAYWGQGTLVTVSA

### Nucleic acid sequence

CAGGTTACTCTGAAAGAGTCTGGCCCTGGGATATTGCAGCCCTCCCAGACCCTCAGTCTGACT
TGTTCTTTCTCTGGGTTTTCACTGAGCACTTCTGGTATGGGTGTGAGCTGGATTCGTCAGCCT
TCAGGAAAGGGTCTGGAGTGGCTGGCACACATTTACTGGGATGATGACAAGCGCTATAACCCA
TCCCTGAAGAGCCGGCTCACAATCTCCAAGGATACCTCCAGAAACCAGGTATTCCTCAAGATC
ACCAGTGTGGACACTGCAGATACTGCCACATACTACTGTGCTCGGTATGGTAAAGGGTTTGCT
TACTGGGGCCAAGGGACTCTGGTCACTGTCTCTGCA

The amino acid sequence of VL is as set forth in SEQ ID NO. 124, the nucleic acid encoding the VL is as set forth in SEQ ID NO. 125, and the LCDR1, LCDR2 and LCDR3 of the VL are as set forth in SEQ ID NOs. 126, 65 and 127, respectively.

<---------FR1--------->      CDR1     <-----FR2-----> CDR2

DVLMTQTPLSLPVSLGDQASISCRSSQSIVHSNGNTYLEWYLQKPGQSPKLLIYKVSNRF
<---------------FR3-------------->    CDR3    <--FR4--->
SGVPDRFSGSGSGTDFTLKISRVEAEDLGVYYCFQGSHVPLTFGAGTKLELK

### Nucleic acid sequence

GATGTTTTGATGACCCAAACTCCACTCTCCCTGCCTGTCAGTCTTGGAGATCAAGCCTCCATC
TCTTGCAGATCTAGTCAGAGCATTGTACATAGTAATGGAAACACCTATTTAGAATGGTACCTG
CAGAAACCAGGCCAGTCTCCAAAGCTCCTGATCTACAAAGTTTCCAACCGATTTTCTGGGGTC
CCAGACAGGTTCAGTGGCAGTGGATCAGGGACAGATTTCACACTCAAGATCAGCAGAGTGGAG
GCTGAGGATCTGGGAGTTTATTACTGCTTTCAAGGTTCACATGTTCCGCTCACGTTCGGTGCT
GGGACCAAGCTGGAGCTGAAAC

### 174J17

The amino acid sequence of VH is as set forth in SEQ ID NO. 128, the nucleic acid encoding the VH is as set forth in SEQ ID NO. 129, and the HCDR1, HCDR2 and HCDR3 of the VH are as set forth in SEQ ID NOs. 130, 131 and 279, respectively.

<-----------FR1-------------> CDR1<----FR2----> CDR2
EVQLVESGGGLVKPGGSLKLSCAASGFTLSDYYMYWVRQTPEKRLEWVATISDGGRYTYY
<---------------FR3-------------> CDR3 <----
PDSVKGRFTISRDIAKNNLYIQMSSLRSEDTAMYYCARGRSTMITTTRNYLALDYWGQGT
-FR4->
SVTVSS

### Nucleic acid sequence

GAAGTGCAGCTGGTGGAGTCTGGGGGAGGCTTAGTGAAGCCTGGAGGGTCCCTGAAACTCTCC
TGTGCAGCCTCTGGATTCACTCTCAGTGACTATTACATGTATTGGGTTCGCCAGACTCCGGAA
AAGAGGCTGGAGTGGGTCGCAACCATTAGTGATGGTGGTCGTTACACCTACTATCCAGATAGT
GTGAAGGGGCGATTCACCATCTCCAGAGACATTGCCAAGAACAACCTGTATATTCAAATGAGC
AGTCTGAGGTCTGAGGACACAGCCATGTATTACTGTGCAAGAGGGAGATCTACTATGATTACG
ACGACGCGGAATTACCTTGCTTTGGACTACTGGGGTCAAGGAACCTCAGTCACCGTCTCCTCA

The amino acid sequence of VL is as set forth in SEQ ID NO. 132, the nucleic acid encoding the VL is as set forth in SEQ ID NO. 133, and the LCDR1, LCDR2 and LCDR3 of the VL are as set forth in SEQ ID NOs. 74, 134 and 76, respectively.

<-----------FR1-----------> CDR1 <-----FR2-----> CDR2 <---
DIQMTQSSSSFSVSVGDRVTVTCKASEDIYNRLAWYQQRPGNAPRLLISGATSLETGVPS
-----------FR3-------------> CDR3 <--FR4--->
RFSGSGSGKDYILSITSLQTEDVGTYYCQQYWRTPLTFGEGTKLELK

### Nucleic acid sequence

GACATCCAGATGACACAATCTTCATCCTCCTTTTCTGTATCTGTAGGAGACAGAGTCACCGTT
ACTTGCAAGGCAAGTGAGGACATATATAATCGGTTAGCCTGGTATCAGCAGAGACCAGGAAAT
GCTCCTAGACTCTTAATATCTGGTGCAACCAGTTTGGAAACTGGGGTTCCTTCAAGATTCAGT
GGCAGTGGATCTGGAAAGGATTACATTCTCAGCATTACCAGTCTTCAGACTGAAGATGTTGGT
ACTTATTACTGTCAACAGTATTGGAGAACTCCGCTCACGTTCGGTGAAGGGACCAAGCTGGAG
CTGAAA

### 193M7

The amino acid sequence of VH is as set forth in SEQ ID NO. 135, the nucleic acid encoding the VH is as set forth in SEQ ID NO. 136, and the HCDR1, HCDR2 and HCDR3 of the VH are as set forth in SEQ ID NOs. 59, 137 and 138, respectively.

<-------------FR1--------------> CDR1<----FR2-----> CDR2
EVKLVESGGGLVKPGGSLKLSCAASGFAFSSYDMSWVRQTPEKRLEWVATISSRGTYTYY
<---------------FR3-------------> CDR3 <---FR4
PDSVKGRFTISRNNARNTLYLQMSSLRSEDTALYYCARHPLYYGNYGYWYFDVWGAGTTV
--->
TVSS

### Nucleic acid sequence

GAAGTGAAGCTGGTGGAGTCTGGGGGAGGCTTAGTGAAGCCTGGAGGGTCCCTGAAACTCTCC
TGTGCAGCCTCTGGATTCGCTTTCAGTAGCTATGACATGTCTTGGGTTCGCCAGACTCCGGAG
AAGAGGCTGGAGTGGGTCGCAACCATTAGTAGTCGTGGTACTTACACCTACTATCCAGACAGT
GTGAAGGGCCGATTCACCATCTCCAGAAACAATGCCAGGAACACCCTGTACCTGCAAATGAGC
AGTCTGAGGTCTGAGGACACGGCCTTGTATTACTGTGCAAGACATCCCCTCTATTATGGTAAC
TACGGCTACTGGTACTTCGATGTCTGGGGCGCAGGGACCACGGTCACCGTCTCCTCA

The amino acid sequence of VL is as set forth in SEQ ID NO. 139, the nucleic acid encoding the VL is as set forth in SEQ ID NO. 140, and the LCDR1, LCDR2 and LCDR3 of the VL are as set forth in SEQ ID NOs. 141, 65 and 66, respectively.

### Nucleic acid sequence

GATGTTGTGATGACCCAAACTCCACTCTCCCTGCCTGTCAGTCTTGGAGATCAAGCCTCCATT
TCTTGCAGATCTAGTCAGAGCCTTGTACACAGTGATGGAAACATCTATTTATATTGGTATCTG
CAGAAGCCAGGCCAGTCTCCAAAGCTCCTGATCTACAAAGTTTCCAACCGATTTTCTGGGGTC
CCAGACAGGTTCAGTGGCAGTGGATCAGGGACAGATTTCACACTCAAGATCAGCAGAGTGGAG
GCTGAGGATCTGGGAGTTTATTTCTGCTCTCAAAGTACACATGTTCCTCTCACGTTCGGTGCT
GGGACCAAGCTGGAGCTGAAA

### 15E11-2

The amino acid sequence of VH is as set forth in SEQ ID NO. 142, the nucleic acid encoding the VH is as set forth in SEQ ID NO. 143, and the HCDR1, HCDR2 and HCDR3 of the VH are as set forth in SEQ ID NOs. 121, 122 and 144, respectively.

<-------------FR1-------------> CDR1 <----FR2-----> CDR2
QVTLKESGPGILQPSQTLSLTCSFSGFSLSTSGMGVSWIRQPSGKGLEWLAHIYWDDDKR
<----------------FR3-------------> CDR3 <---FR4--->
YNPSLKSRLTISKVTSRNQVFLKITSVDTADTATYYCARYDKGFPYWGQGTLVTVSA

### Nucleic acid sequence

CAGGTTACTCTGAAAGAGTCTGGCCCTGGGATATTGCAGCCCTCCCAGACCCTCAGTCTGACT
TGTTCTTTCTCTGGGTTTTCACTGAGCACTTCTGGTATGGGTGTGAGCTGGATTCGTCAGCCT
TCAGGAAAGGGTCTGGAGTGGCTGGCACACATTTACTGGGATGATGACAAGCGCTATAACCCA
TCCCTGAAGAGCCGGCTCACAATCTCCAAGGTTACCTCCAGAAACCAGGTATTCCTCAAGATC
ACCAGTGTGGACACTGCAGATACTGCCACATACTACTGTGCTCGGTATGATAAAGGGTTTCCT
TACTGGGGCCAAGGGACTCTGGTCACTGTCTCTGCA

The amino acid sequence of VL is as set forth in SEQ ID NO. 145, the nucleic acid encoding the VL is as set forth in SEQ ID NO. 146, and the LCDR1, LCDR2 and LCDR3 of the VL are as set forth in SEQ ID NOs. 147, 148 and 149, respectively.

<-----------FR1----------->          CDR1          <------FR2------> CDR2
DTVMTQSPSSLAMSVGQKVTMSCKSSQSLLNSSNQKNYLAWYQQKPGQSPKLLIYFASTR
  <-----------------FR3-----------------> CDR3    <--FR4--->
ESGVPDRFIGSGSGTDFTLTISSVQAEDLADYFCQQHYSTPLTFGAGTKLELK

### Nucleic acid sequence

GACACTGTGATGACACAGTCTCCATCCTCCCTGGCTATGTCAGTAGGACAGAAGGTCACTATG
AGCTGCAAGTCCAGTCAGAGCCTTTTAAATAGTAGCAATCAAAAGAACTATTTGGCCTGGTAT
CAGCAGAAACCAGGACAGTCTCCTAAACTTCTGATATACTTTGCATCCACTAGGGAATCTGGG
GTCCCTGATCGCTTCATAGGCAGTGGATCTGGGACAGATTTCACTCTTACCATCAGCAGTGTG
CAGGCTGAAGACCTGGCAGATTACTTCTGTCAGCAACATTATAGCACGCCTCTCACGTTCGGT
GCTGGGACCAAGCTGGAGCTGAAA

### Example 3. Evaluation of phagocytic activity of anti-Aβ chimeric antibody

Microglia exist in the brain and mediate endogenous immune responses to central nervous system injuries and diseases, thereby exerting neuroprotective or neurotoxic effects. Through phagocytosis, microglia perform functions such as clearing dead cell debris, dendritic spines, extracellular matrix and invading pathogens. Microglia in patients with AD can clear Aβ through phagocytosis. *In vitro*, antibodies can be incubated with 5XFAD mouse brain sections. After the antibodies bind to Aβ in the brain sections, BV2 microglia are added. The Fc of the antibodies binds to the FcR on BV2, thereby mediating the phagocytosis by BV2. In the present invention, this method was used to evaluate the effect of the antibodies in terms of promoting phagocytosis of Aβ.

The chimeric antibody samples were each diluted with medium to 100 µg/mL and 10 µg/mL for later use. Pre-cut 10 µm FAD mouse brain sections were taken, and the sample application areas were circled using a histology pen. Then, 400 µL of each prepared test sample was gently added onto the sections, followed by incubation at 37°C for 1 h. During this period, mouse microglia BV2 were prepared and resuspended in a medium to 4 × 10⁵ cells/mL. After antibody incubation, the original samples on the sections were discarded, and 500 µL of cell suspension was added. After 24 h of culture, the medium was replaced once, and then the culture was continued for another 24 h, with a total culture duration of 48 h.

After phagocytosis, the sections were taken, and the supernatant from the sections was collected and centrifuged. The brain tissue on the sections was collected using 75 µL of guanidine hydrochloride lysis buffer. This collection was performed twice to obtain a total of 150 µL of lysate sample. After thorough mixing, the sample was kept on ice for further lysis for 1-2 h, during which uniform mixing could be performed multiple times. After the lysis was completed, the sample could be stored at -80°C or immediately detected by ELISA quantitation.

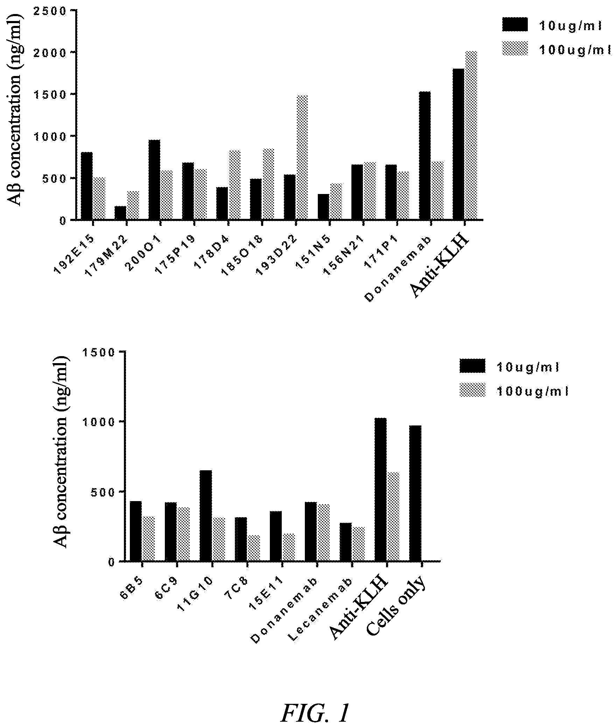

Aβ1-42 in the sample was quantified using the Human Aβ42 ELISA Kit (Cat: KHB3441, Invitrogen). The sample was centrifuged at 3000-4000 rpm, and 20 µL of the supernatant was taken and diluted 1000-fold. The detection was performed according to the kit instructions. Finally, the concentration of Aβ1-42 in the sample was calculated based on the standard curve. The phagocytosis results for different chimeric antibody samples are shown in FIG. 1. Clones such as 151N5, 200O1, 6B5 and 7C8 all promoted the phagocytosis of Aβ by microglia. Among them, the phagocytic activity of clones such as 200O1 and 192E15 was comparable to that of Donanemab, while the phagocytic activity of clones such as 7C8 was comparable to that of Lecanemab, indicating that these chimeric antibodies have great potential in clearing Aβ plaques.

### Example 4. Binding activity of anti-Aβ chimeric antibody to antigen

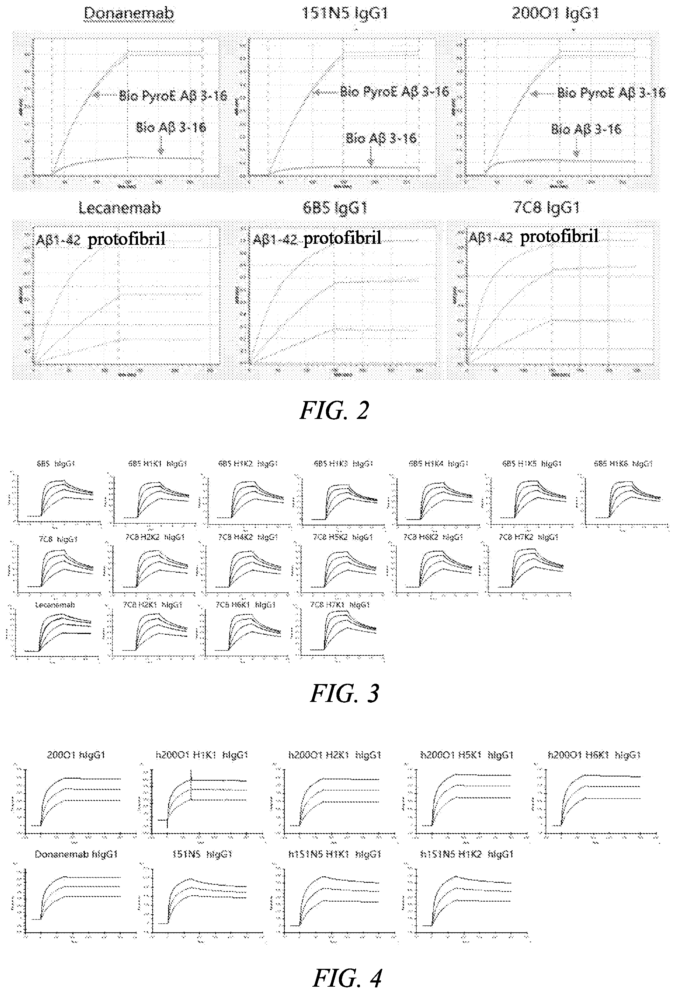

Using bio-layer interferometry (BLI/Gator Prime), the anti-Aβ chimeric antibodies were immobilized on a ProA probe, and Aβ1-42 protofibril was serially diluted with HBS-EP+buffer (Cytiva, BR10069) into three concentration points to detect the affinity of the anti-Aβ chimeric antibodies 7C8 and 6B5 for the antigen. In addition, pyroE Aβ3-16-biotin polypeptide was immobilized on the surface of an SA probe, and the serially diluted anti-Aβ chimeric antibodies 200O1 and 151N5 were then diluted with HBS-EP+ buffer to serve as analytes to detect the binding of pyroE Aβ3-16-biotin polypeptide to the chimeric antibodies. The detection results are shown in FIG. 2. The results showed that the anti-Aβ chimeric antibodies 151N5 and 200O1 exhibited strong binding to pyroE Aβ3-16, while the anti-Aβ chimeric antibodies 6B5 and 7C8 exhibited strong binding to protofibril.

### Example 5. Construction of humanized anti-Aβ antibody

### 1) Design of humanized antibodies

Based on the phagocytosis activity detection results of the mouse brain sections and the evaluation of the developability of the chimeric antibodies, the four chimeric antibodies (7C8, 151N5, 200O1 and 6B5) were humanized.

The sequence of chimeric antibody 7C8 was aligned with human antibody germline sequences, and human germline light chain genes (IMGT_hVK2-28 and IGKJ2*01) and human germline heavy chain genes (IMGT_hVH2-5 or IMGT_hVH4-59 and IGHJ4*01), which exhibited high homology, had identical key amino acid sequences that maintain the structural core (upper hydrophobic core) of the antibody, and showed a high frequency of occurrence in humans, were identified for murine antibody CDR grafting. Then, computer-assisted homology modelling was performed to analyse amino acid sequences of CDRs and surrounding frameworks thereof, avoiding the concentrated distribution of molecular surface charges or hydrophobic regions. A total of seven heavy chain variants (h7C8-H1 to H7) and two light chain variants (h7C8-K1 to K2) were designed.

Similarly, the sequence of chimeric antibody 151N5 was aligned with human antibody germline sequences, and human germline light chain genes (IMGT_hVK1-27 and IGKJ2*01) and human germline heavy chain genes (IMGT_hVH3-21 and IGHJ4*01) were identified for murine antibody CDR grafting. A total of six heavy chain variants (h151N5-H1 to H6) and nine light chain variants (h151N5-K1 to K9) were designed.

Similarly, the sequence of chimeric antibody 200O1 was aligned with human antibody germline sequences, and human germline light chain genes (IMGT_hVK1-27 and IGKJ2*01) and human germline heavy chain genes (IMGT_hVH2-26 or IMGT_hVH4-59 and IGHJ4*01) were identified for murine antibody CDR grafting. A total of nine heavy chain variants (h200O1-H1 to H9) and four light chain variants (h200O1-K1 to K4) were designed.

Similarly, the sequence of chimeric antibody 6B5 was aligned with human antibody germline sequences, and human germline light chain genes (IMGT_hVK3-20 or IMGT_hVK1-39 and IGKJ2*01) and human germline heavy chain genes (IMGT_hVH1-46 and IGHJ4*01) were identified for murine antibody CDR grafting. A total of nine heavy chain variants (h6B5-H1 to H5) and four light chain variants (h6B5-K1 to K6) were designed.

The variable region sequences of the heavy and light chains of the above humanized anti-Aβ antibodies are shown in Tables 3 and 4 below, respectively.

**Table 3. Variable region sequences of heavy chains of humanized anti-Aβ antibodies**

| Humanized anti-Aβ antibody | SEQ ID NO: | | | | |
|---|---|---|---|---|---|
| | VH | | HCDR1 | HCDR2 | HCDR3 |
| | Amino acid sequence | Nucleotide sequence | Amino acid sequence | Amino acid sequence | Amino acid sequence |
| h7C8 H1 | 150 | 151 | 121 | 122 | 123 |
| h7C8 H2 | 152 | 153 | 121 | 122 | 123 |
| h7C8 H3 | 154 | 155 | 121 | 122 | 123 |
| h7C8 H4 | 156 | 157 | 121 | 122 | 123 |
| h7C8 H5 | 158 | 159 | 121 | 122 | 123 |
| h7C8 H6 | 160 | 161 | 121 | 122 | 123 |
| h7C8 H7 | 162 | 163 | 121 | 122 | 123 |
| h200O1 H1 | 164 | 165 | 23 | 24 | 25 |
| h200O1 H2 | 166 | 167 | 23 | 24 | 25 |
| h200O1 H3 | 168 | 169 | 23 | 170 | 25 |
| h200O1 H4 | 171 | 172 | 23 | 173 | 25 |
| h200O1 H5 | 174 | 175 | 23 | 24 | 25 |
| h200O1 H6 | 176 | 177 | 23 | 24 | 25 |
| h200O1 H7 | 178 | 179 | 23 | 170 | 25 |
| h200O1 H8 | 180 | 181 | 23 | 173 | 25 |
| h200O1 H9 | 182 | 183 | 23 | 184 | 25 |
| h6B5 H1 | 185 | 186 | 97 | 98 | 99 |
| h6B5 H2 | 187 | 188 | 97 | 98 | 189 |
| h6B5 H3 | 190 | 191 | 192 | 98 | 189 |
| h6B5 H4 | 193 | 194 | 192 | 98 | 189 |
| h6B5 H5 | 195 | 196 | 192 | 98 | 189 |
| h151N5 H1 | 197 | 198 | 69 | 70 | 71 |
| h151N5 H2 | 199 | 200 | 201 | 202 | 71 |
| h151N5 H3 | 203 | 204 | 201 | 205 | 71 |
| h151N5 H4 | 206 | 207 | 201 | 205 | 279 |
| h151N5 H5 | 208 | 209 | 201 | 205 | 210 |
| h151N5 H6 | 211 | 212 | 201 | 213 | 210 |

### h7C8 H1

The amino acid sequence of the heavy chain VH is as set forth in SEQ ID NO. 150, the nucleic acid encoding the heavy chain VH is as set forth in SEQ ID NO. 151, and the CDR1, CDR2 and CDR3 of the heavy chain VH are as set forth in SEQ ID NOs. 121, 122 and 123, respectively.

<-----------FR1-------------->  CDR1 <----FR2----->   CDR2
QVTLKESGPALVKPTQTLTLTCTFSGFSLSTSGMGVSWIRQPPGKALEWLAHIYWDDDKRYN
     <-------------------FR3------------->  CDR3 <-----FR4->
PSLKSRLTITKDTSRNQVVLTMTNMDPVDTATYYCARYGKGFAYWGQGTLVTVSS

### Nucleic acid sequence

CAAGTGACCCTGAAAGAGTCTGGCCCCGCTCTGGTCAAGCCCACACAGACACTGACCCTGACC
TGTACCTTCAGCGGCTTCAGCCTGAGCACATCTGGCATGGGAGTGTCCTGGATCAGACAGCCT
CCTGGCAAGGCTCTGGAATGGCTGGCTCACATCTACTGGGACGACGACAAGAGATACAACCCC
AGCCTGAAGTCCAGACTGACCATCACCAAGGACACCAGCAGAAACCAGGTGGTGCTGACCATG
ACAAACATGGACCCTGTGGACACCGCCACCTACTACTGTGCCAGATACGGCAAGGGCTTCGCC
TACTGGGGACAGGGAACACTGGTCACCGTTAGCTCT

### h7C8 H2

The amino acid sequence of the heavy chain VH is as set forth in SEQ ID NO. 152, the nucleic acid encoding the heavy chain VH is as set forth in SEQ ID NO. 153, and the CDR1, CDR2 and CDR3 of the heavy chain VH are as set forth in SEQ ID NOs. 121, 122 and 123, respectively.

<-----------FR1-------------->  CDR1 <----FR2----->   CDR2
QVTLKESGPALVKPTQTLTLTCTFSGFSLSTSGMGVSWIRQPPGKALEWLAHIYWDDDKRYN
      <-----------------FR3------------->  CDR3 <-----FR4->
PSLKSRLTITKDTSKNQVVLTMTNMDPVDTATYYCARYGKGFAYWGQGTLVTVSS

### Nucleic acid sequence

CAAGTGACCCTGAAAGAGTCTGGCCCCGCTCTGGTCAAGCCCACACAGACACTGACCCTGACC
TGTACCTTCAGCGGCTTCAGCCTGAGCACATCTGGCATGGGAGTGTCCTGGATCAGACAGCCT
CCTGGCAAGGCTCTGGAATGGCTGGCTCACATCTACTGGGACGACGACAAGAGATACAACCCC
AGCCTGAAGTCCAGACTGACCATCACCAAGGACACCAGCAAAAACCAGGTGGTGCTGACCATG
ACAAACATGGACCCTGTGGACACCGCCACCTACTACTGTGCCAGATACGGCAAGGGCTTCGCC
TACTGGGGACAGGGAACACTGGTCACCGTTAGCTCT

### h7C8 H3

The amino acid sequence of the heavy chain VH is as set forth in SEQ ID NO. 154, the nucleic acid encoding the heavy chain VH is as set forth in SEQ ID NO. 155, and the CDR1, CDR2 and CDR3 of the heavy chain VH are as set forth in SEQ ID NOs. 121, 122 and 123, respectively.

<-------------FR1-------------->  CDR1 <----FR2----->   CDR2
QVQLQESGPGLVKPSETLSLTCTFSGFSISTSGMGVSWIRQPPGKGLEWIGHIYWDDDKRYN
     <---------------FR3------------>  CDR3 <-----FR4->
PSLKSRVTISKDTSKNQFSLKLSSVTAADTAVYYCARYGKGFAYWGQGTLVTVSS

### Nucleic acid sequence

CAGGTTCAGCTGCAAGAGTCTGGACCTGGCCTGGTCAAGCCTAGCGAGACACTGAGCCTGACC
TGTACCTTCAGCGGCTTCAGCATCAGCACATCTGGCATGGGAGTGTCCTGGATCAGACAGCCT

CCTGGCAAAGGCCTGGAATGGATCGGCCACATCTACTGGGACGACGACAAGAGATACAACCCC
AGCCTGAAGTCCAGAGTGACCATCAGCAAGGACACCAGCAAAAACCAGTTCTCCCTGAAGCTG
AGCAGCGTGACAGCCGCTGATACCGCCGTGTACTACTGTGCCAGATACGGCAAGGGCTTCGCC
TACTGGGGACAGGGAACACTGGTCACCGTTAGCTCT

### h7C8 H4

The amino acid sequence of the heavy chain VH is as set forth in SEQ ID NO. 156, the nucleic acid encoding the heavy chain VH is as set forth in SEQ ID NO. 157, and the CDR1, CDR2 and CDR3 of the heavy chain VH are as set forth in SEQ ID NOs. 121, 122 and 123, respectively.

<-------------FR1-------------> CDR1 <----FR2----> CDR2
QVQLQESGPGLVKPSETLSLTCTFSGFSLSTSGMGVSWIRQPPGKGLEWLAHIYWDDDKRYN
<---------------FR3-------------> CDR3 <-----FR4->
PSLKSRVTISKDTSRNQVSLKLSSVTAADTAVYYCARYGKGFAYWGQGTLVTVSS

### Nucleic acid sequence

CAGGTTCAGCTGCAAGAGTCTGGACCTGGCCTGGTCAAGCCTAGCGAGACACTGAGCCTGACC
TGTACCTTCAGCGGCTTCAGCCTGAGCACATCTGGCATGGGAGTGTCCTGGATCAGACAGCCT
CCTGGCAAAGGCCTGGAATGGCTCGCCCACATCTACTGGGACGACGACAAGAGATACAACCCC
AGCCTGAAGTCCAGAGTGACCATCAGCAAGGACACCAGCAGAAACCAGGTGTCCCTGAAGCTG
AGCAGCGTGACAGCCGCTGATACCGCCGTGTACTACTGTGCCAGATACGGCAAGGGCTTCGCC
TACTGGGGACAGGGAACACTGGTCACCGTTAGCTCT

### h7C8 H5

The amino acid sequence of the heavy chain VH is as set forth in SEQ ID NO. 158, the nucleic acid encoding the heavy chain VH is as set forth in SEQ ID NO. 159, and the CDR1, CDR2 and CDR3 of the heavy chain VH are as set forth in SEQ ID NOs. 121, 122 and 123, respectively.

<-------------FR1-------------->  CDR1 <----FR2----->   CDR2
QVQLQESGPGLVKPSETLSLTCTFSGFSLSTSGMGVSWIRQPPGKGLEWIAHIYWDDDKRYN
      <----------------FR3------------->  CDR3 <-----FR4->
PSLKSRVTISKDTSKNQVSLKLSSVTAADTAVYYCARYGKGFAYWGQGTLVTVSS

### Nucleic acid sequence

CAGGTTCAGCTGCAAGAGTCTGGACCTGGCCTGGTCAAGCCTAGCGAGACACTGAGCCTGACC
TGTACCTTCAGCGGCTTCAGCCTGAGCACATCTGGCATGGGAGTGTCCTGGATCAGACAGCCT
CCTGGCAAAGGCCTGGAATGGATCGCCCACATCTACTGGGACGACGACAAGAGATACAACCCC
AGCCTGAAGTCCAGAGTGACCATCAGCAAGGACACCAGCAAAAACCAGGTGTCCCTGAAGCTG
AGCAGCGTGACAGCCGCTGATACCGCCGTGTACTACTGTGCCAGATACGGCAAGGGCTTCGCC
TACTGGGGACAGGGAACACTGGTCACCGTTAGCTCT

### h7C8 H6

The amino acid sequence of the heavy chain VH is as set forth in SEQ ID NO. 160, the nucleic acid encoding the heavy chain VH is as set forth in SEQ ID NO. 161, and the CDR1, CDR2 and CDR3 of the heavy chain VH are as set forth in SEQ ID NOs. 121, 122 and 123, respectively.

<-------------FR1-------------->  CDR1 <----FR2----->   CDR2
QVQLQESGPGLVKPSETLSLTCTFSGFSISTSGMGVSWIRQPPGKGLEWIGHIYWDDDKRYN
     <---------------FR3------------->  CDR3 <-----FR4->
PSLKSRVTISKDTSKNQVSLKLSSVTAADTAVYYCARYGKGFAYWGQGTLVTVSS

### Nucleic acid sequence

CAGGTTCAGCTGCAAGAGTCTGGACCTGGCCTGGTCAAGCCTAGCGAGACACTGAGCCTGACC
TGTACCTTCAGCGGCTTCAGCATCAGCACATCTGGCATGGGAGTGTCCTGGATCAGACAGCCT
CCTGGCAAAGGCCTGGAATGGATCGGCCACATCTACTGGGACGACGACAAGAGATACAACCCC
AGCCTGAAGTCCAGAGTGACCATCAGCAAGGACACCAGCAAAAACCAGGTGTCCCTGAAGCTG
AGCAGCGTGACAGCCGCTGATACCGCCGTGTACTACTGTGCCAGATACGGCAAGGGCTTCGCC
TACTGGGGACAGGGAACACTGGTCACCGTTAGCTCT

### h7C8 H7

The amino acid sequence of the heavy chain VH is as set forth in SEQ ID NO. 162, the nucleic acid encoding the heavy chain VH is as set forth in SEQ ID NO. 163, and the CDR1, CDR2 and CDR3 of the heavy chain VH are as set forth in SEQ ID NOs. 121, 122 and 123, respectively.

<-----------FR1-------------->  CDR1  <----FR2----->    CDR2
QVQLQESGPGLVKPSETLSLTCTVSGFSISTSGMGVSWIRQPPGKGLEWIGHIYWDDDKRYN
      <---------------FR3------------->  CDR3  <-----FR4->
PSLKSRVTISKDTSKNQVSLKLSSVTAADTAVYYCARYGKGFAYWGQGTLVTVSS

### Nucleic acid sequence

CAGGTTCAGCTGCAAGAGTCTGGACCTGGCCTGGTCAAGCCTAGCGAGACACTGAGCCTGACC
TGTACCGTCAGCGGCTTCAGCATCAGCACATCTGGCATGGGAGTGTCCTGGATCAGACAGCCT
CCTGGCAAAGGCCTGGAATGGATCGGCCACATCTACTGGGACGACGACAAGAGATACAACCCC
AGCCTGAAGTCCAGAGTGACCATCAGCAAGGACACCAGCAAAAACCAGGTGTCCCTGAAGCTG
AGCAGCGTGACAGCCGCTGATACCGCCGTGTACTACTGTGCCAGATACGGCAAGGGCTTCGCC
TACTGGGGACAGGGAACACTGGTCACCGTTAGCTCT

### h200O1 H1

The amino acid sequence of the heavy chain VH is as set forth in SEQ ID NO. 164, the nucleic acid encoding the heavy chain VH is as set forth in SEQ ID NO. 165, and the CDR1, CDR2 and CDR3 of the heavy chain VH are as set forth in SEQ ID NOs. 23, 24 and 25, respectively.

<-------------FR1-------------> CDR1<----FR2----> CDR2
QVTLKESGPVLVKPTETLTLTCTVSGFSLSRYIVHWYRQPPGKALEWLAMIWGGGRIDYNSA
<---------------FR3-------------> CDR3 <---FR4--->
LKSRLTISKDNSKSQVVLTMTNMDPVDTATYYCARNFGENAEAYWGQGTLVTVSS

### Nucleic acid sequence

CAAGTGACCCTGAAAGAAAGCGGCCCTGTGCTGGTCAAGCCCACCGAAACACTGACCCTGACC
TGTACCGTGTCCGGCTTCAGCCTGTCTCGGTACATCGTGCACTGGTACAGACAGCCTCCAGGC

AAGGCTCTGGAATGGCTGGCTATGATTTGGGGCGGAGGCAGAATCGACTACAACAGCGCCCTG
AAGTCCAGACTGACCATCAGCAAGGACAACAGCAAGAGCCAGGTGGTGCTGACCATGACCAAC
ATGGACCCTGTGGACACCGCCACCTACTACTGCGCTAGAAACTTCGGCGAGAACGCCGAGGCT
TACTGGGGACAGGGAACACTGGTCACCGTGTCTAGT

### h200O1 H2

The amino acid sequence of the heavy chain VH is as set forth in SEQ ID NO. 166, the nucleic acid encoding the heavy chain VH is as set forth in SEQ ID NO. 167, and the CDR1, CDR2 and CDR3 of the heavy chain VH are as set forth in SEQ ID NOs. 23, 24 and 25, respectively.

<-----------FR1-------------> CDR1<----FR2----> CDR2
QVTLKESGPVLVKPTETLTLTCTVSGFSLSRYIVHWYRQPPGKALEWLAMIWGGGRIDYNSA
<---------------FR3-------------> CDR3 <---FR4--->
LKSRLTISKDTSKSQVVLTMTNMDPVDTATYYCARNFGENAEAYWGQGTLVTVSS

### Nucleic acid sequence

CAAGTGACCCTGAAAGAAAGCGGCCCTGTGCTGGTCAAGCCCACCGAAACACTGACCCTGACC
TGTACCGTGTCCGGCTTCAGCCTGTCTCGGTACATCGTGCACTGGTACAGACAGCCTCCAGGC
AAGGCTCTGGAATGGCTGGCTATGATTTGGGGCGGAGGCAGAATCGACTACAACAGCGCCCTG
AAGTCCAGACTGACCATCAGCAAGGACACCAGCAAGAGCCAGGTGGTGCTGACCATGACCAAC
ATGGACCCTGTGGACACCGCCACCTACTACTGCGCTAGAAACTTCGGCGAGAACGCCGAGGCT
TACTGGGGACAGGGAACACTGGTCACCGTGTCTAGT

### h200O1 H3

The amino acid sequence of the heavy chain VH is as set forth in SEQ ID NO. 168, the nucleic acid encoding the heavy chain VH is as set forth in SEQ ID NO. 169, and the CDR1, CDR2 and CDR3 of the heavy chain VH are as set forth in SEQ ID NOs. 23, 170 and 25, respectively.

<-----------FR1-------------> CDR1<----FR2----> CDR2
QVTLKESGPVLVKPTETLTLTCTVSGFSLSRYIVHWYRQPPGKALEWLAMIFGGGRIDYNSA
<---------------FR3------------> CDR3 <---FR4--->
LKSRLTISKDTSKSQVVLTMTNMDPVDTATYYCARNFGENAEAYWGQGTLVTVSS

### Nucleic acid sequence

CAAGTGACCCTGAAAGAAAGCGGCCCTGTGCTGGTCAAGCCCACCGAAACACTGACCCTGACC
TGTACCGTGTCCGGCTTCAGCCTGTCTCGGTACATCGTGCACTGGTACAGACAGCCTCCAGGC
AAGGCTCTGGAATGGCTGGCTATGATTTTCGGCGGAGGCAGAATCGACTACAACAGCGCCCTG
AAGTCCAGACTGACCATCAGCAAGGACACCAGCAAGAGCCAGGTGGTGCTGACCATGACCAAC
ATGGACCCTGTGGACACCGCCACCTACTACTGCGCTAGAAACTTCGGCGAGAACGCCGAGGCT
TACTGGGGACAGGGAACACTGGTCACCGTGTCTAGT

### h200O1 H4

The amino acid sequence of the heavy chain VH is as set forth in SEQ ID NO. 171, the nucleic acid encoding the heavy chain VH is as set forth in SEQ ID NO. 172, and the CDR1, CDR2 and CDR3 of the heavy chain VH are as set forth in SEQ ID NOs. 23, 173 and 25, respectively.

<------------FR1-------------> CDR1<----FR2----->   CDR2
QVTLKESGPVLVKPTETLTLTCTVSGFSLSRYIVHWYRQPPGKALEWLAMIFGSGRIDYNSA
    <---------------FR3------------->    CDR3   <---FR4--->
LKSRLTISKDTSKSQVVLTMTNMDPVDTATYYCARNFGENAEAYWGQGTLVTVSS

### Nucleic acid sequence

CAAGTGACCCTGAAAGAAAGCGGCCCTGTGCTGGTCAAGCCCACCGAAACACTGACCCTGACC
TGTACCGTGTCCGGCTTCAGCCTGTCTCGGTACATCGTGCACTGGTACAGACAGCCTCCAGGC
AAGGCTCTGGAATGGCTGGCTATGATTTTCGGCAGCGGCAGAATCGACTACAACAGCGCCCTG
AAGTCCAGACTGACCATCAGCAAGGACACCAGCAAGAGCCAGGTGGTGCTGACCATGACCAAC
ATGGACCCTGTGGACACCGCCACCTACTACTGCGCTAGAAACTTCGGCGAGAACGCCGAGGCT
TACTGGGGACAGGGAACACTGGTCACCGTGTCTAGT

### h200O1 H5

The amino acid sequence of the heavy chain VH is as set forth in SEQ ID NO. 174, the nucleic acid encoding the heavy chain VH is as set forth in SEQ ID NO. 175, and the CDR1, CDR2 and CDR3 of the heavy chain VH are as set forth in SEQ ID NOs. 23, 24 and 25, respectively.

<-------------FR1-------------> CDR1<----FR2----> CDR2
QVQLQESGPGLVKPSETLSLTCTVSGFSLSRYIVHWYRQPPGKGLEWIGMIWGGGRIDYNSA
<----------------FR3-------------> CDR3 <---FR4--->
LKSRVTISKDNSKSQVSLKLSSVTAADTAVYYCARNFGENAEAYWGQGTLVTVSS

### Nucleic acid sequence

CAGGTTCAGCTGCAAGAGTCTGGACCTGGCCTGGTCAAGCCTAGCGAGACACTGAGCCTGACC
TGTACCGTGTCTGGCTTCAGCCTGAGCCGGTACATCGTGCACTGGTACAGACAGCCTCCAGGC
AAAGGCCTGGAATGGATCGGCATGATTTGGGGCGGAGGCAGAATCGACTACAACAGCGCCCTG
AAGTCCAGAGTGACCATCAGCAAGGACAACAGCAAGAGCCAGGTGTCCCTGAAGCTGAGCAGC
GTGACAGCCGCTGATACCGCCGTGTACTACTGCGCCAGAAACTTCGGCGAGAACGCCGAGGCT
TATTGGGGCCAGGGAACACTGGTCACCGTGTCTAGT

### h200O1 H6

The amino acid sequence of the heavy chain VH is as set forth in SEQ ID NO. 176, the nucleic acid encoding the heavy chain VH is as set forth in SEQ ID NO. 177, and the CDR1, CDR2 and CDR3 of the heavy chain VH are as set forth in SEQ ID NOs. 23, 24 and 25, respectively.

<-------------FR1-------------> CDR1<----FR2----> CDR2
QVQLQESGPGLVKPSETLSLTCTVSGFSLSRYIVHWYRQPPGKGLEWIGMIWGGGRIDYNSA
<-----------------FR3-------------> CDR3 <---FR4--->
LKSRVTISKDTSKNQVSLKLSSVTAADTAVYYCARNFGENAEAYWGQGTLVTVSS

### Nucleic acid sequence

CAGGTTCAGCTGCAAGAGTCTGGACCTGGCCTGGTCAAGCCTAGCGAGACACTGAGCCTGACC
TGTACCGTGTCTGGCTTCAGCCTGAGCCGGTACATCGTGCACTGGTACAGACAGCCTCCAGGC

AAAGGCCTGGAATGGATCGGCATGATTTGGGGCGGAGGCAGAATCGACTACAACAGCGCCCTG
AAGTCCAGAGTGACCATCAGCAAGGACACCAGCAAGAACCAGGTGTCCCTGAAGCTGAGCAGC
GTGACAGCCGCTGATACCGCCGTGTACTACTGCGCCAGAAACTTCGGCGAGAACGCCGAGGCT
TATTGGGGCCAGGGAACACTGGTCACCGTGTCTAGT

### h200O1 H7

The amino acid sequence of the heavy chain VH is as set forth in SEQ ID NO. 178, the nucleic acid encoding the heavy chain VH is as set forth in SEQ ID NO. 179, and the CDR1, CDR2 and CDR3 of the heavy chain VH are as set forth in SEQ ID NOs. 23, 170 and 25, respectively.

<-------------FR1-------------> CDR1<----FR2----> CDR2
QVQLQESGPGLVKPSETLSLTCTVSGFSLSRYIVHWYRQPPGKGLEWIGMIFGGGRIDYNSA
<----------------FR3-------------> CDR3 <---FR4--->
LKSRVTISKDTSKNQVSLKLSSVTAADTAVYYCARNFGENAEAYWGQGTLVTVSS

### Nucleic acid sequence

CAGGTTCAGCTGCAAGAGTCTGGACCTGGCCTGGTCAAGCCTAGCGAGACACTGAGCCTGACC
TGTACCGTGTCTGGCTTCAGCCTGAGCCGGTACATCGTGCACTGGTACAGACAGCCTCCAGGC
AAAGGCCTGGAATGGATCGGCATGATTTTCGGCGGAGGCAGAATCGACTACAACAGCGCCCTG
AAGTCCAGAGTGACCATCAGCAAGGACACCAGCAAGAACCAGGTGTCCCTGAAGCTGAGCAGC
GTGACAGCCGCTGATACCGCCGTGTACTACTGCGCCAGAAACTTCGGCGAGAACGCCGAGGCT
TATTGGGGCCAGGGAACACTGGTCACCGTGTCTAGT

### h200O1 H8

The amino acid sequence of the heavy chain VH is as set forth in SEQ ID NO. 180, the nucleic acid encoding the heavy chain VH is as set forth in SEQ ID NO. 181, and the CDR1, CDR2 and CDR3 of the heavy chain VH are as set forth in SEQ ID NOs. 23, 173 and 25, respectively.

<-------------FR1-------------> CDR1<----FR2----> CDR2
QVQLQESGPGLVKPSETLSLTCTVSGFSLSRYIVHWYRQPPGKGLEWIGMIFGSGRIDYNSA
<---------------FR3-------------> CDR3 <---FR4--->
LKSRVTISKDTSKNQVSLKLSSVTAADTAVYYCARNFGENAEAYWGQGTLVTVSS

### Nucleic acid sequence

CAGGTTCAGCTGCAAGAGTCTGGACCTGGCCTGGTCAAGCCTAGCGAGACACTGAGCCTGACC
TGTACCGTGTCTGGCTTCAGCCTGAGCCGGTACATCGTGCACTGGTACAGACAGCCTCCAGGC
AAAGGCCTGGAATGGATCGGCATGATTTTCGGCAGCGGCAGAATCGACTACAACAGCGCCCTG
AAGTCCAGAGTGACCATCAGCAAGGACACCAGCAAGAACCAGGTGTCCCTGAAGCTGAGCAGC
GTGACAGCCGCTGATACCGCCGTGTACTACTGCGCCAGAAACTTCGGCGAGAACGCCGAGGCT
TATTGGGGCCAGGGAACACTGGTCACCGTGTCTAGT

### h200O1 H9

The amino acid sequence of the heavy chain VH is as set forth in SEQ ID NO. 182, the nucleic acid encoding the heavy chain VH is as set forth in SEQ ID NO. 183, and the CDR1, CDR2 and CDR3 of the heavy chain VH are as set forth in SEQ ID NOs. 23, 184 and 25, respectively.

<------------FR1-------------> CDR1<----FR2---->   CDR2
QVTLKESGPVLVKPTETLTLTCTVSGFSLSRYIVHWYRQPPGKALEWLAMIWSGGRIDYNSA
   <---------------FR3------------->   CDR3  <---FR4--->
LKSRLTISKDTSKSQVVLTMTNMDPVDTATYYCARNFGENAEAYWGQGTLVTVSS
[?]

### Nucleic acid sequence

CAAGTGACCCTGAAAGAAAGCGGCCCTGTGCTGGTCAAGCCCACCGAAACACTGACCCTGACC
TGTACCGTGTCCGGCTTCAGCCTGTCTCGGTACATCGTGCACTGGTACAGACAGCCTCCAGGC
AAGGCTCTGGAATGGCTGGCTATGATTTGGAGCGGAGGCAGAATCGACTACAACAGCGCCCTG
AAGTCCAGACTGACCATCAGCAAGGACACCAGCAAGAGCCAGGTGGTGCTGACCATGACCAAC
ATGGACCCTGTGGACACCGCCACCTACTACTGCGCTAGAAACTTCGGCGAGAACGCCGAGGCT
TACTGGGGACAGGGAACACTGGTCACCGTGTCTAGT

### h6B5 H1

The amino acid sequence of the heavy chain VH is as set forth in SEQ ID NO. 185, the nucleic acid encoding the heavy chain VH is as set forth in SEQ ID NO. 186, and the CDR1, CDR2 and CDR3 of the heavy chain VH are as set forth in SEQ ID NOs. 97, 98 and 99, respectively.

<-----------FR1-------------> CDR1<----FR2----> CDR2
QVQLVQSGAEVKKPGASVKVSCKASGYTFTTYWMHWVRQAPGQGLEWIGEIDPSDSYTNYNQ
<---------------FR3-------------> CDR3 <-----FR4->
KFKGRATLTVDKSSSTAYMELSSLRSEDTAVYYCARSFHGNFFVYWGQGTLVTVSS

### Nucleic acid sequence

CAGGTTCAGCTGGTTCAGTCTGGCGCCGAAGTGAAGAAACCTGGCGCCTCTGTGAAGGTGTCC
TGCAAGGCTAGCGGCTACACATTCACCACCTACTGGATGCACTGGGTCCGACAGGCTCCAGGA
CAGGGACTTGAGTGGATCGGCGAGATCGACCCTAGCGACAGCTACACCAACTACAACCAGAAA
TTCAAGGGCAGAGCGACCCTGACCGTGGACAAGTCTAGCAGCACCGCCTACATGGAACTGAGC
AGCCTGAGAAGCGAGGACACCGCCGTGTACTACTGCGCCAGAAGCTTCCACGGCAACTTTTTC
GTGTACTGGGGCCAGGGCACCCTGGTCACAGTTTCTTCT

### h6B5 H2

The amino acid sequence of the heavy chain VH is as set forth in SEQ ID NO. 187, the nucleic acid encoding the heavy chain VH is as set forth in SEQ ID NO. 188, and the CDR1, CDR2 and CDR3 of the heavy chain VH are as set forth in SEQ ID NOs. 97, 98 and 189, respectively.

<-------------FR1-------------> CDR1<----FR2----> CDR2
QVQLVQSGAEVKKPGASVKVSCKASGYTFTTYWMHWVRQAPGQGLEWMGEIDPSDSYTNYNQ
<----------------FR3-------------> CDR3 <----FR4->
KFKGRVTLTVDKSSSTAYMELSSLRSEDTAVYYCARSFHGNLFVYWGQGTLVTVSS

### Nucleic acid sequence

CAGGTTCAGCTGGTTCAGTCTGGCGCCGAAGTGAAGAAACCTGGCGCCTCTGTGAAGGTGTCC
TGCAAGGCTAGCGGCTACACATTCACCACCTACTGGATGCACTGGGTCCGACAGGCTCCAGGA

CAGGGACTTGAGTGGATGGGCGAGATCGACCCTAGCGACAGCTACACCAACTACAACCAGAAA
TTCAAGGGCAGAGTGACCCTGACCGTGGACAAGTCTAGCAGCACCGCCTACATGGAACTGAGC
AGCCTGAGAAGCGAGGACACCGCCGTGTACTACTGCGCCAGATCTTTCCACGGCAACCTGTTC
GTGTACTGGGGCCAGGGAACACTGGTCACCGTTAGCTCT

### h6B5 H3

The amino acid sequence of the heavy chain VH is as set forth in SEQ ID NO. 190, the nucleic acid encoding the heavy chain VH is as set forth in SEQ ID NO. 191, and the CDR1, CDR2 and CDR3 thereof are as set forth in SEQ ID NOs. 192, 98 and 189, respectively.

<------------FR1-------------> CDR1<----FR2----->   CDR2
QVQLVQSGAEVKKPGASVKVSCKASGYTFTSYWMHWVRQAPGQGLEWMGEIDPSDSYTNYNQ
    <---------------FR3------------>   CDR3  <-----FR4->
KFKGRVTLTVDKSTSTAYMELSSLRSEDTAVYYCARSFHGNLFVYWGQGTLVTVSS

### Nucleic acid sequence

CAGGTTCAGCTGGTTCAGTCTGGCGCCGAAGTGAAGAAACCTGGCGCCTCTGTGAAGGTGTCC
TGCAAGGCTAGCGGCTACACATTCACCAGCTACTGGATGCACTGGGTCCGACAGGCTCCAGGA
CAGGGACTTGAGTGGATGGGCGAGATCGACCCTAGCGACAGCTACACCAACTACAACCAGAAA
TTCAAGGGCAGAGTGACCCTGACCGTGGACAAGTCTACCAGCACCGCCTACATGGAACTGAGC
AGCCTGAGAAGCGAGGACACCGCCGTGTACTACTGCGCCAGATCTTTCCACGGCAACCTGTTC
GTGTACTGGGGCCAGGGAACACTGGTCACCGTTAGCTCT

### h6B5 H4

The amino acid sequence of the heavy chain VH is as set forth in SEQ ID NO. 193, the nucleic acid encoding the heavy chain VH is as set forth in SEQ ID NO. 194, and the CDR1, CDR2 and CDR3 of the heavy chain VH are as set forth in SEQ ID NOs. 192, 98 and 189, respectively.

### Nucleic acid sequence

CAGGTTCAGCTGGTTCAGTCTGGCGCCGAAGTGAAGAAACCTGGCGCCTCTGTGAAGGTGTCC
TGCAAGGCTAGCGGCTACACATTCACCAGCTACTGGATGCACTGGGTCCGACAGGCTCCAGGA
CAGGGACTTGAGTGGATGGGCGAGATCGACCCTAGCGACAGCTACACCAACTACAACCAGAAA
TTCAAGGGCAGAGTGACCATGACCGTGGACAAGTCTACCAGCACCGCCTACATGGAACTGAGC
AGCCTGAGAAGCGAGGACACCGCCGTGTACTACTGCGCCAGATCTTTCCACGGCAACCTGTTC
GTGTACTGGGGCCAGGGAACACTGGTCACCGTTAGCTCT

### h6B5 H5

The amino acid sequence of the heavy chain VH is as set forth in SEQ ID NO. 195, the nucleic acid encoding the heavy chain VH is as set forth in SEQ ID NO. 196, and the CDR1, CDR2 and CDR3 of the heavy chain VH are as set forth in SEQ ID NOs. 192, 98 and 189, respectively.

<-----------FR1-------------> CDR1<----FR2----> CDR2
QVQLVQSGAEVKKPGASVKVSCKASGYTFTSYWMHWVRQAPGQGLEWMGEIDPSDSYTNYNQ
<---------------FR3------------> CDR3 <----FR4->
KFKGRVTMTVDKSTSTVYMELSSLRSEDTAVYYCARSFHGNLFVYWGQGTLVTVSS

### Nucleic acid sequence

CAGGTTCAGCTGGTTCAGTCTGGCGCCGAAGTGAAGAAACCTGGCGCCTCTGTGAAGGTGTCC
TGCAAGGCTAGCGGCTACACATTCACCAGCTACTGGATGCACTGGGTCCGACAGGCTCCAGGA
CAGGGACTTGAGTGGATGGGCGAGATCGACCCTAGCGACAGCTACACCAACTACAACCAGAAA
TTCAAGGGCAGAGTGACCATGACCGTGGACAAGTCTACCAGCACCGTCTACATGGAACTGAGC
AGCCTGAGAAGCGAGGACACCGCCGTGTACTACTGCGCCAGATCTTTCCACGGCAACCTGTTC
GTGTACTGGGGCCAGGGAACACTGGTCACCGTTAGCTCT

### h151N5 H1

The amino acid sequence of the heavy chain VH is as set forth in SEQ ID NO. 197, the nucleic acid encoding the heavy chain VH is as set forth in SEQ ID NO. 198, and the CDR1, CDR2 and CDR3 of the heavy chain VH are as set forth in SEQ ID NOs. 69, 70 and 71, respectively.

<-------------FR1-------------> CDR1<----FR2-----> CDR2
EVQLVESGGGLVKPGGSLRLSCAASGFTFSDFYMYWVRQAPGKGLEWVATISDGGRYTYYPD
<---------------FR3------------> CDR3 <--------
RLKGRFTISRDIAKNSLYLQMNSLRAEDTAVYYCARGRSTMITTTRNYFALDYWGQGTLVTV
->
SS

### Nucleic acid sequence

GAGGTGCAGCTGGTTGAATCTGGCGGAGGACTGGTTAAGCCTGGCGGCTCTCTGAGACTGTCT
TGTGCCGCTTCTGGCTTCACCTTCAGCGACTTCTACATGTACTGGGTCCGACAGGCCCCTGGC
AAAGGACTGGAATGGGTGGCCACAATCAGCGACGGCGGCAGATACACCTACTATCCCGACAGA
CTGAAGGGCAGATTCACCATCAGCAGAGATATCGCCAAGAACAGCCTGTACCTGCAGATGAAC
TCCCTGAGAGCCGAGGACACCGCCGTGTACTATTGTGCCAGAGGCAGGTCCACCATGATCACC
ACCACCAGAAACTACTTCGCCCTGGACTACTGGGGCCAGGGAACACTGGTTACCGTGTCTAGT

### h151N5 H2

The amino acid sequence of the heavy chain VH is as set forth in SEQ ID NO. 199, the nucleic acid encoding the heavy chain VH is as set forth in SEQ ID NO. 200, and the CDR1, CDR2 and CDR3 of the heavy chain VH are as set forth in SEQ ID NOs. 201, 202 and 71, respectively.

<-------------FR1-------------> CDR1<----FR2-----> CDR2
EVQLVESGGGLVKPGGSLRLSCAASGFTFSDYYMYWVRQAPGKGLEWVATISDGGRYTYYPD
<---------------FR3-------------> CDR3 <--------
RVKGRFTISRDIAKNSLYLQMNSLRAEDTAVYYCARGRSTMITTTRNYFALDYWGQGTLVTV
->
SS

### Nucleic acid sequence

GAGGTGCAGCTGGTTGAATCTGGCGGAGGACTGGTTAAGCCTGGCGGCTCTCTGAGACTGTCT
TGTGCCGCTTCTGGCTTCACCTTCAGCGACTACTACATGTACTGGGTCCGACAGGCCCCTGGC
AAAGGACTGGAATGGGTGGCCACAATCAGCGACGGCGGCAGATACACCTACTATCCCGACAGA
GTGAAGGGCAGATTCACCATCAGCAGAGATATCGCCAAGAACAGCCTGTACCTGCAGATGAAC
TCCCTGAGAGCCGAGGACACCGCCGTGTACTATTGTGCCAGAGGCAGGTCCACCATGATCACC
ACCACCAGAAACTACTTCGCCCTGGACTACTGGGGCCAGGGAACACTGGTTACCGTGTCTAGT

### h151N5 H3

The amino acid sequence of the heavy chain VH is as set forth in SEQ ID NO. 203, the nucleic acid encoding the heavy chain VH is as set forth in SEQ ID NO. 204, and the CDR1, CDR2 and CDR3 of the heavy chain VH are as set forth in SEQ ID NOs. 201, 205 and 71, respectively.

<-------------FR1-------------> CDR1<----FR2----> CDR2
EVQLVESGGGLVKPGGSLRLSCAASGFTFSDYYMYWVRQAPGKGLEWVSTISDGGRYTYYAD
<-----------------FR3-----------> CDR3 <--------
RVKGRFTISRDIAKNSLYLQMNSLRAEDTAVYYCARGRSTMITTTRNYFALDYWGQGTLVTV
->
SS

### Nucleic acid sequence

GAGGTGCAGCTGGTTGAATCTGGCGGAGGACTGGTTAAGCCTGGCGGCTCTCTGAGACTGTCT
TGTGCCGCTTCTGGCTTCACCTTCAGCGACTACTACATGTACTGGGTCCGACAGGCCCCTGGC
AAAGGACTGGAATGGGTGTCCACAATCAGCGACGGCGGCAGATACACCTACTATGCCGACAGA
GTGAAGGGCAGATTCACCATCAGCAGAGATATCGCCAAGAACAGCCTGTACCTGCAGATGAAC
TCCCTGAGAGCCGAGGACACCGCCGTGTACTATTGTGCCAGAGGCAGGTCCACCATGATCACC
ACCACCAGAAACTACTTCGCCCTGGACTACTGGGGCCAGGGAACACTGGTTACCGTGTCTAGT

### h151N5 H4

The amino acid sequence of the heavy chain VH is as set forth in SEQ ID NO. 206, the nucleic acid encoding the heavy chain VH is as set forth in SEQ ID NO. 207, and the CDR1, CDR2 and CDR3 of the heavy chain VH are as set forth in SEQ ID NOs. 201, 205 and 279, respectively.

<-------------FR1-------------> CDR1<----FR2-----> CDR2
EVQLVESGGGLVKPGGSLRLSCAASGFTFSDYYMYWVRQAPGKGLEWVSTISDGGRYTYYAD
<----------------FR3-------------> CDR3 <--------
RVKGRFTISRDNAKNSLYLQMNSLRAEDTAVYYCARGRSTMITTTRNYLALDYWGQGTLVTV
->
SS

### Nucleic acid sequence

GAGGTGCAGCTGGTTGAATCTGGCGGAGGACTGGTTAAGCCTGGCGGCTCTCTGAGACTGTCT
TGTGCCGCTTCTGGCTTCACCTTCAGCGACTACTACATGTACTGGGTCCGACAGGCCCCTGGC
AAAGGACTGGAATGGGTGTCCACAATCAGCGACGGCGGCAGATACACCTACTATGCCGACAGA
GTGAAGGGCAGATTCACCATCAGCAGAGATAACGCCAAGAACAGCCTGTACCTGCAGATGAAC

TCCCTGAGAGCCGAGGACACCGCCGTGTACTATTGTGCCAGAGGCAGGTCCACCATGATCACC
ACCACCAGAAACTACCTGGCTCTGGACTACTGGGGCCAGGGAACACTGGTCACAGTGTCTAGT

### h151N5 H5

The amino acid sequence of the heavy chain VH is as set forth in SEQ ID NO. 208, the nucleic acid encoding the heavy chain VH is as set forth in SEQ ID NO. 209, and the CDR1, CDR2 and CDR3 of the heavy chain VH are as set forth in SEQ ID NOs. 201, 205 and 210, respectively.

<-------------FR1-------------> CDR1<----FR2-----> CDR2
EVQLVESGGGLVKPGGSLRLSCAASGFTFSDYYMYWVRQAPGKGLEWVSTISDGGRYTYYAD
<----------------FR3-------------> CDR3 <--------
RVKGRFTISRDNAKNSLYLQMNSLRAEDTAVYYCARGRSTLITTTRNYLALDYWGQGTLVTV
->
SS

### Nucleic acid sequence

GAGGTGCAGCTGGTTGAATCTGGCGGAGGACTGGTTAAGCCTGGCGGCTCTCTGAGACTGTCT
TGTGCCGCTTCTGGCTTCACCTTCAGCGACTACTACATGTACTGGGTCCGACAGGCCCCTGGC
AAAGGACTGGAATGGGTGTCCACAATCAGCGACGGCGGCAGATACACCTACTATGCCGACAGA
GTGAAGGGCAGATTCACCATCAGCAGAGATAACGCCAAGAACAGCCTGTACCTGCAGATGAAC
TCCCTGAGAGCCGAGGACACCGCCGTGTACTATTGTGCCAGAGGCAGGTCCACCCTGATCACC
ACCACCAGAAACTACCTGGCTCTGGACTACTGGGGCCAGGGAACACTGGTCACAGTGTCTAGT

### h151N5 H6

The amino acid sequence of the heavy chain VH is as set forth in SEQ ID NO. 211, the nucleic acid encoding the heavy chain VH is as set forth in SEQ ID NO. 212, and the CDR1, CDR2 and CDR3 of the heavy chain VH are as set forth in SEQ ID NOs. 201, 213 and 210, respectively.

<-------------FR1-------------> CDR1<----FR2-----> CDR2
EVQLVESGGGLVKPGGSLRLSCAASGFTFSDYYMYWVRQAPGKGLEWVSTISESGRYTYYAD
<----------------FR3-------------> CDR3 <--------
RVKGRFTISRDNAKNSLYLQMNSLRAEDTAVYYCARGRSTLITTTRNYLALDYWGQGTLVTV
->
SS

### Nucleic acid sequence

GAGGTGCAGCTGGTTGAATCTGGCGGAGGACTGGTTAAGCCTGGCGGCTCTCTGAGACTGTCT
TGTGCCGCTTCTGGCTTCACCTTCAGCGACTACTACATGTACTGGGTCCGACAGGCCCCTGGC
AAAGGACTGGAATGGGTGTCCACAATCAGCGAAAGCGGCAGATACACCTACTATGCCGACAGA
GTGAAGGGCAGATTCACCATCAGCAGAGATAACGCCAAGAACAGCCTGTACCTGCAGATGAAC
TCCCTGAGAGCCGAGGACACCGCCGTGTACTATTGTGCCAGAGGCAGGTCCACCCTGATCACC
ACCACCAGAAACTACCTGGCTCTGGACTACTGGGGCCAGGGAACACTGGTCACAGTGTCTAGT

**Table 4. Variable region sequences of light chains of humanized anti-Aβ antibodies**

| SEQ ID NO: | | | | | |
|---|---|---|---|---|---|
| Humanized anti-Aβ antibody | VL | | LCDR1 | LCDR2 | LCDR3 |
| | Amino acid sequence | Nucleotide sequence | Amino acid sequence | Amino acid sequence | Amino acid sequence |
| h7C8 K1 | 214 | 215 | 126 | 65 | 127 |
| h7C8 K2 | 216 | 217 | 218 | 65 | 127 |
| h200O1 K1 | 219 | 220 | 28 | 29 | 30 |
| h200O1 K2 | 221 | 222 | 223 | 29 | 30 |
| h200O1 K3 | 224 | 225 | 226 | 29 | 30 |
| h200O1 K4 | 227 | 228 | 226 | 229 | 30 |
| h6B5 K1 | 230 | 231 | 232 | 103 | 104 |
| h6B5 K2 | 233 | 234 | 232 | 235 | 104 |
| h6B5 K3 | 236 | 237 | 232 | 238 | 104 |
| h6B5 K4 | 239 | 240 | 232 | 103 | 104 |
| h6B5 K5 | 241 | 242 | 232 | 103 | 104 |
| h6B5 K6 | 243 | 244 | 232 | 245 | 104 |
| h151N5 K1 | 246 | 247 | 74 | 248 | 76 |
| h151N5 K2 | 249 | 250 | 74 | 248 | 76 |
| h151N5 K3 | 251 | 252 | 74 | 253 | 76 |
| h151N5 K4 | 254 | 255 | 256 | 257 | 76 |
| h151N5 K5 | 258 | 259 | 260 | 261 | 76 |
| h151N5 K6 | 262 | 263 | 260 | 261 | 264 |
| h151N5 K7 | 265 | 266 | 74 | 75 | 76 |
| h151N5 K8 | 267 | 268 | 74 | 75 | 76 |
| h151N5 K9 | 269 | 270 | 74 | 75 | 76 |

### h7C8 K1

The amino acid sequence of the light chain VK is as set forth in SEQ ID NO. 214, the nucleic acid encoding the light chain VK is as set forth in SEQ ID NO. 215, and the CDR1, CDR2 and CDR3 of the light chain VK are as set forth in SEQ ID NOs. 126, 65 and 127, respectively.

<-----------FR1-----------> CDR1 <------FR2------> CDR2
DVVMTQSPLSLPVTPGEPASISCRSSQSIVHSNGNTYLEWYLQKPGQSPKLLIYKVSNRFSG
<---------------FR3---------------> CDR3 <--FR4--->
VPDRFSGSGSGTDFTLKISRVEAEDVGVYYCFQGSHVPLTFGQGTKLEIK

### Nucleic acid sequence

GACGTGGTCATGACACAGAGCCCTCTGAGCCTGCCTGTGACACCTGGCGAACCTGCCAGCATC
AGCTGTAGAAGCAGCCAGAGCATCGTGCACAGCAACGGCAACACCTACCTGGAATGGTATCTG
CAGAAGCCCGGCCAGTCTCCTAAGCTGCTGATCTACAAGGTGTCCAACAGATTCAGCGGCGTG

CCCGACAGATTCTCTGGCTCTGGATCTGGCACCGACTTCACCCTGAAGATCTCCAGAGTGGAA
GCCGAGGACGTGGGCGTGTACTACTGCTTCCAAGGCTCTCACGTGCCCCTGACATTTGGCCAG
GGCACCAAGCTGGAAATCAAG

### h7C8 K2

The amino acid sequence of the light chain VK is as set forth in SEQ ID NO. 216, the nucleic acid encoding the light chain VK is as set forth in SEQ ID NO. 217, and the CDR1, CDR2 and CDR3 of the light chain VK are as set forth in SEQ ID NOs. 218, 65 and 127, respectively.

<-----------FR1-----------> CDR1 <------FR2------> CDR2
DIVMTQSPLSLPVTPGEPASISCRSSQSIVHSNGNTYLDWYLQKPGQSPQLLIYKVSNRFSG
<-----------------FR3-----------------> CDR3 <--FR4--->
VPDRFSGSGSGTDFTLKISRVEAEDVGVYYCFQGSHVPLTFGQGTKLEIK

### Nucleic acid sequence

GACATCGTCATGACACAGAGCCCTCTGAGCCTGCCTGTGACACCTGGCGAACCTGCCAGCATC
AGCTGTAGAAGCAGCCAGAGCATCGTGCACAGCAACGGCAACACCTACCTGGACTGGTATCTG
CAGAAGCCCGGCCAGTCTCCTCAGCTGCTGATCTACAAGGTGTCCAACAGATTCAGCGGCGTG
CCCGACAGATTCTCTGGCTCTGGATCTGGCACCGACTTCACCCTGAAGATCTCCAGAGTGGAA
GCCGAGGACGTGGGCGTGTACTACTGCTTCCAAGGCTCTCACGTGCCCCTGACATTTGGCCAG
GGCACCAAGCTGGAAATCAAG

### h200O1 K1

The amino acid sequence of the light chain VK is as set forth in SEQ ID NO. 219, the nucleic acid encoding the light chain VK is as set forth in SEQ ID NO. 220, and the CDR1, CDR2 and CDR3 of the light chain VK are as set forth in SEQ ID NOs. 28, 29 and 30, respectively.

<-----------FR1--------> CDR1 <-----FR2----> CDR2 <-----
DIQMTQSPSSLSASVGDRVTITCRASQDINIYLNWYQQKPGKVVKLLIYYTSRLHSGVPSRF
----------FR3-----------> CDR3 <--FR4--->
SGSGSGTDFTLTISSLQPEDVATYFCQQGHTLPYTFGQGTKLEIK

### Nucleic acid sequence

GACATCCAGATGACACAGAGCCCTAGCAGCCTGTCTGCCAGCGTGGGAGACAGAGTGACCATC
ACCTGTAGAGCCAGCCAGGACATCAACATCTACCTGAACTGGTATCAGCAGAAACCCGGCAAG
GTGGTCAAGCTGCTGATCTACTACACCAGCAGACTGCACAGCGGCGTGCCCTCTAGATTTTCT
GGCAGCGGCTCTGGCACCGACTTCACCCTGACAATCTCTAGCCTGCAGCCTGAGGACGTGGCA
ACCTACTTCTGCCAGCAAGGCCACACACTGCCCTACACATTTGGCCAGGGCACCAAGCTGGAA
ATCAAG

### h200O1 K2

The amino acid sequence of the light chain VK is as set forth in SEQ ID NO. 221, the nucleic acid encoding the light chain VK is as set forth in SEQ ID NO. 222, and the CDR1, CDR2 and CDR3 of the light chain VK are as set forth in SEQ ID NOs. 223, 29 and 30, respectively.

<-----------FR1--------->    CDR1    <-----FR2----->  CDR2 <-----
DIQMTQSPSSLSASVGDRVTITCRASQDISIYLNWYQQKPGKVPKLLIYYTSRLHSGVPSRF
-----------FR3-------------->  CDR3    <--FR4--->
SGSGSGTDFTLTISSLQPEDVATYFCQQGHTLPYTFGQGTKLEIK

### Nucleic acid sequence

GACATCCAGATGACACAGAGCCCTAGCAGCCTGTCTGCCAGCGTGGGAGACAGAGTGACCATC
ACCTGTAGAGCCAGCCAGGACATCAGCATCTACCTGAACTGGTATCAGCAGAAACCCGGCAAG
GTGCCCAAGCTGCTGATCTACTACACCAGCAGACTGCACAGCGGCGTGCCCTCTAGATTTTCT
GGCAGCGGCTCTGGCACCGACTTCACCCTGACAATCTCTAGCCTGCAGCCTGAGGACGTGGCA
ACCTACTTCTGCCAGCAAGGCCACACACTGCCCTACACATTTGGCCAGGGCACCAAGCTGGAA
ATCAAG

### h200O1 K3

The amino acid sequence of the light chain VK is as set forth in SEQ ID NO. 224, the nucleic acid encoding the light chain VK is as set forth in SEQ ID NO. 225, and the CDR1, CDR2 and CDR3 of the light chain VK are as set forth in SEQ ID NOs. 226, 29 and 30, respectively.

<----------FR1-------->    CDR1    <-----FR2----> CDR2  <-----
DIQMTQSPSSLSASVGDRVTITCRASQGISIYLNWYQQKPGKVPKLLIYYTSRLHSGVPSRF
----------FR3------------>    CDR3   <--FR4-->
SGSGSGTDFTLTISSLQPEDVATYFCQQGHTLPYTFGQGTKLEIK

### Nucleic acid sequence

GACATCCAGATGACACAGAGCCCTAGCAGCCTGTCTGCCAGCGTGGGAGACAGAGTGACCATC
ACCTGTAGAGCCAGCCAGGGCATCAGCATCTACCTGAACTGGTATCAGCAGAAACCCGGCAAG
GTGCCCAAGCTGCTGATCTACTACACCAGCAGACTGCACAGCGGCGTGCCCTCTAGATTTTCT
GGCAGCGGCTCTGGCACCGACTTCACCCTGACAATCTCTAGCCTGCAGCCTGAGGACGTGGCA
ACCTACTTCTGCCAGCAAGGCCACACACTGCCCTACACATTTGGCCAGGGCACCAAGCTGGAA
ATCAAG

### h200O1 K4

The amino acid sequence of the light chain VK is as set forth in SEQ ID NO. 227, the nucleic acid encoding the light chain VK is as set forth in SEQ ID NO. 228, and the CDR1, CDR2 and CDR3 of the light chain VK are as set forth in SEQ ID NOs. 226, 229 and 30, respectively.

<-----------FR1--------->    CDR1    <-----FR2----> CDR2  <-----
DIQMTQSPSSLSASVGDRVTITCRASQGISIYLNWYQQKPGKVPKLLIYYASRLHSGVPSRF
----------FR3-----------> CDR3  <--FR4--->
SGSGSGTDFTLTISSLQPEDVATYFCQQGHTLPYTFGQGTKLEIK

### Nucleic acid sequence

GACATCCAGATGACACAGAGCCCTAGCAGCCTGTCTGCCAGCGTGGGAGACAGAGTGACCATC
ACCTGTAGAGCCAGCCAGGGCATCAGCATCTACCTGAACTGGTATCAGCAGAAACCCGGCAAG

GTGCCCAAGCTGCTGATCTACTACGCCAGCAGACTGCACAGCGGCGTGCCCTCTAGATTTTCT
GGCAGCGGCTCTGGCACCGACTTCACCCTGACAATCTCTAGCCTGCAGCCTGAGGACGTGGCA
ACCTACTTCTGCCAGCAAGGCCACACACTGCCCTACACATTTGGCCAGGGCACCAAGCTGGAA
ATCAAG

### h6B5 K1

The amino acid sequence of the light chain VK is as set forth in SEQ ID NO. 230, the nucleic acid encoding the light chain VK is as set forth in SEQ ID NO. 231, and the CDR1, CDR2 and CDR3 of the light chain VK are as set forth in SEQ ID NOs. 232, 103 and 104, respectively.

<-----------FR1----------->    CDR1   <-----FR2----->  CDR2 <-----
EIVLTQSPATLSLSPGERATLSCRASSSVSYMHWFQQKPGQAPRLWIYSTSNLASGIPARFS
---------FR3--------------->    CDR3    <--FR4--->
GSGSGTDYTLTISRLEPEDFAVYYCQQRSSYPLTFGQGTKLEIK

### Nucleic acid sequence

GAGATCGTGCTGACACAGAGCCCTGCCACACTGTCACTGTCTCCAGGCGAGAGAGCCACACTG
AGCTGTAGAGCCAGCAGCAGCGTGTCCTACATGCACTGGTTCCAGCAGAAGCCTGGACAGGCC
CCTAGACTGTGGATCTACAGCACAAGCAACCTGGCCAGCGGCATCCCCGCTAGATTTTCTGGC
AGCGGCAGCGGAACCGACTACACCCTGACAATCAGCAGACTGGAACCCGAGGACTTCGCCGTG
TACTACTGCCAGCAGAGAAGCAGCTACCCTCTGACATTCGGCCAGGGCACCAAGCTGGAAATC
AAG

### h6B5 K2

The amino acid sequence of the light chain VK is as set forth in SEQ ID NO. 233, the nucleic acid encoding the light chain VK is as set forth in SEQ ID NO. 234, and the CDR1, CDR2 and CDR3 of the light chain VK are as set forth in SEQ ID NOs. 232, 235 and 104, respectively.

<-----------FR1----------> CDR1 <-----FR2-----> CDR2 <------
EIVLTQSPATLSLSPGERATLSCRASSSVSYMHWFQQKPGQAPRLLIYSTSNLATGIPARFS
--------FR3--------------> CDR3 <--FR4--->
GSGSGTDYTLTISRLEPEDFAVYYCQQRSSYPLTFGQGTKLEIK

### Nucleic acid sequence

GAGATCGTGCTGACACAGAGCCCTGCCACACTGTCACTGTCTCCAGGCGAGAGAGCCACACTG
AGCTGTAGAGCCAGCAGCAGCGTGTCCTACATGCACTGGTTCCAGCAGAAGCCTGGACAGGCC
CCTAGACTGCTGATCTACAGCACAAGCAACCTGGCCACAGGCATCCCCGCTAGATTTTCTGGC
AGCGGCAGCGGAACCGACTACACCCTGACAATCAGCAGACTGGAACCCGAGGACTTCGCCGTG
TACTACTGCCAGCAGAGAAGCAGCTACCCTCTGACATTCGGCCAGGGCACCAAGCTGGAAATC
AAG

### h6B5 K3

The amino acid sequence of the light chain VK is as set forth in SEQ ID NO. 236, the nucleic acid encoding the light chain VK is as set forth in SEQ ID NO. 237, and the CDR1, CDR2 and CDR3 of the light chain VK are as set forth in SEQ ID NOs. 232, 238 and 104, respectively.

<-----------FR1----------->    CDR1   <-----FR2----->  CDR2 <------
EIVLTQSPATLSLSPGERATLSCRASSSVSYMHWFQQKPGQAPRLLIYSTSSRATGIPARFS
---------FR3--------------->    CDR3    <--FR4--->
GSGSGTDYTLTISRLEPEDFAVYYCQQRSSYPLTFGQGTKLEIK

### Nucleic acid sequence

GAGATCGTGCTGACACAGAGCCCTGCCACACTGTCACTGTCTCCAGGCGAGAGAGCCACACTG
AGCTGTAGAGCCAGCAGCAGCGTGTCCTACATGCACTGGTTCCAGCAGAAGCCTGGACAGGCC
CCTAGACTGCTGATCTACAGCACAAGCAGCAGGGCCACAGGCATCCCCGCTAGATTTTCTGGC
AGCGGCAGCGGAACCGACTACACCCTGACAATCAGCAGACTGGAACCCGAGGACTTCGCCGTG
TACTACTGCCAGCAGAGAAGCAGCTACCCTCTGACATTCGGCCAGGGCACCAAGCTGGAAATC
AAG

### h6B5 K4

The amino acid sequence of the light chain VK is as set forth in SEQ ID NO. 239, the nucleic acid encoding the light chain VK is as set forth in SEQ ID NO. 240, and the CDR1, CDR2 and CDR3 of the light chain VK are as set forth in SEQ ID NOs. 232, 103 and 104, respectively.

<-----------FR1---------->    CDR1   <-----FR2----->   CDR2  <------
DIQLTQSPSSLSASVGDRVTITCRASSSVSYMHWFQQKPGKAPKLWIYSTSNLASGVPSRFS
--------FR3--------------->    CDR3    <--FR4--->
GSGSGTDYTLTISSLQPEDFATYYCQQRSSYPLTFGQGTKLEIK

### Nucleic acid sequence

GACATCCAGCTGACACAGAGCCCTTCTAGCCTGTCTGCCAGCGTGGGCGACAGAGTGACCATC
ACATGTAGAGCCAGCAGCAGCGTGTCCTACATGCACTGGTTCCAGCAGAAGCCTGGCAAGGCC
CCTAAGCTGTGGATCTACAGCACAAGCAACCTGGCCTCAGGCGTGCCCTCTAGATTTTCTGGC
AGCGGCTCTGGCACCGACTACACCCTGACAATCTCTAGCCTGCAGCCTGAGGACTTCGCCACC
TACTACTGCCAGCAGAGAAGCAGCTACCCTCTGACATTCGGCCAGGGCACCAAGCTGGAAATC
AAG

### h6B5 K5

The amino acid sequence of the light chain VK is as set forth in SEQ ID NO. 241, the nucleic acid encoding the light chain VK is as set forth in SEQ ID NO. 242, and the CDR1, CDR2 and CDR3 of the light chain VK are as set forth in SEQ ID NOs. 232, 103 and 104, respectively.

<-----------FR1----------->   CDR1   <------FR2------>   CDR2 <-------
DIQMTQSPSSLSASVGDRVTITCRASSSVSYMHWFQQKPGKAPKLLIYSTSNLASGVPSRFS
---------FR3--------------->   CDR3   <--FR4--->
GSGSGTDYTLTISSLQPEDFATYYCQQRSSYPLTFGQGTKLEIK

### Nucleic acid sequence

GACATCCAGATGACACAGAGCCCTTCTAGCCTGTCTGCCAGCGTGGGCGACAGAGTGACCATC
ACATGTAGAGCCAGCAGCAGCGTGTCCTACATGCACTGGTTCCAGCAGAAGCCTGGCAAGGCC

CCTAAGCTGCTGATCTACAGCACAAGCAACCTGGCCTCAGGCGTGCCCTCTAGATTTTCTGGC
AGCGGCTCTGGCACCGACTACACCCTGACAATCTCTAGCCTGCAGCCTGAGGACTTCGCCACC
TACTACTGCCAGCAGAGAAGCAGCTACCCTCTGACATTCGGCCAGGGCACCAAGCTGGAAATC
AAG

### h6B5 K6

The amino acid sequence of the light chain VK is as set forth in SEQ ID NO. 243, the nucleic acid encoding the light chain VK is as set forth in SEQ ID NO. 244, and the CDR1, CDR2 and CDR3 of the light chain VK are as set forth in SEQ ID NOs. 232, 245 and 104, respectively.

<-----------FR1---------->    CDR1   <-----FR2----->  CDR2 <------
DIQMTQSPSSLSASVGDRVTITCRASSSVSYMHWFQQKPGKAPKLLIYSTSSLQSGVPSRFS
---------FR3-------------->    CDR3    <--FR4--->
GSGSGTDYTLTISSLQPEDFATYYCQQRSSYPLTFGQGTKLEIK

### Nucleic acid sequence

GACATCCAGATGACACAGAGCCCTTCTAGCCTGTCTGCCAGCGTGGGCGACAGAGTGACCATC
ACATGTAGAGCCAGCAGCAGCGTGTCCTACATGCACTGGTTCCAGCAGAAGCCTGGCAAGGCC
CCTAAGCTGCTGATCTACAGCACAAGCAGCCTGCAGTCAGGCGTGCCCTCTAGATTTTCTGGC
AGCGGCTCTGGCACCGACTACACCCTGACAATCTCTAGCCTGCAGCCTGAGGACTTCGCCACC
TACTACTGCCAGCAGAGAAGCAGCTACCCTCTGACATTCGGCCAGGGCACCAAGCTGGAAATC
AAG

### h151N5 K1

The amino acid sequence of the light chain VK is as set forth in SEQ ID NO. 246, the nucleic acid encoding the light chain VK is as set forth in SEQ ID NO. 247, and the CDR1, CDR2 and CDR3 of the light chain VK are as set forth in SEQ ID NOs. 74, 248 and 76, respectively.

<---------FR1--------->    CDR1    <-----FR2----->  CDR2 <-----
DIQMTQSPSSLSASVGDRVTITCKASEDIYNRLAWYQQKPGKAPKLLISGATSLETGVPSRF
---------FR3-------------->    CDR3    <--FR4--->
SGSGSGKDYTLTISSLQPEDIATYYCQQYWRTPLTFGQGTKLEIK

### Nucleic acid sequence

GACATCCAGATGACACAGAGCCCTAGCAGCCTGTCTGCCAGCGTGGGAGACAGAGTGACCATC
ACCTGTAAGGCCAGCGAGGACATCTACAACAGACTGGCCTGGTATCAGCAGAAGCCCGGCAAG
GCTCCTAAGCTGCTGATCTCTGGCGCCACCTCTCTGGAAACAGGCGTGCCATCTAGATTCAGC
GGCAGCGGCTCTGGCAAGGACTACACCCTGACAATCAGCAGCCTGCAGCCTGAGGATATCGCC
ACCTACTACTGCCAGCAGTACTGGCGGACCCCTCTGACATTTGGCCAGGGCACCAAGCTGGAA
ATCAAG

### h151N5 K2

The amino acid sequence of the light chain VK is as set forth in SEQ ID NO. 249, the nucleic acid encoding the light chain VK is as set forth in SEQ ID NO. 250, and the CDR1, CDR2 and CDR3 of the light chain VK are as set forth in SEQ ID NOs. 74, 248 and 76, respectively.

<-----------FR1---------->    CDR1    <-----FR2----->  CDR2 <-----
DIQMTQSPSSLSASVGDRVTITCKASEDIYNRLAWYQQKPGKAPKLLISGATSLETGVPSRF
---------FR3--------------->    CDR3    <--FR4--->
SGSGSGKDYTLTISSLQPEDVATYYCQQYWRTPLTFGQGTKLEIK

### Nucleic acid sequence

GACATCCAGATGACACAGAGCCCTAGCAGCCTGTCTGCCAGCGTGGGAGACAGAGTGACCATC
ACCTGTAAGGCCAGCGAGGACATCTACAACAGACTGGCCTGGTATCAGCAGAAGCCCGGCAAG
GCTCCTAAGCTGCTGATCTCTGGCGCCACCTCTCTGGAAACAGGCGTGCCATCTAGATTCAGC
GGCAGCGGCTCTGGCAAGGACTACACCCTGACAATCAGCAGCCTGCAGCCTGAGGATGTCGCC
ACCTACTACTGCCAGCAGTACTGGCGGACCCCTCTGACATTTGGCCAGGGCACCAAGCTGGAA
ATCAAG

### h151N5 K3

The amino acid sequence of the light chain VK is as set forth in SEQ ID NO. 251, the nucleic acid encoding the light chain VK is as set forth in SEQ ID NO. 252, and the CDR1, CDR2 and CDR3 of the light chain VK are as set forth in SEQ ID NOs. 74, 253 and 76, respectively.

<-----------FR1----------->    CDR1    <-----FR2----->  CDR2 <-----
DIQMTQSPSSLSASVGDRVTITCKASEDIYNRLAWYQQKPGKAPKLLIYGATSLQSGVPSRF
-----------FR3--------------->    CDR3    <--FR4--->
SGSGSGKDYTLTISSLQPEDVATYYCQQYWRTPLTFGQGTKLEIK

### Nucleic acid sequence

GACATCCAGATGACACAGAGCCCTAGCAGCCTGTCTGCCAGCGTGGGAGACAGAGTGACCATC
ACCTGTAAGGCCAGCGAGGACATCTACAACAGACTGGCCTGGTATCAGCAGAAGCCCGGCAAG
GCTCCTAAGCTGCTGATCTATGGCGCCACCTCTCTGCAATCAGGCGTGCCATCTAGATTCAGC
GGCAGCGGCTCTGGCAAGGACTACACCCTGACAATCAGCAGCCTGCAGCCTGAGGATGTCGCC
ACCTACTACTGCCAGCAGTACTGGCGGACCCCTCTGACATTTGGCCAGGGCACCAAGCTGGAA
ATCAAG

### h151N5 K4

The amino acid sequence of the light chain VK is as set forth in SEQ ID NO. 254, the nucleic acid encoding the light chain VK is as set forth in SEQ ID NO. 255, and the CDR1, CDR2 and CDR3 of the light chain VK are as set forth in SEQ ID NOs. 256, 257 and 76, respectively.

<-----------FR1---------->    CDR1    <-----FR2----->  CDR2 <-----
DIQMTQSPSSLSASVGDRVTITCKASQDIYNRLAWYQQKPGKVPKLLIYAATSLQSGVPSRF
---------FR3--------------->    CDR3    <--FR4--->
SGSGSGKDYTLTISSLQPEDVATYYCQQYWRTPLTFGQGTKLEIK

### Nucleic acid sequence

GACATCCAGATGACACAGAGCCCTAGCAGCCTGTCTGCCAGCGTGGGAGACAGAGTGACCATC
ACCTGTAAGGCCAGCCAGGACATCTACAACAGACTGGCCTGGTATCAGCAGAAGCCCGGCAAG

GTTCCTAAGCTGCTGATCTATGCCGCCACCTCTCTGCAATCAGGCGTGCCATCTAGATTCAGC
GGCAGCGGCTCTGGCAAGGACTACACCCTGACAATCAGCAGCCTGCAGCCTGAGGATGTCGCC
ACCTACTACTGCCAGCAGTACTGGCGGACCCCTCTGACATTTGGCCAGGGCACCAAGCTGGAA
ATCAAG

### h151N5 K5

The amino acid sequence of the light chain VK is as set forth in SEQ ID NO. 258, the nucleic acid encoding the light chain VK is as set forth in SEQ ID NO. 259, and the CDR1, CDR2 and CDR3 of the light chain VK are as set forth in SEQ ID NOs. 260, 261 and 76, respectively.

<-----------FR1---------->    CDR1    <-----FR2----->  CDR2 <-----
DIQMTQSPSSLSASVGDRVTITCKASQGIYNRLAWYQQKPGKVPKLLIYAASTLQSGVPSRF
----------FR3--------------->    CDR3    <--FR4--->
SGSGSGKDYTLTISSLQPEDVATYYCQQYWRTPLTFGQGTKLEIK

### Nucleic acid sequence

GACATCCAGATGACACAGAGCCCTAGCAGCCTGTCTGCCAGCGTGGGAGACAGAGTGACCATC
ACCTGTAAGGCCAGCCAGGGCATCTACAACAGACTGGCCTGGTATCAGCAGAAGCCCGGCAAG
GTTCCTAAGCTGCTGATCTATGCCGCCTCCACTCTGCAATCAGGCGTGCCATCTAGATTCAGC
GGCAGCGGCTCTGGCAAGGACTACACCCTGACAATCAGCAGCCTGCAGCCTGAGGATGTCGCC
ACCTACTACTGCCAGCAGTACTGGCGGACCCCTCTGACATTTGGCCAGGGCACCAAGCTGGAA
ATCAAG

### h151N5 K6

The amino acid sequence of the light chain VK is as set forth in SEQ ID NO. 262, the nucleic acid encoding the light chain VK is as set forth in SEQ ID NO. 263, and the CDR1, CDR2 and CDR3 of the light chain VK are as set forth in SEQ ID NOs. 260, 261 and 264, respectively.

<-----------FR1----------->    CDR1    <------FR2------>  CDR2  <------
DIQMTQSPSSLSASVGDRVTITCKASQGIYNRLAWYQQKPGKVPKLLIYAASTLQSGVPSRF
-----------FR3--------------->    CDR3    <---FR4--->
SGSGSGKDYTLTISSLQPEDVATYYCQQYYRTPLTFGQGTKLEIK

### Nucleic acid sequence

GACATCCAGATGACACAGAGCCCTAGCAGCCTGTCTGCCAGCGTGGGAGACAGAGTGACCATC
ACCTGTAAGGCCAGCCAGGGCATCTACAACAGACTGGCCTGGTATCAGCAGAAGCCCGGCAAG
GTTCCTAAGCTGCTGATCTATGCCGCCTCCACTCTGCAATCAGGCGTGCCATCTAGATTCAGC
GGCAGCGGCTCTGGCAAGGACTACACCCTGACAATCAGCAGCCTGCAGCCTGAGGATGTCGCC
ACCTACTACTGCCAGCAGTACTACCGGACCCCTCTGACATTTGGCCAGGGCACCAAGCTGGAA
ATCAAG

### h151N5 K7

The amino acid sequence of the light chain VK is as set forth in SEQ ID NO. 265, the nucleic acid encoding the light chain VK is as set forth in SEQ ID NO. 266, and the CDR1, CDR2 and CDR3 of the light chain VK are as set forth in SEQ ID NOs. 74, 75 and 76, respectively.

<-----------FR1----------->    CDR1    <------FR2------>  CDR2 <-----
DIQMTQSPSSLSASVGDRVTITCKASEDIYNRLAWYQQKPGKAPKLLISGAIRLETGVPSRF
-----------FR3--------------->    CDR3    <--FR4--->
SGSGSGKDYTLTISSLQPEDIATYYCQQYWRTPLTFGQGTKLEIK

### Nucleic acid sequence

GACATCCAGATGACACAGAGCCCTAGCAGCCTGTCTGCCAGCGTGGGAGACAGAGTGACCATC
ACCTGTAAGGCCAGCGAGGACATCTACAACAGACTGGCCTGGTATCAGCAGAAGCCCGGCAAG
GCTCCTAAGCTGCTGATCTCTGGCGCCATCAGACTGGAAACAGGCGTGCCATCTAGATTCAGC
GGCAGCGGCTCTGGCAAGGACTACACCCTGACAATCAGCAGCCTGCAGCCTGAGGATATCGCC
ACCTACTACTGCCAGCAGTACTGGCGGACCCCTCTGACATTTGGCCAGGGCACCAAGCTGGAA
ATCAAG

### h151N5 K8

The amino acid sequence of the light chain VK is as set forth in SEQ ID NO. 267, the nucleic acid encoding the light chain VK is as set forth in SEQ ID NO. 268, and the CDR1, CDR2 and CDR3 of the light chain VK are as set forth in SEQ ID NOs. 74, 75 and 76, respectively.

<-----------FR1----------->   CDR1   <------FR2------>   CDR2 <-----
DIQMTQSPSSLSASVGDRVTITCKASEDIYNRLAWYQQKPGKAPKLLISGAIRLETGVPSRF
-----------FR3----------------->   CDR3   <---FR4--->
SGSGSGKDYTLTISSLQPEDVATYYCQQYWRTPLTFGQGTKLEIK

### Nucleic acid sequence

GACATCCAGATGACACAGAGCCCTAGCAGCCTGTCTGCCAGCGTGGGAGACAGAGTGACCATC
ACCTGTAAGGCCAGCGAGGACATCTACAACAGACTGGCCTGGTATCAGCAGAAGCCCGGCAAG
GCTCCTAAGCTGCTGATCTCTGGCGCCATCAGACTGGAAACAGGCGTGCCATCTAGATTCAGC
GGCAGCGGCTCTGGCAAGGACTACACCCTGACAATCAGCAGCCTGCAGCCTGAGGATGTCGCC
ACCTACTACTGCCAGCAGTACTGGCGGACCCCTCTGACATTTGGCCAGGGCACCAAGCTGGAA
ATCAAG

### h151N5 K9

The amino acid sequence of the light chain VK is as set forth in SEQ ID NO. 269, the nucleic acid encoding the light chain VK is as set forth in SEQ ID NO. 270, and the CDR1, CDR2 and CDR3 of the light chain VK are as set forth in SEQ ID NOs. 74, 75 and 76, respectively.

<-----------FR1----------->     CDR1     <------FR2------> CDR2   <-----
DIQMTQSPSSLSASVGDRVTITCKASEDIYNRLAWYQQKPGKAPKLLIYGAIRLETGVPSRF
----------FR3-------------->     CDR3    <--FR4--->
SGSGSGKDYTLTISSLQPEDVATYYCQQYWRTPLTFGQGTKLEIK

### Nucleic acid sequence

GACATCCAGATGACACAGAGCCCTAGCAGCCTGTCTGCCAGCGTGGGAGACAGAGTGACCATC
ACCTGTAAGGCCAGCGAGGACATCTACAACAGACTGGCCTGGTATCAGCAGAAGCCCGGCAAG

GCTCCTAAGCTGCTGATCTATGGCGCCATCAGACTGGAAACAGGCGTGCCATCTAGATTCAGC
GGCAGCGGCTCTGGCAAGGACTACACCCTGACAATCAGCAGCCTGCAGCCTGAGGATGTCGCC
ACCTACTACTGCCAGCAGTACTGGCGGACCCCTCTGACATTTGGCCAGGGCACCAAGCTGGAA
ATCAAG

### 2) Construction of humanized antibodies

The variable region sequence of the light chain was synthesised and cloned into a eukaryotic expression plasmid containing the antibody kappa chain constant region Ckappa. Meanwhile, the variable region sequence of the heavy chain was synthesised and cloned into a eukaryotic expression plasmid containing the human IgG1 constant regions CH1-CH3. After the above light and heavy chain plasmids were combined and paired, the plasmids were transfected into CHO-S cells, followed by expression at 37°C for 5-6 days. The culture supernatant was collected and purified using a Protein A column to obtain humanized bivalent antibodies.

### Example 6. Detection of affinity of humanized anti-Aβ antibody

Using surface plasmon resonance (SPR/Biacore T200), Aβ1-42 protofibril was immobilized on the surface of a CM5 chip via amino coupling, and then the serially diluted 6B5/7C8 humanized antibodies flowed over the chip as analytes to detect the real-time binding signals between the antibodies and the antigen. The affinity of the 6B5/7C8 humanized antibodies is shown in FIG. 3 and Table 5. Using surface plasmon resonance (SPR/Biacore T200), pyroE Aβ3-16-biotin and Aβ3-16-biotin antigens were coupled to different channels of an SA chip, respectively, and the 200O1/151N5 humanized antibodies were serially diluted with HBS-EP+buffer (Cytiva, BR10069) and then flowed over the chip surface as analytes to detect the real-time binding signals between the antibodies and the antigens. The affinity of the antibodies is shown in FIG. 4 and Table 6.

**Table 5. Affinity of 7C8 and 6B5 humanized antibodies**

| Humanized antibody | ka (1/Ms) | kd (1/s) | KD (M) |
|---|---|---|---|
| 6B5 | 1.15E+06 | 1.96E-03 | 1.71E-09 |
| 6B5 H1K1 hIgG1 | 1.49E+06 | 2.55E-03 | 1.70E-09 |
| 6B5 H1K2 hIgG1 | 1.71E+06 | 3.61E-03 | 2.11E-09 |
| 6B5 H1K3 hIgG1 | 2.85E+06 | 4.04E-03 | 1.42E-09 |
| 6B5 H1K4 hIgG1 | 1.43E+06 | 2.29E-03 | 1.60E-09 |
| 6B5 H1K5 hIgG1 | 1.90E+06 | 3.54E-03 | 1.87E-09 |
| 6B5 H1K6 hIgG1 | 2.44E+06 | 4.01E-03 | 1.64E-09 |
| 7C8 | 1.37E+06 | 4.34E-03 | 3.16E-09 |
| 7C8 H2K2 hIgG1 | 1.06E+06 | 2.93E-03 | 2.76E-09 |
| 7C8 H4K2 hIgG1 | 1.20E+06 | 3.70E-03 | 3.08E-09 |
| 7C8 H5K2 hIgG1 | 1.34E+06 | 3.95E-03 | 2.95E-09 |
| 7C8 H6K2 hIgG1 | 1.46E+06 | 4.10E-03 | 2.80E-09 |
| 7C8 H7K2 hIgG1 | 1.78E+06 | 4.15E-03 | 2.33E-09 |
| 7C8 H6K1 hIgG1 | 1.79E+06 | 4.72E-03 | 2.63E-09 |
| 7C8 H2K1 hIgG1 | 1.02E+06 | 2.80E-03 | 2.74E-09 |
| 7C8 H7K1 hIgG1 | 2.42E+06 | 4.66E-03 | 1.93E-09 |

**Table 6. Affinity of 200O1 and 151N5 humanized antibodies**

| Humanized antibody | ka (1/Ms) | kd (1/s) | KD (M) |
|---|---|---|---|
| 200O1 | 7.60E+05 | 5.44E-08 | 7.16E-14 |
| 200O1 H1K1 | 6.82E+05 | 7.49E-10 | 1.10E-15 |
| 200O1 H2K1 | 3.81E+05 | 2.38E-07 | 6.25E-13 |
| 200O1 H5K1 | 7.76E+05 | 5.70E-08 | 7.36E-14 |
| 200O1 H6K1 | 7.47E+05 | 4.55E-07 | 6.10E-13 |
| 151N5 | 1.10E+06 | 3.19E-04 | 2.91E-10 |
| 151N5 H1K1 | 6.22E+05 | 2.00E-04 | 3.21E-10 |
| 151N5 H1K2 | 6.48E+05 | 2.07E-04 | 3.19E-10 |

### Example 7. Phagocytic activity of humanized antibody

The humanized antibody samples with good phagocytic activity after preliminary screening were each diluted with medium to 100 µg/mL and 10 µg/mL for later use. Pre-cut 10 µm FAD mouse brain sections were taken, and the sample application areas were circled using a histology pen. Then, 400 µL of each prepared test sample was gently added onto the sections, followed by incubation at 37°C for 1 h. During this period, mouse microglia BV2 were prepared and resuspended in a medium to 4 × 10⁵ cells/mL. After antibody incubation, the original samples on the sections were discarded, and 500 µL of cell suspension was added. After 24 h of culture, the medium was replaced once, and then the culture was continued for another 24 h, with a total culture duration of 48 h.

After phagocytosis, the sections were taken, and the supernatant from the sections was collected and centrifuged. The brain tissue on the sections was collected using 75 µL of guanidine hydrochloride lysis buffer. This collection was performed twice to obtain a total of 150 µL of lysate sample. After thorough mixing, the sample was kept on ice for further lysis for 1-2 h, during which uniform mixing could be performed multiple times. After the lysis was completed, the sample could be stored at -80°C or immediately detected by ELISA quantitation.

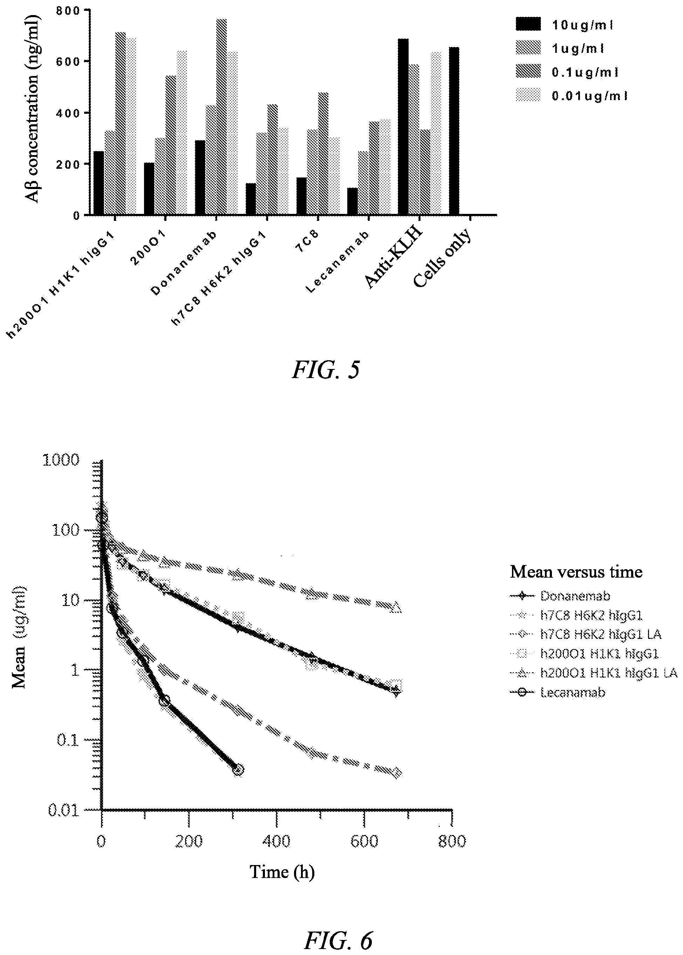

Aβ1-42 in the sample was quantified using the Human Aβ42 ELISA Kit (Cat: KHB3441, Invitrogen). The sample was centrifuged at 3000-4000 rpm, and 20 µL of the supernatant was taken and diluted 1000-fold. The detection was performed according to the kit instructions. Finally, the concentration of Aβ1-42 in the sample was calculated based on the standard curve. The phagocytosis results of the humanized samples are shown in FIG. 5. Both 200O1 H1K1 and 7C8 H6K2 promoted the phagocytosis of Aβ by microglia BV2.

### Example 8. Construction of long-acting humanized anti-Aβ antibody

Patients with Alzheimer's disease require long-term medication. Therefore, the development of long-acting anti-Aβ antibodies, which aim to enhance the convenience of antibodies and reduce antibody dosage, has broad prospects. In the Phase I clinical trial of Lecanemab, the antibody concentration in the 1 mg/kg group rapidly fell below the detection limit, making it impossible to calculate the half-life. The half-life in the 10 mg/kg group was 6.9 days. In the Phase I/II clinical trial of Donanemab, the half-life was 3.19 days in the 1 mg/kg intravenous administration group and 10.5 days in the 10 mg/kg group. The half-lives of both antibodies are shorter than those of conventional monoclonal antibody drugs. Therefore, monoclonal antibody drugs with longer half-lives may achieve better efficacy. On the basis of 7C8 H6K2 and 200O1 H1K1, sequence optimization was performed to increase the half-life of the antibodies. The sequences of the constructed long-acting antibodies are shown in Table 7 below.

**Table 7. Sequences of long-acting humanized anti-Aβ antibodies**

| Humanized anti-Aβ antibody | Heavy chain amino acid sequence | Heavy chain nucleotide sequence | Light chain amino acid sequence | Light chain nucleotide sequence |
|---|---|---|---|---|
| h7C8 H6K2 hIgG1 LA | 271 | 272 | 273 | 274 |
| h200O1 H1K1 hIgG1 LA | 275 | 276 | 277 | 278 |

For h7C8 H6K2 hIgG1 LA, the amino acid sequence of the heavy chain is as set forth in SEQ ID NO. 271, and the nucleic acid encoding the heavy chain is as set forth in SEQ ID NO. 272.

QVQLQESGPGLVKPSETLSLTCTFSGFSISTSGMGVSWIRQPPGKGLEWIGHIYWDDDKRYNP
SLKSRVTISKDTSKNQVSLKLSSVTAADTAVYYCARYGKGFAYWGQGTLVTVSSASTKGPSVF
PLAPSSKSTSGGTAALGCLVKDYFPEPVTVSWNSGALTSGVHTFPAVLQSSGLYSLSSVVTVP
SSSLGTQTYICNVNHKPSNTKVDKRVEPKSCDKTHTCPPCPAPELLGGPSVFLFPPKPKDTLM
ISRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVLHEALHAHYTQKSL
SLSPGK

### Nucleic acid sequence

CAGGTTCAGCTGCAAGAGTCTGGACCTGGCCTGGTCAAGCCTAGCGAGACACTGAGCCTGACC
TGTACCTTCAGCGGCTTCAGCATCAGCACATCTGGCATGGGAGTGTCCTGGATCAGACAGCCT
CCTGGCAAAGGCCTGGAATGGATCGGCCACATCTACTGGGACGACGACAAGAGATACAACCCC
AGCCTGAAGTCCAGAGTGACCATCAGCAAGGACACCAGCAAAAACCAGGTGTCCCTGAAGCTG
AGCAGCGTGACAGCCGCTGATACCGCCGTGTACTACTGTGCCAGATACGGCAAGGGCTTCGCC
TACTGGGGACAGGGAACACTGGTCACCGTTAGCTCTGCTAGCACCAAGGGCCCATCCGTCTTC
CCCCTGGCACCCTCCTCCAAGAGCACCTCTGGGGGCACAGCGGCCCTGGGCTGCCTGGTCAAG
GACTACTTCCCCGAACCGGTGACGGTGTCGTGGAACTCAGGCGCCCTGACCAGCGGCGTGCAC
ACCTTCCCGGCTGTCCTACAGTCCTCAGGACTCTACTCCCTCAGCAGCGTGGTGACCGTGCCC
TCCAGCAGCTTGGGCACCCAGACCTACATCTGCAACGTGAATCACAAGCCCAGCAACACCAAG
GTGGACAAGAGAGTTGAGCCCAAATCTTGTGACAAAACTCACACATGCCCACCGTGCCCAGCA
CCTGAACTCCTGGGGGGACCGTCAGTCTTCCTCTTCCCCCCAAAACCCAAGGACACCCTCATG
ATCTCCCGGACCCCTGAGGTCACATGCGTGGTGGTGGACGTGAGCCACGAAGACCCTGAGGTC
AAGTTCAACTGGTACGTGGACGGCGTGGAGGTGCATAATGCCAAGACAAAGCCGCGGGAGGAG
CAGTACAACAGCACGTACCGTGTGGTCAGCGTCCTCACCGTCCTGCACCAGGACTGGCTGAAT
GGCAAGGAGTACAAGTGCAAGGTCTCCAACAAAGCCCTCCCAGCCCCCATCGAGAAAACCATC
TCCAAAGCCAAAGGGCAGCCCCGAGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGCC
GTGGAGTGGGAGAGCAATGGGCAGCCGGAGAACAACTACAAGACCACGCCTCCCGTGCTGGAC
TCCGACGGCTCCTTCTTCCTCTATAGCAAGCTCACCGTGGACAAGAGCAGGTGGCAGCAGGGG
AACGTCTTCTCATGCTCCGTGCTGCATGAGGCTCTGCACGCCCACTACACGCAGAAGTCCCTC
TCCCTGTCTCCGGGTAAA

For h7C8 H6K2 hIgG1 LA, the amino acid sequence of the light chain is as set forth in SEQ ID NO. 273, and the nucleic acid encoding the light chain is as set forth in SEQ ID NO. 274.

DIVMTQSPLSLPVTPGEPASISCRSSQSIVHSNGNTYLDWYLQKPGQSPQLLIYK
VSNRFSGVPDRFSGSGSGTDFTLKISRVEAEDVGVYYCFQGSHVPLTFGQGTKLE
IKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC

### Nucleic acid sequence

GACATCGTCATGACACAGAGCCCTCTGAGCCTGCCTGTGACACCTGGCGAACCTGCCAGCATC
AGCTGTAGAAGCAGCCAGAGCATCGTGCACAGCAACGGCAACACCTACCTGGACTGGTATCTG
CAGAAGCCCGGCCAGTCTCCTCAGCTGCTGATCTACAAGGTGTCCAACAGATTCAGCGGCGTG
CCCGACAGATTCTCTGGCTCTGGATCTGGCACCGACTTCACCCTGAAGATCTCCAGAGTGGAA
GCCGAGGACGTGGGCGTGTACTACTGCTTCCAAGGCTCTCACGTGCCCCTGACATTTGGCCAG
GGCACCAAGCTGGAAATCAAGCGAACTGTGGCTGCACCATCTGTCTTCATCTTCCCGCCATCT
GATGAGCAGTTGAAATCTGGAACTGCCTCTGTTGTGTGCCTGCTGAATAACTTCTATCCCAGA
GAGGCCAAAGTACAGTGGAAGGTGGATAACGCCCTCCAATCGGGTAACTCCCAGGAGAGTGTC
ACAGAGCAGGACAGCAAGGACAGCACCTACAGCCTCAGCAGCACCCTGACGCTGAGCAAAGCA
GACTACGAGAAACACAAAGTCTACGCCTGCGAAGTCACCCATCAGGGCCTGAGCTCGCCCGTC
ACAAAGAGCTTCAACAGGGGAGAGTGT

For h200O1 H1K1 hIgG1 LA, the amino acid sequence of the heavy chain is as set forth in SEQ ID NO. 275, and the nucleic acid encoding the heavy chain is as set forth in SEQ ID NO. 276.

QVTLKESGPVLVKPTETLTLTCTVSGFSLSRYIVHWYRQPPGKALEWLAMIWGGGRIDYNSAL
KSRLTISKDNSKSQVVLTMTNMDPVDTATYYCARNFGENAEAYWGQGTLVTVSSASTKGPSVF
PLAPSSKSTSGGTAALGCLVKDYFPEPVTVSWNSGALTSGVHTFPAVLQSSGLYSLSSVVTVP
SSSLGTQTYICNVNHKPSNTKVDKRVEPKSCDKTHTCPPCPAPELLGGPSVFLFPPKPKDTLM
ISRTPEVTCVVVDVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVLHEALHAHYTQKSL
SLSPGK

### Nucleic acid sequence

CAAGTGACCCTGAAAGAAAGCGGCCCTGTGCTGGTCAAGCCCACCGAAACACTGACCCTGACC
TGTACCGTGTCCGGCTTCAGCCTGTCTCGGTACATCGTGCACTGGTACAGACAGCCTCCAGGC
AAGGCTCTGGAATGGCTGGCTATGATTTGGGGCGGAGGCAGAATCGACTACAACAGCGCCCTG
AAGTCCAGACTGACCATCAGCAAGGACAACAGCAAGAGCCAGGTGGTGCTGACCATGACCAAC
ATGGACCCTGTGGACACCGCCACCTACTACTGCGCTAGAAACTTCGGCGAGAACGCCGAGGCT
TACTGGGGACAGGGAACACTGGTCACCGTGTCTAGTGCTAGCACCAAGGGCCCATCCGTCTTC
CCCCTGGCACCCTCCTCCAAGAGCACCTCTGGGGGCACAGCGGCCCTGGGCTGCCTGGTCAAG
GACTACTTCCCCGAACCGGTGACGGTGTCGTGGAACTCAGGCGCCCTGACCAGCGGCGTGCAC
ACCTTCCCGGCTGTCCTACAGTCCTCAGGACTCTACTCCCTCAGCAGCGTGGTGACCGTGCCC
TCCAGCAGCTTGGGCACCCAGACCTACATCTGCAACGTGAATCACAAGCCCAGCAACACCAAG

GTGGACAAGAGAGTTGAGCCCAAATCTTGTGACAAAACTCACACATGCCCACCGTGCCCAGCA
CCTGAACTCCTGGGGGGACCGTCAGTCTTCCTCTTCCCCCCAAAACCCAAGGACACCCTCATG
ATCTCCCGGACCCCTGAGGTCACATGCGTGGTGGTGGACGTGAGCCACGAAGACCCTGAGGTC
AAGTTCAACTGGTACGTGGACGGCGTGGAGGTGCATAATGCCAAGACAAAGCCGCGGGAGGAG
CAGTACAACAGCACGTACCGTGTGGTCAGCGTCCTCACCGTCCTGCACCAGGACTGGCTGAAT
GGCAAGGAGTACAAGTGCAAGGTCTCCAACAAAGCCCTCCCAGCCCCCATCGAGAAAACCATC
TCCAAAGCCAAAGGGCAGCCCCGAGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGCC
GTGGAGTGGGAGAGCAATGGGCAGCCGGAGAACAACTACAAGACCACGCCTCCCGTGCTGGAC
TCCGACGGCTCCTTCTTCCTCTATAGCAAGCTCACCGTGGACAAGAGCAGGTGGCAGCAGGGG
AACGTCTTCTCATGCTCCGTGCTGCATGAGGCTCTGCACGCCCACTACACGCAGAAGTCCCTC
TCCCTGTCTCCGGGTAAA

For h200O1 H1K1 hIgG1 LA, the amino acid sequence of the light chain is as set forth in SEQ ID NO. 277, and the nucleic acid encoding the light chain is as set forth in SEQ ID NO. 278.

DIQMTQSPSSLSASVGDRVTITCRASQDINIYLNWYQQKPGKVVKLLIYYTSRLHSGVPSRFS
GSGSGTDFTLTISSLQPEDVATYFCQQGHTLPYTFGQGTKLEIKRTVAAPSVFIFPPSDEQLK
SGTASVVCLLNNFYPREAKVQWKVDNALQSGNSQESVTEQDSKDSTYSLSSTLTLSKADYEKH
KVYACEVTHQGLSSPVTKSFNRGEC

### Nucleic acid sequence

GACATCCAGATGACACAGAGCCCTAGCAGCCTGTCTGCCAGCGTGGGAGACAGAGTGACCATC
ACCTGTAGAGCCAGCCAGGACATCAACATCTACCTGAACTGGTATCAGCAGAAACCCGGCAAG
GTGGTCAAGCTGCTGATCTACTACACCAGCAGACTGCACAGCGGCGTGCCCTCTAGATTTTCT
GGCAGCGGCTCTGGCACCGACTTCACCCTGACAATCTCTAGCCTGCAGCCTGAGGACGTGGCA
ACCTACTTCTGCCAGCAAGGCCACACACTGCCCTACACATTTGGCCAGGGCACCAAGCTGGAA
ATCAAGCGAACTGTGGCTGCACCATCTGTCTTCATCTTCCCGCCATCTGATGAGCAGTTGAAA
TCTGGAACTGCCTCTGTTGTGTGCCTGCTGAATAACTTCTATCCCAGAGAGGCCAAAGTACAG
TGGAAGGTGGATAACGCCCTCCAATCGGGTAACTCCCAGGAGAGTGTCACAGAGCAGGACAGC
AAGGACAGCACCTACAGCCTCAGCAGCACCCTGACGCTGAGCAAAGCAGACTACGAGAAACAC
AAAGTCTACGCCTGCGAAGTCACCCATCAGGGCCTGAGCTCGCCCGTCACAAAGAGCTTCAAC
AGGGGAGAGTGTTAG

### Example 9. Detection of half-life of long-acting humanized anti-Aβ antibody

A total of 30 female B-FcRn mice aged 6 to 8 weeks and weighing 18-20 g (Biocytogen Jiangsu Co., Ltd.) were selected. The mice were randomly divided into six groups, with five mice in each group. A single dose of 10 mg/kg was administered via tail vein injection, with an injection volume of 100 µL/mouse. The administration and grouping details are shown in Table 8. Blood samples were collected from the submandibular vein of mice at 0 h, 1 h, 4 h, D1, D3, D5, D7, D14, D21 and D28 after administration. About seven to eight drops of fresh blood were collected from each mouse, which was then left to stand at 37°C for 30 min, followed by centrifugation at 4500 rpm for 15 min to collect the serum. The concentrations of antibodies in serum were detected by ELISA. The administration concentration was calculated on the basis of an average body weight of 19 g for the B-FcRn mice. The specific administration groups and administration doses are shown in Table 8 below.

A curve can be plotted after subtracting the blank well values from the standard and sample values. If replicate wells are set, the average value should be used for calculation. A curve was plotted with the concentration of drug in serum as the ordinate and the blood collection time as the abscissa. If the sample was diluted before detection, the calculated concentration must be multiplied by the dilution ratio in the final calculation to obtain the final actual concentration of the sample.

As shown in FIG. 6, the half-life of the h7C8 H6K2 antibody molecule was substantially comparable to that of Lecanemab, but shorter than that of h7C8 H6K2 LA, while the half-life of the h200O1 H1K1 antibody molecule was substantially comparable to that of Donanemab, but shorter than that of h200O1 H1K1 LA.

**Table 8. Administration groups and administration doses**

| Name | Concentration mg/mL | Stock solution volume/µL | Diluent volume/µL | Final concentration mg/mL |
|---|---|---|---|---|
| h7C8 H6K2 hIgG1 | 12.72 | 97.1 | 552.9 | 1.9 |
| h7C8 H6K2 hIgG1 LA | 12.99 | 95.1 | 554.9 | 1.9 |
| h200O1 H1K1 hIgG1 | 10.48 | 118.0 | 532 | 1.9 |
| h200O1 H1K1 hIgG1 LA | 9.91 | 125 | 525 | 1.9 |
| Donanemab | 7.11 | 174 | 476 | 1.9 |
| Lecanemab | 5.90 | 209 | 441 | 1.9 |

### Example 10. Detection of phagocytic activity of long-acting humanized anti-Aβ antibody

The long-acting antibody samples were each diluted with medium to 100 µg/mL and 10 µg/mL for later use. Pre-cut 10 µm FAD mouse brain sections were taken, and the sample application areas were circled using a histology pen. Then, 400 µL of each prepared test sample was gently added onto the sections, followed by incubation at 37°C for 1 h. During this period, BV2 cells (mouse microglia) were prepared and resuspended in a medium to 4 × 10⁵ cells/mL. After antibody incubation, the original samples on the sections were discarded, and 500 µL of cell suspension was added. After 24 h of culture, the medium was replaced once, and then the culture was continued for another 24 h, with a total culture duration of 48 h.

After phagocytosis, the sections were taken, and the supernatant from the sections was collected and centrifuged. The brain tissue on the sections was collected using 75 µL of guanidine hydrochloride lysis buffer. This collection was performed twice to obtain a total of 150 µL of lysate sample. After thorough mixing, the sample was kept on ice for further lysis for 1-2 h, during which uniform mixing could be performed multiple times. After the lysis was completed, the sample could be stored at -80°C or immediately detected by ELISA quantitation.

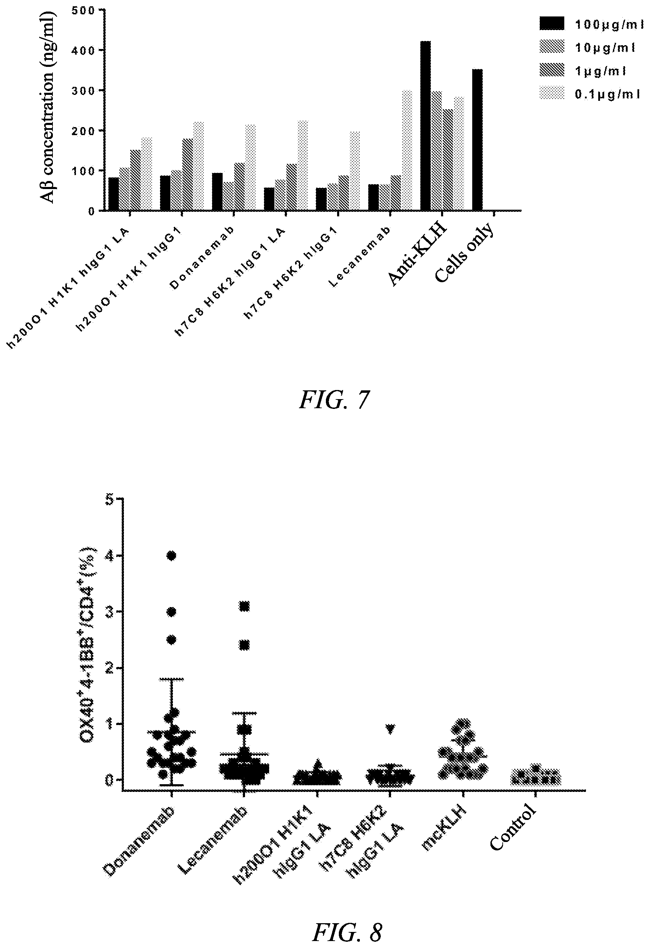

Aβ1-42 in the sample was quantified using the Human Aβ42 ELISA Kit (Cat: KHB3441, Invitrogen). The sample was centrifuged at 3000-4000 rpm, and 20 µL of the supernatant was taken and diluted 1000-fold. The detection was performed according to the kit instructions. Finally, the concentration of Aβ1-42 in the sample was calculated based on the standard curve. The phagocytosis results of the long-acting antibodies are shown in FIG. 7. Both 200O1 H1K1 LA and 7C8 H6K2 LA effectively cleared Aβ in brain sections.

### Example 11. Detection of immunogenicity of long-acting antibody

In this study, peripheral venous blood was collected from multiple healthy volunteers. PBMCs were isolated, and resuspended in X-vivo15 medium (Lonza, 04-418Q) at 2.5 × 10⁵ cells/well. The PBMCs were incubated with 100 µg/mL CM383 monoclonal antibody for 48 hours. The percentage of CD4⁺ T cells double-positive for CD134⁺ (OX40⁺) and CD137⁺ (4-1BB⁺) was detected by flow cytometry. Keyhole limpet hemocyanin (KLH) was used as a positive control, and the determination was performed using the same method. The results showed (see FIG. 8 and Table 9) that the immunogenicity of both 200O1 H1K1 LA and 7C8 H6K2 LA was lower than that of Donanemab and Lecanemab.

**Table 9. Percentage of CD4+ T cells double-positive for CD134+ (OX40+) and CD137+ (4-1BB+)**

| Name | Percentage of double-positive cells (%) |
|---|---|
| h7C8 H6K2 hIgG1 LA | 0.08 |
| h200O1 H1K1 hIgG1 LA | 0.06 |
| Donanemab | 0.85 |
| Lecanemab | 0.47 |
| mcKLH | 0.42 |
| Control | 0.06 |

### Example 12. Analysis of binding activity of humanized antibody and long-acting antibody to negative screening cells

A HEK293-APP stable transfected cell line expressing amyloid precursor protein (APP) was used as target cells. The cell density was adjusted to 1 × 10⁷ cells per 1 mL using a prepared BSA blocking solution, followed by incubation on ice for 30 minutes. During this period, antibody samples were prepared at a concentration of 50 µg/mL and diluted 3-fold. After blocking, the cell density was diluted to 5 × 10⁵ cells per 1 mL by adding flow cytometry buffer, and 100 µL of the cell suspension was seeded into a U-shaped 96-well plate. After centrifugation, the supernatant was discarded. 100 µL of each serially diluted antibody sample was added to each well and incubated with the cells at 4°C for 60 minutes.

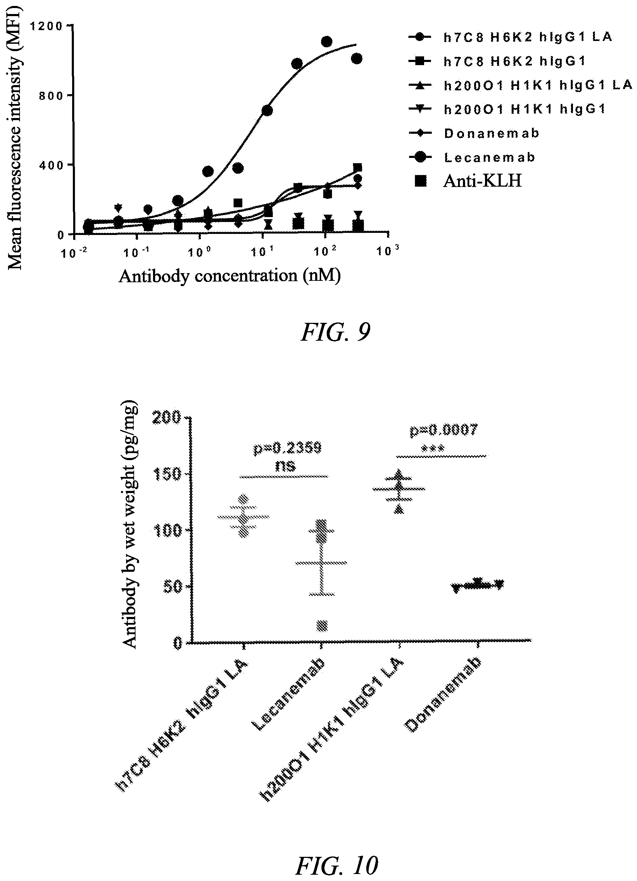

After incubation, the cells were washed once with 200 µL of flow cytometry buffer. Then, 50 µL of AF647 goat anti-human IgG, Fcγ secondary antibody dilution (Jackson ImmunoResearch, 109-606-170, 1 : 300 dilution) was added to each well to label the antibody on the cell surface. After incubation on ice for 20 minutes, 50 µL of PI solution (Sigma, P4170, 1 : 100 dilution) was added, and the incubation was continued for 5 minutes. The cells were washed once. Then, 70 µL of PBS was added to resuspend the cells. The mean fluorescence intensity of the cells was measured using a flow cytometer, and the results were recorded. An antibody concentration-mean fluorescence intensity curve was obtained by fitting with a fourparameter model. The results are shown in FIG. 9 and Table 10. h200O1 H1K1 LA did not bind to amyloid precursor protein, indicating excellent binding specificity; h7C8 H6K2 LA exhibited weak binding to amyloid precursor protein, but with binding intensity significantly weaker than that of Lecanemab, indicating that h7C8 H6K2 LA has better binding specificity.

**Table 10. Binding of different antibodies to APP**

| Name | EC₅₀ (nM) |
|---|---|
| h7C8 H6K2 hIgG1 | N.A |
| h200O1 H1K1 hIgG1 | N.A |
| h7C8 H6K2 hIgG1 LA | N.A |
| h200O1 H1K1 hIgG1 LA | N.A |
| Donanemab | N.A |
| Lecanemab | 6.83 |

| | |
|---|---|
| N.A indicates weak or no binding of antibody to APP, making it impossible to fit and obtain the EC₅₀ result. | |

### Example 13. Detection of intracerebral concentration of long-acting antibody

A total of 12 C57BL/6 mice aged 12 months and weighing 25-30 g (Biocytogen Jiangsu Co., Ltd.) were selected. The mice were randomly divided into four groups, with three mice in each group. h7C8 H6K2 LA, h200O1 H1K1 LA, Lecanemab and Donanemab were biotinylated before administration. The antibody concentrations in mouse brain tissues were detected using ELISA. A single dose of 30 mg/kg was administered via tail vein injection, with an injection volume of 100 µL/mouse. Seven days after administration, the mouse brains were collected, homogenized, and then centrifuged. The supernatant was collected. The antibody concentrations in brain tissues were detected using ELISA. As shown in FIG. 10 and Table 11, seven days after administration, the intracerebral concentration of the variant h7C8 H6K2 LA of the 7C8 humanized antibody tended to be higher than that of Lecanemab, while the intracerebral concentration of the variant h200O1 H1K1 LA of the 200O1 humanized antibody was significantly higher than that of Donanemab.

**Table 11. Concentrations of different long-acting antibodies in brain tissue**

| Name | Intracerebral concentration (pg/mg) |
|---|---|
| h7C8 H6K2 LA | 112.34 |
| h200O1 H1K1 LA | 137.57 |
| Lecanemab | 73.23 |
| Donanemab | 49.45 |

### Example 14. Effect of humanized antibody on Aβ plaque area in hippocampus and striatum of mice

A total of 25 5X FAD transgenic mice aged 6 weeks and weighing about 20 g (Jiangsu Jinzhihe Biotechnology Co., Ltd.) were selected. The mice were randomly divided into five groups, with five mice in each group. The administration was performed at a dose of 10 mg/kg twice a week for three months. To avoid the generation of anti-drug antibodies against the humanized antibodies in mice during long-term administration, the Fc of the test drug was modified to mIgG2a in this example.

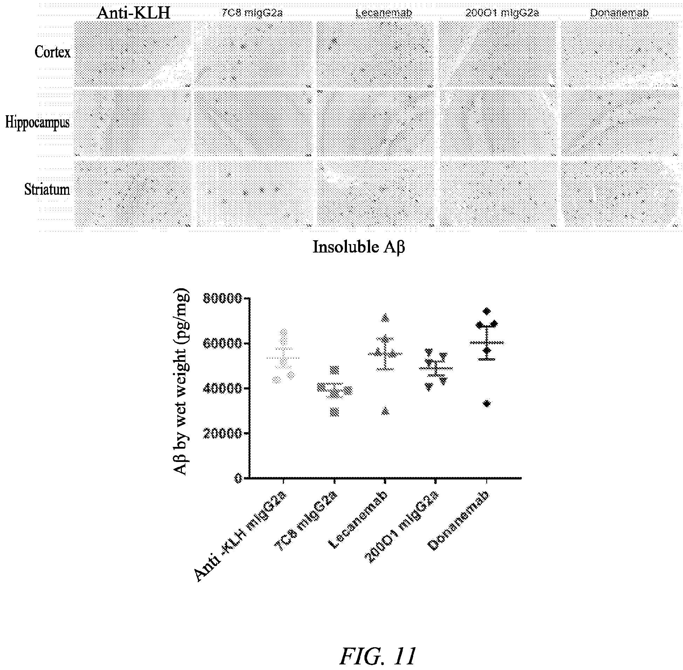

At the end of the administration, the mouse brain was collected. One half of the brain was used to prepare frozen sections, and the cortex, cerebellum and corpus callosum were stained for Aβ by immunohistochemistry. The other half of the brain was used to extract insoluble Aβ with guanidine hydrochloride, and the Aβ1-42 in the samples was quantified using the Human Aβ42 ELISA Kit (Cat: KHB3441, Invitrogen). As shown in FIG. 11 and Table 12, 7C8 mIgG2a and 200O1 mIgG2a exhibited a more significant trend in reducing insoluble Aβ compared to Lecanemab and Donanemab, and 7C8 mIgG2a and 200O1 mIgG2a significantly reduced the Aβ plaque area in the hippocampus and striatum.

**Table 12. Average Aβ plaque area in hippocampus and striatum of 5X FAD mice after administration of different antibodies**

| Name | Hippocampus (µm²) | Striatum (µm²) |
|---|---|---|
| 7C8 mIgG2a | 136.39 | 310.49 |
| 200O1 mIgG2a | 211.57 | 517.93 |
| Lecanemab | 328.45 | 1102.34 |
| Donanemab | 488.67 | 1180.84 |
| Anti-KLH | 530.43 | 1407.23 |

### Example 15. Effect of humanized antibody on soluble and insoluble Aβ levels in mouse brain

### 5X FAD mice

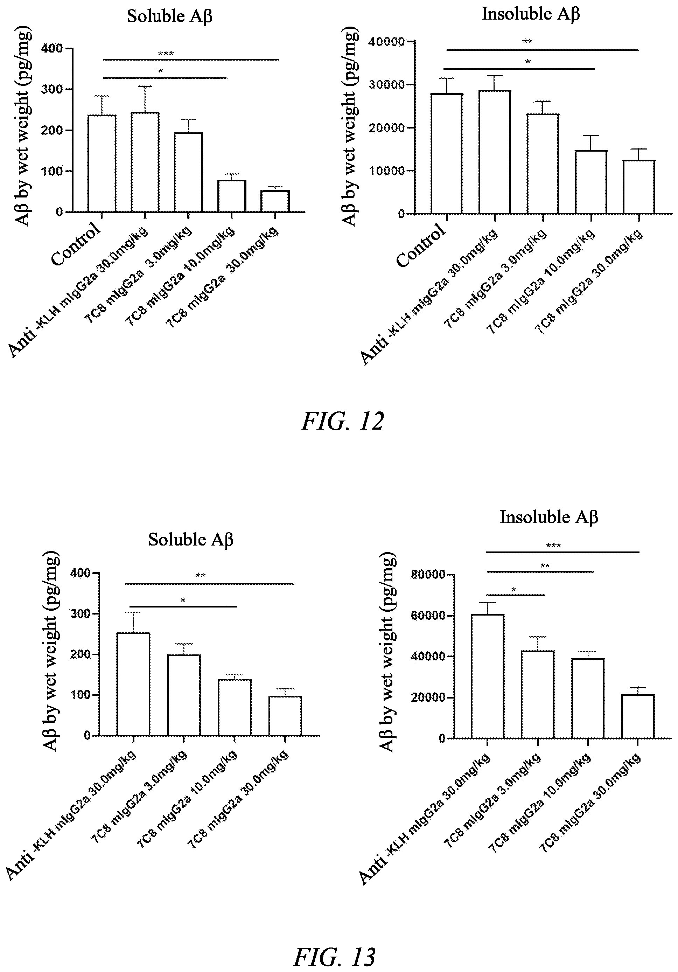

6-Week-old 5X FAD transgenic mice (Jiangsu Jinzhihe Biotechnology Co., Ltd.) were selected. The mice were divided into a 7C8 mIgG2a low-dose group (3 mg/kg), a 7C8 mIgG2a medium-dose group (10 mg/kg), a 7C8 mIgG2a high-dose group (30 mg/kg), and an anti-KLH mIgG2a group (30 mg/kg). The animals were administered twice a week for 13 weeks, for a total of 27 administrations. A blank control was also set up. At 13 weeks, the right hemispheres of the animals were collected for the detection of soluble Aβ1-42, insoluble Aβ1-42, and brain plaque staining in brain tissues, and the percentage reduction in soluble and insoluble Aβ was calculated relative to the anti-KLH mIgG2a group. The results are shown in FIG. 12 and Table 13. 7C8 mIgG2a significantly reduced the soluble and insoluble Aβ levels in the brain of 5X FAD mice.

**Table 13. Percentage reduction in soluble and insoluble Aβ in brain tissue of 5X FAD mice after administration of humanized antibodies**

| Name | Soluble Aβ (%) | Insoluble Aβ (%) |
|---|---|---|
| 7C8 mIgG2a 3 mg/kg | 18.23 | 16.92 |
| 7C8 mIgG2a 10 mg/kg | 66.97 | 46.86 |
| 7C8 mIgG2a 30 mg/kg | 87.10 | 54.80 |
| Anti-KLH | / | / |

### 6-Week-old FAD 4T mice

6-Week-old FAD 4T transgenic mice (GemPharmatech Co., Ltd.) were selected. The mice were divided into a 7C8 mIgG2a low-dose group (3 mg/kg), a 7C8 mIgG2a medium-dose group (10 mg/kg), a 7C8 mIgG2a high-dose group (30 mg/kg), and an anti-KLH mIgG2a group (30 mg/kg). The animals were administered twice a week for 13 weeks, for a total of 27 administrations. At 13 weeks, the right hemispheres of the animals were collected for the detection of soluble Aβ1-42, insoluble Aβ1-42, and brain plaque staining in brain tissues, and the percentage reduction in soluble and insoluble Aβ was calculated relative to the anti-KLH mIgG2a group. The results are shown in FIG. 13 and Table 14. 7C8 mIgG2a significantly reduced the soluble and insoluble Aβ levels in the brain of 6-week-old FAD 4T mice.

**Table 14. Percentage reduction in soluble and insoluble Aβ in brain tissue of 6-week-old FAD 4T mice after administration of humanized antibodies**

| Name | Soluble Aβ (%) | Insoluble Aβ (%) |
|---|---|---|
| 7C8 mIgG2a 3 mg/kg | 21.52 | 29.33 |
| 7C8 mIgG2a 10 mg/kg | 45.44 | 35.67 |
| 7C8 mIgG2a 30 mg/kg | 61.61 | 64.65 |
| Anti-KLH | / | / |

### 15-Week-old FAD 4T mice

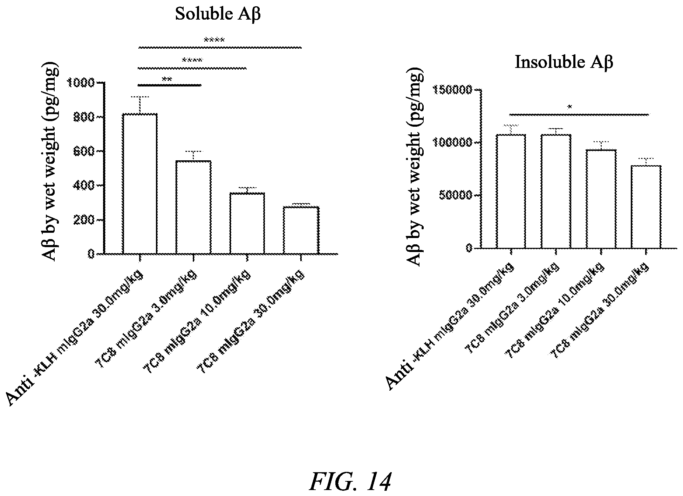

15-Week-old FAD 4T transgenic mice (GemPharmatech Co., Ltd.) were selected. The mice were divided into a 7C8 mIgG2a low-dose group (3 mg/kg), a 7C8 mIgG2a medium-dose group (10 mg/kg), a 7C8 mIgG2a high-dose group (30 mg/kg), and an anti-KLH mIgG2a group (30 mg/kg). The animals were administered twice a week for 13 weeks, for a total of 27 administrations. At 13 weeks, the right hemispheres of the animals were collected for the detection of soluble Aβ1-42, insoluble Aβ1-42, and brain plaque staining in brain tissues, and the percentage reduction in soluble and insoluble Aβ was calculated relative to the anti-KLH mIgG2a group. The results are shown in FIG. 14 and Table 15. 7C8 mIgG2a also reduced the soluble and insoluble Aβ levels in the brain of 15-week-old FAD 4T mice.

**Table 15. Percentage reduction in soluble and insoluble Aβ in brain tissue of 15-week-old FAD 4T mice after administration of humanized antibodies**

| Name | Soluble Aβ (%) | Insoluble Aβ (%) |
|---|---|---|
| 7C8 mIgG2a 3 mg/kg | 33.51 | / |
| 7C8 mIgG2a 10 mg/kg | 56.48 | 13.59 |
| 7C8 mIgG2a 30 mg/kg | 66.07 | 27.54 |
| Anti-KLH | / | / |

## Claims

1. An antibody or an antigen-binding fragment thereof that binds to β-amyloid (Aβ), **characterized in that** the antibody comprises a heavy chain variable region (VH) and a light chain variable region (VL), wherein
(1) the VH comprises a heavy chain complementarity determining region (HCDR) 1 as set forth in SEQ ID NO: 23, a HCDR2 as set forth in SEQ ID NO: 24, and a HCDR3 as set forth in SEQ ID NO: 25, and the VL comprises a light chain complementarity determining region (LCDR) 1 as set forth in SEQ ID NO: 28, a LCDR2 as set forth in SEQ ID NO: 29, and a LCDR3 as set forth in SEQ ID NO: 30;
(2) the VH comprises a HCDR1 as set forth in SEQ ID NO: 69, a HCDR2 as set forth in SEQ ID NO: 70, and a HCDR3 as set forth in SEQ ID NO: 71, and the VL comprises a LCDR1 as set forth in SEQ ID NO: 74, a LCDR2 as set forth in SEQ ID NO: 248, and a LCDR3 as set forth in SEQ ID NO: 76;
(3) the VH comprises a HCDR1 as set forth in SEQ ID NO: 69, a HCDR2 as set forth in SEQ ID NO: 70, and a HCDR3 as set forth in SEQ ID NO: 71, and the VL comprises a LCDR1 as set forth in SEQ ID NO: 74, a LCDR2 as set forth in SEQ ID NO: 75, and a LCDR3 as set forth in SEQ ID NO: 76;
(4) the VH comprises a HCDR1 as set forth in SEQ ID NO: 3, a HCDR2 as set forth in SEQ ID NO: 4, and a HCDR3 as set forth in SEQ ID NO: 5, and the VL comprises a LCDR1 as set forth in SEQ ID NO: 8, a LCDR2 as set forth in SEQ ID NO: 9, and a LCDR3 as set forth in SEQ ID NO: 10;
(5) the VH comprises a HCDR1 as set forth in SEQ ID NO: 13, a HCDR2 as set forth in SEQ ID NO: 14, and a HCDR3 as set forth in SEQ ID NO: 15, and the VL comprises a LCDR1 as set forth in SEQ ID NO: 18, a LCDR2 as set forth in SEQ ID NO: 19, and a LCDR3 as set forth in SEQ ID NO: 20;
(6) the VH comprises a HCDR1 as set forth in SEQ ID NO: 33, a HCDR2 as set forth in SEQ ID NO: 34, and a HCDR3 as set forth in SEQ ID NO: 35, and the VL comprises a LCDR1 as set forth in SEQ ID NO: 38, a LCDR2 as set forth in SEQ ID NO: 19, and a LCDR3 as set forth in SEQ ID NO: 20;
(7) the VH comprises a HCDR1 as set forth in SEQ ID NO: 41, a HCDR2 as set forth in SEQ ID NO: 42, and a HCDR3 as set forth in SEQ ID NO: 43, and the VL comprises a LCDR1 as set forth in SEQ ID NO: 46, a LCDR2 as set forth in SEQ ID NO: 47, and a LCDR3 as set forth in SEQ ID NO: 48;
(8) the VH comprises a HCDR1 as set forth in SEQ ID NO: 51, a HCDR2 as set forth in SEQ ID NO: 52, and a HCDR3 as set forth in SEQ ID NO: 53, and the VL comprises a LCDR1 as set forth in SEQ ID NO: 56, a LCDR2 as set forth in SEQ ID NO: 19, and a LCDR3 as set forth in SEQ ID NO: 20;
(9) the VH comprises a HCDR1 as set forth in SEQ ID NO: 59, a HCDR2 as set forth in SEQ ID NO: 60, and a HCDR3 as set forth in SEQ ID NO: 61, and the VL comprises a LCDR1 as set forth in SEQ ID NO: 64, a LCDR2 as set forth in SEQ ID NO: 65, and a LCDR3 as set forth in SEQ ID NO: 66;
(10) the VH comprises a HCDR1 as set forth in SEQ ID NO: 3, a HCDR2 as set forth in SEQ ID NO: 4, and a HCDR3 as set forth in SEQ ID NO: 79, and the VL comprises a LCDR1 as set forth in SEQ ID NO: 82, a LCDR2 as set forth in SEQ ID NO: 83, and a LCDR3 as set forth in SEQ ID NO: 84;
(11) the VH comprises a HCDR1 as set forth in SEQ ID NO: 87, a HCDR2 as set forth in SEQ ID NO: 88, and a HCDR3 as set forth in SEQ ID NO: 89, and the VL comprises a LCDR1 as set forth in SEQ ID NO: 92, a LCDR2 as set forth in SEQ ID NO: 93, and a LCDR3 as set forth in SEQ ID NO: 94;
(12) the VH comprises a HCDR1 as set forth in SEQ ID NO: 130, a HCDR2 as set forth in SEQ ID NO: 131, and a HCDR3 as set forth in SEQ ID NO: 279, and the VL comprises a LCDR1 as set forth in SEQ ID NO: 74, a LCDR2 as set forth in SEQ ID NO: 134, and a LCDR3 as set forth in SEQ ID NO: 76;
(13) the VH comprises a HCDR1 as set forth in SEQ ID NO: 59, a HCDR2 as set forth in SEQ ID NO: 137, and a HCDR3 as set forth in SEQ ID NO: 138, and the VL comprises a LCDR1 as set forth in SEQ ID NO: 141, a LCDR2 as set forth in SEQ ID NO: 65, and a LCDR3 as set forth in SEQ ID NO: 66;
(14) the VH comprises a HCDR1 as set forth in SEQ ID NO: 121, a HCDR2 as set forth in SEQ ID NO: 122, and a HCDR3 as set forth in SEQ ID NO: 144, and the VL comprises a LCDR1 as set forth in SEQ ID NO: 147, a LCDR2 as set forth in SEQ ID NO: 148, and a LCDR3 as set forth in SEQ ID NO: 149;
(15) the VH comprises a HCDR1 as set forth in SEQ ID NO: 121, a HCDR2 as set forth in SEQ ID NO: 122, and a HCDR3 as set forth in SEQ ID NO: 123, and the VL comprises a LCDR1 as set forth in SEQ ID NO: 218, a LCDR2 as set forth in SEQ ID NO: 65, and a LCDR3 as set forth in SEQ ID NO: 127;
(16) the VH comprises a HCDR1 as set forth in SEQ ID NO: 121, a HCDR2 as set forth in SEQ ID NO: 122, and a HCDR3 as set forth in SEQ ID NO: 123, and the VL comprises a LCDR1 as set forth in SEQ ID NO: 126, a LCDR2 as set forth in SEQ ID NO: 65, and a LCDR3 as set forth in SEQ ID NO: 127;
(17) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 103, and a LCDR3 as set forth in SEQ ID NO: 104;
(18) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 235, and a LCDR3 as set forth in SEQ ID NO: 104;
(19) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 238, and a LCDR3 as set forth in SEQ ID NO: 104;
(20) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 232, a LCDR2 as set forth in SEQ ID NO: 245, and a LCDR3 as set forth in SEQ ID NO: 104;
(21) the VH comprises a HCDR1 as set forth in SEQ ID NO: 97, a HCDR2 as set forth in SEQ ID NO: 98, and a HCDR3 as set forth in SEQ ID NO: 99, and the VL comprises a LCDR1 as set forth in SEQ ID NO: 102, a LCDR2 as set forth in SEQ ID NO: 103, and a LCDR3 as set forth in SEQ ID NO: 104;
(22) the VH comprises a HCDR1 as set forth in SEQ ID NO: 107, a HCDR2 as set forth in SEQ ID NO: 108, and a HCDR3 as set forth in SEQ ID NO: 99, and the VL comprises a LCDR1 as set forth in SEQ ID NO: 102, a LCDR2 as set forth in SEQ ID NO: 103, and a LCDR3 as set forth in SEQ ID NO: 111; or
(23) the VH comprises a HCDR1 as set forth in SEQ ID NO: 114, a HCDR2 as set forth in SEQ ID NO: 98, and a HCDR3 as set forth in SEQ ID NO: 115, and the VL comprises a LCDR1 as set forth in SEQ ID NO: 102; a LCDR2 as set forth in SEQ ID NO: 118, and a LCDR3 as set forth in SEQ ID NO: 104.

2. The antibody or the antigen-binding fragment thereof according to claim 1, **characterized in that** the antibody or the antigen-binding fragment thereof binds to pyroE Aβ3-42 peptide.

3. The antibody or the antigen-binding fragment thereof according to claim 1, **characterized in that** the antibody or the antigen-binding fragment thereof binds to Aβ1-42 protofibril.

4. The antibody or the antigen-binding fragment thereof according to any one of claims 1-3, **characterized in that**
(1) the VH and VL comprise amino acid sequences that have at least 80%, at least 85%, at least 90%, at least 95%, at least 96%, at least 97%, at least 98%, at least 99% or 100% sequence identity to SEQ ID NO: 164 and SEQ ID NO: 219, respectively;
(2) the VH and VL comprise amino acid sequences that have at least 80%, at least 85%, at least 90%, at least 95%, at least 96%, at least 97%, at least 98%, at least 99% or 100% sequence identity to SEQ ID NO: 166 and SEQ ID NO: 219, respectively;
(3) the VH and VL comprise amino acid sequences that have at least 80%, at least 85%, at least 90%, at least 95%, at least 96%, at least 97%, at least 98%, at least 99% or 100% sequence identity to SEQ ID NO: 174 and SEQ ID NO: 219, respectively;
(4) the VH and VL comprise amino acid sequences that have at least 80%, at least 85%, at least 90%, at least 95%, at least 96%, at least 97%, at least 98%, at least 99% or 100% sequence identity to SEQ ID NO: 176 and SEQ ID NO: 219, respectively;
(5) the VH and VL comprise amino acid sequences that have at least 80%, at least 85%, at least 90%, at least 95%, at least 96%, at least 97%, at least 98%, at least 99% or 100% sequence identity to SEQ ID NO: 21 and SEQ ID NO: 26, respectively;
(6) the VH and VL comprise amino acid sequences that have at least 80%, at least 85%, at least 90%, at least 95%, at least 96%, at least 97%, at least 98%, at least 99% or 100% sequence identity to SEQ ID NO: 197 and SEQ ID NO: 246, respectively;
(7) the VH and VL comprise amino acid sequences that have at least 80%, at least 85%, at least 90%, at least 95%, at least 96%, at least 97%, at least 98%, at least 99% or 100% sequence identity to SEQ ID NO: 197 and SEQ ID NO: 249, respectively;
(8) the VH and VL comprise amino acid sequences that have at least 80%, at least 85%, at least 90%, at least 95%, at least 96%, at least 97%, at least 98%, at least 99% or 100% sequence identity to SEQ ID NO: 67 and SEQ ID NO: 72, respectively;
(9) the VH and VL comprise amino acid sequences that have at least 80%, at least 85%, at least 90%, at least 95%, at least 96%, at least 97%, at least 98%, at least 99% or 100% sequence identity to SEQ ID NO: 1 and SEQ ID NO: 6, respectively;
(10) the VH and VL comprise amino acid sequences that have at least 80%, at least 85%, at least 90%, at least 95%, at least 96%, at least 97%, at least 98%, at least 99% or 100% sequence identity to SEQ ID NO: 11 and SEQ ID NO: 16, respectively;
(11) the VH and VL comprise amino acid sequences that have at least 80%, at least 85%, at least 90%, at least 95%, at least 96%, at least 97%, at least 98%, at least 99% or 100% sequence identity to SEQ ID NO: 31 and SEQ ID NO: 36, respectively;
(12) the VH and VL comprise amino acid sequences that have at least 80%, at least 85%, at least 90%, at least 95%, at least 96%, at least 97%, at least 98%, at least 99% or 100% sequence identity to SEQ ID NO: 39 and SEQ ID NO: 44, respectively;
(13) the VH and VL comprise amino acid sequences that have at least 80%, at least 85%, at least 90%, at least 95%, at least 96%, at least 97%, at least 98%, at least 99% or 100% sequence identity to SEQ ID NO: 49 and SEQ ID NO: 54, respectively;
(14) the VH and VL comprise amino acid sequences that have at least 80%, at least 85%, at least 90%, at least 95%, at least 96%, at least 97%, at least 98%, at least 99% or 100% sequence identity to SEQ ID NO: 57 and SEQ ID NO: 62, respectively;
(15) the VH and VL comprise amino acid sequences that have at least 80%, at least 85%, at least 90%, at least 95%, at least 96%, at least 97%, at least 98%, at least 99% or 100% sequence identity to SEQ ID NO: 77 and SEQ ID NO: 80, respectively;
(16) the VH and VL comprise amino acid sequences that have at least 80%, at least 85%, at least 90%, at least 95%, at least 96%, at least 97%, at least 98%, at least 99% or 100% sequence identity to SEQ ID NO: 85 and SEQ ID NO: 90, respectively;
(17) the VH and VL comprise amino acid sequences that have at least 80%, at least 85%, at least 90%, at least 95%, at least 96%, at least 97%, at least 98%, at least 99% or 100% sequence identity to SEQ ID NO: 128 and SEQ ID NO: 132, respectively;
(18) the VH and VL comprise amino acid sequences that have at least 80%, at least 85%, at least 90%, at least 95%, at least 96%, at least 97%, at least 98%, at least 99% or 100% sequence identity to SEQ ID NO: 135 and SEQ ID NO: 139, respectively;
(19) the VH and VL comprise amino acid sequences that have at least 80%, at least 85%, at least 90%, at least 95%, at least 96%, at least 97%, at least 98%, at least 99% or 100% sequence identity to SEQ ID NO: 142 and SEQ ID NO: 145, respectively;
(20) the VH and VL comprise amino acid sequences that have at least 80%, at least 85%, at least 90%, at least 95%, at least 96%, at least 97%, at least 98%, at least 99% or 100% sequence identity to SEQ ID NO: 160 and SEQ ID NO: 216, respectively;
(21) the VH and VL comprise amino acid sequences that have at least 80%, at least 85%, at least 90%, at least 95%, at least 96%, at least 97%, at least 98%, at least 99% or 100% sequence identity to SEQ ID NO: 152 and SEQ ID NO: 216, respectively;
(22) the VH and VL comprise amino acid sequences that have at least 80%, at least 85%, at least 90%, at least 95%, at least 96%, at least 97%, at least 98%, at least 99% or 100% sequence identity to SEQ ID NO: 156 and SEQ ID NO: 216, respectively;
(23) the VH and VL comprise amino acid sequences that have at least 80%, at least 85%, at least 90%, at least 95%, at least 96%, at least 97%, at least 98%, at least 99% or 100% sequence identity to SEQ ID NO: 158 and SEQ ID NO: 216, respectively;
(24) the VH and VL comprise amino acid sequences that have at least 80%, at least 85%, at least 90%, at least 95%, at least 96%, at least 97%, at least 98%, at least 99% or 100% sequence identity to SEQ ID NO: 162 and SEQ ID NO: 216, respectively;
(25) the VH and VL comprise amino acid sequences that have at least 80%, at least 85%, at least 90%, at least 95%, at least 96%, at least 97%, at least 98%, at least 99% or 100% sequence identity to SEQ ID NO: 160 and SEQ ID NO: 214, respectively;
(26) the VH and VL comprise amino acid sequences that have at least 80%, at least 85%, at least 90%, at least 95%, at least 96%, at least 97%, at least 98%, at least 99% or 100% sequence identity to SEQ ID NO: 152 and SEQ ID NO: 214, respectively;
(27) the VH and VL comprise amino acid sequences that have at least 80%, at least 85%, at least 90%, at least 95%, at least 96%, at least 97%, at least 98%, at least 99% or 100% sequence identity to SEQ ID NO: 162 and SEQ ID NO: 214, respectively;
(28) the VH and VL comprise amino acid sequences that have at least 80%, at least 85%, at least 90%, at least 95%, at least 96%, at least 97%, at least 98%, at least 99% or 100% sequence identity to SEQ ID NO: 119 and SEQ ID NO: 124, respectively;
(29) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 230, respectively;
(30) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 233, respectively;
(31) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 236, respectively;
(32) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 239, respectively;
(33) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 241, respectively;
(34) the VH and VL comprise amino acid sequences that have at least 80%, at least 85%, at least 90%, at least 95%, at least 96%, at least 97%, at least 98%, at least 99% or 100% sequence identity to SEQ ID NO: 185 and SEQ ID NO: 243, respectively;
(35) the VH and VL comprise amino acid sequences that have at least 80%, at least 85%, at least 90%, at least 95%, at least 96%, at least 97%, at least 98%, at least 99% or 100% sequence identity to SEQ ID NO: 95 and SEQ ID NO: 100, respectively;
(36) the VH and VL comprise amino acid sequences that have at least 80%, at least 85%, at least 90%, at least 95%, at least 96%, at least 97%, at least 98%, at least 99% or 100% sequence identity to SEQ ID NO: 105 and SEQ ID NO: 109, respectively; or
(37) the VH and VL comprise amino acid sequences that have at least 80%, at least 85%, at least 90%, at least 95%, at least 96%, at least 97%, at least 98%, at least 99% or 100% sequence identity to SEQ ID NO: 112 and SEQ ID NO: 116, respectively.

5. The antibody or the antigen-binding fragment thereof according to any one of claims 1-4, **characterized in that** the antibody is selected from a murine antibody, a chimeric antibody, a humanized antibody or a fully human antibody.

6. The antibody or the antigen-binding fragment thereof according to any one of claims 1-5, **characterized in that** the antibody is of an antibody isotype selected from: IgG, IgA, IgM, IgE and IgD.

7. The antibody or the antigen-binding fragment thereof according to any one of claims 1-6, **characterized in that** the antibody is of an antibody subclass selected from: IgG1, IgG2, IgG3 and IgG4.

8. The antibody or the antigen-binding fragment thereof according to any one of claims 1-7, **characterized in that** the antibody comprises an IgG1 constant region (Fc region); optionally, the Fc region comprises an amino acid substitution that extends the half-life of the antibody, preferably, the amino acid substitution comprises L234A and L235A substitutions.

9. The antibody or the antigen-binding fragment thereof according to any one of claims 1-8, **characterized in that** the antibody comprises a heavy chain (HC) and a light chain (LC), wherein
(1) the HC and LC comprise amino acid sequences that have at least 80%, at least 85%, at least 90%, at least 95%, at least 96%, at least 97%, at least 98%, at least 99% or 100% sequence identity to SEQ ID NO: 275 and SEQ ID NO: 277, respectively; or
(2) the HC and LC comprise amino acid sequences that have at least 80%, at least 85%, at least 90%, at least 95%, at least 96%, at least 97%, at least 98%, at least 99% or 100% sequence identity to SEQ ID NO: 271 and SEQ ID NO: 273, respectively.

10. The antibody or the antigen-binding fragment thereof according to any one of claims 1-9, **characterized in that** the antigen-binding fragment is selected from: Fab, Fab', F(ab')₂, Fv, single-chain Fv (scFv) and ds-scFv.

11. The antibody or the antigen-binding fragment thereof according to any one of claims 1-10, **characterized in that** the antibody is a monovalent antibody, a bivalent antibody or a multivalent antibody.

12. The antibody or the antigen-binding fragment thereof according to any one of claims 1-11, **characterized in that** the antibody is a monoclonal antibody, a bispecific antibody or a multispecific antibody.

13. A nucleic acid molecule, **characterized by** comprising a nucleotide sequence encoding the antibody or the antigen-binding fragment thereof according to any one of claims 1-12.

14. A vector, **characterized by** comprising the nucleic acid molecule according to claim 13.

15. A cell, **characterized by** comprising the nucleic acid molecule according to claim 13 or the vector according to claim 14.

16. A method for preparing the antibody or the antigen-binding fragment thereof according to any one of claims 1-12, **characterized in that** the method comprises:
a) culturing the cell according to claim 15 under conditions suitable for expression of the antibody or antigen-binding fragment thereof; and
b) isolating the antibody or the antigen-binding fragment thereof from a culture and/or a culture supernatant of the cell.

17. A pharmaceutical composition, **characterized by** comprising the antibody or the antigen-binding fragment thereof according to any one of claims 1-12, the nucleic acid molecule according to claim 13, the vector according to claim 14 or the cell according to claim 15, and optionally, a pharmaceutically acceptable carrier or excipient.

18. A kit, **characterized by** comprising the antibody or the antigen-binding fragment thereof according to any one of claims 1-12, the nucleic acid molecule according to claim 13, the vector according to claim 14, the cell according to claim 15 or the pharmaceutical composition according to claim 17, and instructions for use; optionally, the kit further comprises an administration device.

19. Use of the antibody or the antigen-binding fragment thereof according to any one of claims 1-12, the nucleic acid molecule according to claim 13, the vector according to claim 14, the cell according to claim 15 or the pharmaceutical composition according to claim 17 in the preparation of a drug for treating or preventing a disease caused by abnormal accumulation or deposition of Aβ in a subject.

20. Use of the antibody or the antigen-binding fragment thereof according to any one of claims 1-12, the nucleic acid molecule according to claim 13, the vector according to claim 14, the cell according to claim 15 or the pharmaceutical composition according to claim 17 in the preparation of a drug for clearing or alleviating abnormal accumulation or deposition of Aβ in a subject.

21. The use according to claim 19 or 20, **characterized in that** the subject has a degenerative disease of the central nervous system.

22. The use according to claim 21, **characterized in that** the subject has Alzheimer's disease (AD), Parkinson's disease (PD), Down syndrome or cerebral amyloid angiopathy, or is at risk of having AD, PD, Down syndrome or cerebral amyloid angiopathy.

23. The use according to claim 22, **characterized in that** the subject has preclinical AD or clinical AD, e.g., prodromal AD, mild AD, moderate AD or severe AD.
